# EUROPEAN PATENT SPECIFICATION

(11) **EP 2 699 683 B1**
(45) Date of publication and mention of the grant of the patent: **28.09.2016**
(21) Application number: 12717108.0
(22) Date of filing: 23.04.2012
(51) Int. Cl.: C12N 15/82, A01H 5/00, C07K 14/415

(54) **METHODS AND MEANS TO PRODUCE ABIOTIC STRESS TOLERANT PLANTS**
VERFAHREN UND VORICHTUNG ZUR HERSTELLUNG VON GEGENÜBER ABIOTISCHEM STRESS TOLERANTEN PFLANZEN
PROCÉDÉS ET MOYENS DE PRODUCTION DE PLANTES TOLÉRANT UN STRESS ABIOTIQUE

(30) Priority: 22.04.2011 US 201161517622 P; 26.04.2011 GB 201106845
(43) Date of publication of application: 26.02.2014
(73) Proprietor: VIB VZW, 9052 Gent (BE); Universiteit Gent, 9000 Gent (BE)
(72) Inventor: INZÉ, Dirk, Gustaaf, 9310 Moorsel - Aalst (BE); SKIRYCZ, Aleksandra, 9000 Gent (BE); CLAEYS, Hannes, 8600 Diksmuide (BE); COPPENS, Frederik, 9968 Bassevelde (BE)
(86) International application number: PCT/EP2012/057342
(87) International publication number: WO 2012/143545

(56) References cited:
- WO-A2-2012/007919
- A. SKIRYCZ ET AL: "Developmental Stage Specificity and the Role of Mitochondrial Metabolism in the Response of Arabidopsis Leaves to Prolonged Mild Osmotic Stress", PLANT PHYSIOLOGY, vol. 152, no. 1, 1 January 2010 (2010-01-01), pages 226-244, XP55032033, ISSN: 0032-0889, DOI: 10.1104/pp.109.148965 cited in the application
- ALEKSANDRA SKIRYCZ ET AL: "More from less: plant growth under limited water", CURRENT OPINION IN BIOTECHNOLOGY, vol. 21, no. 2, 2 April 2010 (2010-04-02), pages 197-203, XP028282836, ISSN: 0958-1669, DOI: 10.1016/J.COPBIO.2010.03.002 [retrieved on 2010-03-10] cited in the application
- TALEISNIK E ET AL: "Leaf expansion in grasses under salt stress", JOURNAL OF PLANT PHYSIOLOGY, FISCHER, STUTTGART, DE, vol. 166, no. 11, 15 July 2009 (2009-07-15), pages 1123-1140, XP026195736, ISSN: 0176-1617, DOI: 10.1016/J.JPLPH.2009.03.015 [retrieved on 2009-05-20]
- SENTHIL-KUMAR M ET AL: "Virus-induced gene silencing and its application in characterizing genes involved in water-deficit-stress tolerance", JOURNAL OF PLANT PHYSIOLOGY, FISCHER, STUTTGART, DE, vol. 165, no. 13, 8 September 2008 (2008-09-08), pages 1404-1421, XP023784777, ISSN: 0176-1617, DOI: 10.1016/J.JPLPH.2008.04.007 [retrieved on 2008-06-09]

## Description

### Field of the invention

The present invention relates to the field of plant molecular biology, more particularly to the field of agriculture, and concerns methods for enhancing the abiotic stress tolerance in plants by modulating the expression of a gene involved in the ethylene signal transduction pathway during the period of abiotic stress. The present invention also provides chimeric constructs useful in the methods in the invention. In addition, the invention provides transgenic plants having an enhanced abiotic stress resistance.

### Introduction to the invention

Abiotic stress is defined as the negative impact of non-living factors on the living organisms in a specific environment. The non-living variable must influence the environment beyond its normal range of variation to adversely affect the population performance or individual physiology of the organism in a significant way. Abiotic stress is essentially unavoidable. Abiotic stress affects animals, but plants are especially dependent on environmental factors, so it is particularly constraining. Abiotic stress is the most harmful factor concerning the growth and productivity of crops worldwide. Drought, temperature extremes, and saline soils are the most common abiotic stresses that plants encounter. Globally, approximately 22% of agricultural land is saline and areas under drought are already expanding and this is expected to increase further. Other crops are exposed to multiple stresses, and the manner in which a plant senses and responds to different environmental factors appears to be overlapping. The most obvious detriment concerning abiotic stress involves farming. It has been calculated that abiotic stress causes the most crop loss of any other factor and that most major crops are reduced in their yield by more than 50% from their potential yield. In addition, it has been speculated that this yield reduction will only worsen with the dramatic climate changes expected in the future. Because abiotic stress is widely considered a detrimental effect, the research on this branch of the issue is extensive. When subjected to environmental stress, plants actively reduce their vegetative growth to save and redistribute resources and thus increase their chance of survival when the stress becomes severe (Skirycz and Inzé, 2010). However, when the stress does not threaten survival, growth inhibition can be seen as counter-productive because it leads to an unnecessary drop in productivity and substantial yield penalties. "Bolder" plants that are able to grow during mild stress episodes might prove an efficient way to boost productivity in regions that do not experience severe weather conditions (Tardieu, 2003). Therefore, understanding the mechanisms underlying growth inhibition in response to stress can lead to entry points for interfering with the stress induced growth reductions. In plants, organ growth is driven by two tightly controlled and dynamic processes: cell proliferation and subsequent cell expansion. The coordination of these two processes during leaf growth ultimately determines leaf size and shape. In dicots, such as the model species *Arabidopsis thaliana,* leaves initiate at the flank of the meristem and, in the initial phase, their growth is driven exclusively by cell proliferation (Donnelly et al., 1999). In somewhat older leaves, cells will exit the mitotic cell cycle and start expanding from the tip onward. This transition is manifested by the onset of endoreduplication, which is a modified cell cycle in which replication proceeds without mitosis with higher ploidy levels as a consequence (Beemster et al., 2005). In water-limited environments, plants respond by a rapid initial growth reduction followed by growth adaptation, resulting in leaves with fewer and smaller cells (Schuppler et al., 1998; Granier and Tardieu, 1999; Aguirrezabal et al., 2006; Skirycz et al., 2010). Whereas previously we investigated processes involved in growth adaptation to long-term exposure to stress (Skirycz et al., 2010), the aim of our research was to learn more about the mechanisms underlying acute stress-mediated growth inhibition. Although reduction of cell proliferation upon stress onset is a well-known phenomenon, how changes in the environment translate into reduced proliferation rates is only poorly understood. At the level of the cell cycle machinery the most often proposed scenario that mediates stress-induced cell cycle inhibition assumes transcriptional up-regulation of cell cycle inhibitors that belong to the inhibitor of cyclin-dependent kinase (CDK) (ICK)/KIP-related protein (KRP) and/or the SIAMESE family. These inhibitors are thought to transiently arrest cell proliferation by inhibiting CDKA/cyclin complexes (De Veylder et al., 2001; Churchman et al., 2006; Peres et al., 2007; Rymen et al., 2007). CYCLIN DEPENDENT KINASE A (CDKA) activity, which is a main driver of cell cycle progression, can also be reduced *via* targeted degradation of cyclins and/or inhibitory phosphorylation, as shown for wheat (*Triticum aestivum*) plants subjected to drought stress (Schuppler et al., 1998). Upstream of the cell cycle machinery, the plant hormone abscisic acid (ABA) has been demonstrated to affect the expression of the *ICK*/*KRP* and/or *SIAMESE* genes (Wang et al., 1998; Pettkó-Szandtner et al., 2006). Another classical stress hormone is ethylene, which was shown to accumulate upon drought (Kalantari et al., 2000; Sobeih et al., 2004) and similarly to ABA, the ethylene precursor 1-aminocyclopropane-1-carboxylate (ACC) is known to be transported from root to shoot (reviewed by Sobeih et al., 2004). As such, ABA and ethylene are considered good candidates to communicate changes in the soil water status to the meristems. Examples of positive as well as negative effects of ethylene and ABA on growth can be found in the literature (reviewed by Sharp and LeNoble, 2002; Pierik et al., 2006) but their exact role in cell cycle regulation remains largely unknown.

In the present invention we examined how mild drought stress affects cell proliferation during early leaf development. In contrast to expanding leaves, entirely proliferating *Arabidopsis* leaves are extremely small (less than 0.1 mm² in size) and, thus, it is a technical challenge to obtain a molecular basis of stress-induced cell cycle arrest with sufficient developmental and temporal resolution. To this end, a novel experimental set-up had to be established to enable the simultaneous analysis of growth-related parameters and molecular mechanisms specifically in the proliferating leaves upon short term exposure to stress. Unlike many previous studies focusing on very severe stress in mature leaves or complete seedlings (e.g. Fujita et al., 2007; Kant et al., 2007; Papdi et al., 2008), this mild stress set-up slowed down growth without affecting plant survival. The examples of the invention clearly demonstrate that cell cycle arrest is a very rapid response to stress mediated by posttranscriptional mechanisms rather than a transcriptional cascade, with the plant hormone ethylene upstream of the reversible cell cycle arrest. Whereas ethylene is a primary signal for growth arrest, the subsequent ethylene-independent cell cycle exit occurs relatively late and only when the stress persists. Such highly temporal regulation allows plants to fine-tune their growth response according to the stress duration. Thus, the present invention shows that ethylene is a prime signal responsible for meristem arrest during stress exposure and thus substantially contributes to stress associated growth penalty and yield losses. However, since ethylene has pleiotropic effects on plant growth and development, and ectopic modification of ethylene metabolism and/or signalling may result in a number of undesirable phenotypes, there was a need to reduce the ethylene production, during the period of abiotic stress, in the growth meristems of the plant. We have shown that lowering ethylene levels in meristematic tissues can therefore relieve observed growth repression and thus limit yield losses. This was achieved by constructing dedicated chimeric genes for either downregulation or upregulation of genes in the ethylene signal transduction pathway. We surprisingly showed that the growth arrest imposed by the abiotic stress could be overcome with a group of specific meristematic promoters, while this effect was abscent with other meristematic promoters or with promoters which direct a constitutive expression in plants.

### Figure legends

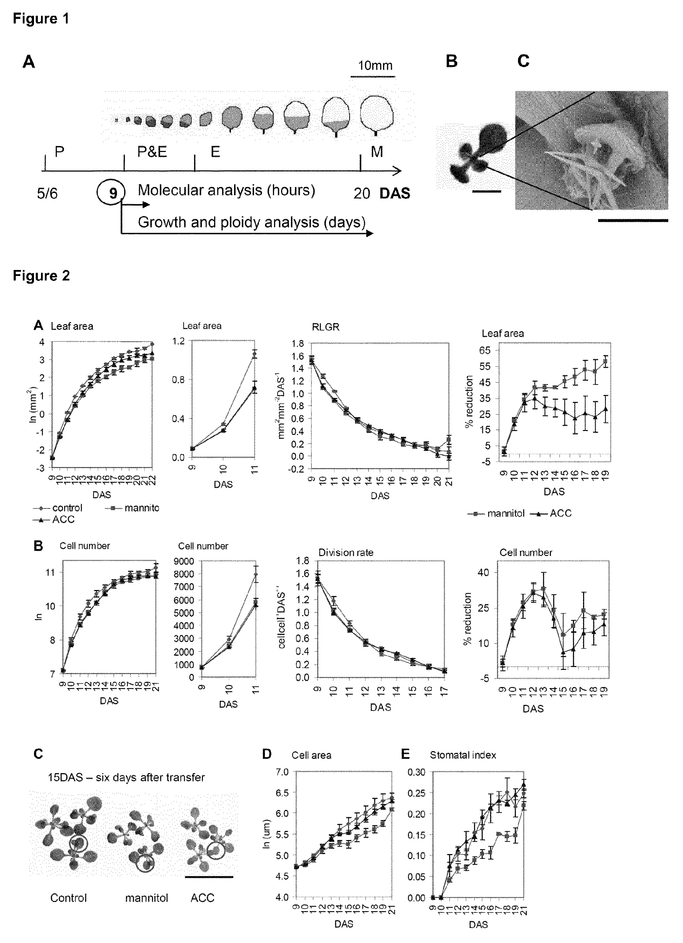

Figure 1: Experimental setup.
   (A) Schematic representation of leaf 3 development with proliferating (P, red), expanding (E, green) and mature (M, white) cells. At 9 days after stratification (DAS), plants were transferred to mannitol and leaf 3 was dissected for growth, ploidy, and molecular analysis.
   (B) Nine-day-old seedling.
   (C) Electron micrograph of the 3rd and 4th leaves at 9 DAS.
      Bars = 2 mm in (B) and 200 µm in (C).
Figure 2: Kinematic analysis of leaf 3 dissected from plants transferred to control, mannitol- or ACC-containing media at 9 DAS, when the 3rd leaf is fully proliferating.
   (A) Leaf area, relative leaf growth rate, and percent reduction of leaf area caused by mannitol or ACC.
   (B) Cell number, relative cell division rates, and percent reduction of cell number caused by mannitol or ACC.
   (C) Plants 6 days after transfer to control, mannitol- or ACC-containing media. The red circle marks the 3rd leaf. Bar = 2 cm.
   (D) Cell area.
   (E) Stomatal index.
   (A-E) Data ± standard error (SE) are the means of three independent experiments. Leaf area was measured for 8-10 leaves in each experiment. Cellular data are from four leaves in each experiment.

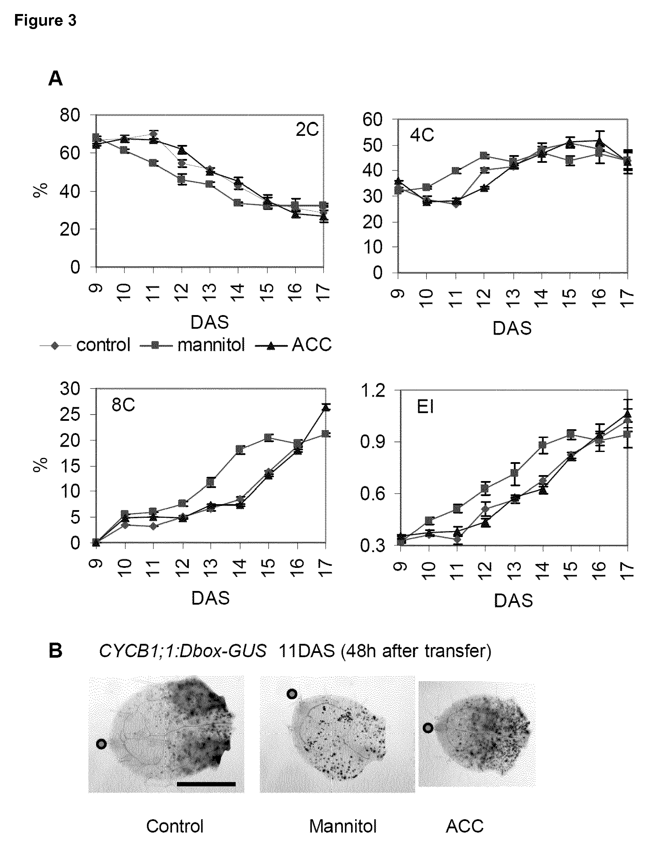

Figure 3: Osmotic stress arrests cell cycle and subsequently triggers cell cycle exit.
   (A) Ploidy analysis of leaf 3 dissected from plants transferred to control, mannitol- or ACC-containing media at 9 DAS when 3rd leaf is fully proliferating. Percentage of 2C, 4C, and 8C nuclei is presented. EI stands for endoreduplication index and represents the average number of endocycles undergone by a typical nucleus (EI=1*4C+2*8C+3*16C). Data ± SE are means of three independent experiments with multiple leaves pooled in each experiment.
   (B) Leaf 3 of *CYCB1;1:DBox-GUS* plants 48 h (11 DAS) after transfer to control, mannitol- or ACC-containing medium. Blue staining indicates mitotic activity. Orange dot indicates leaf tip. Scale bar = 0.5 mm.

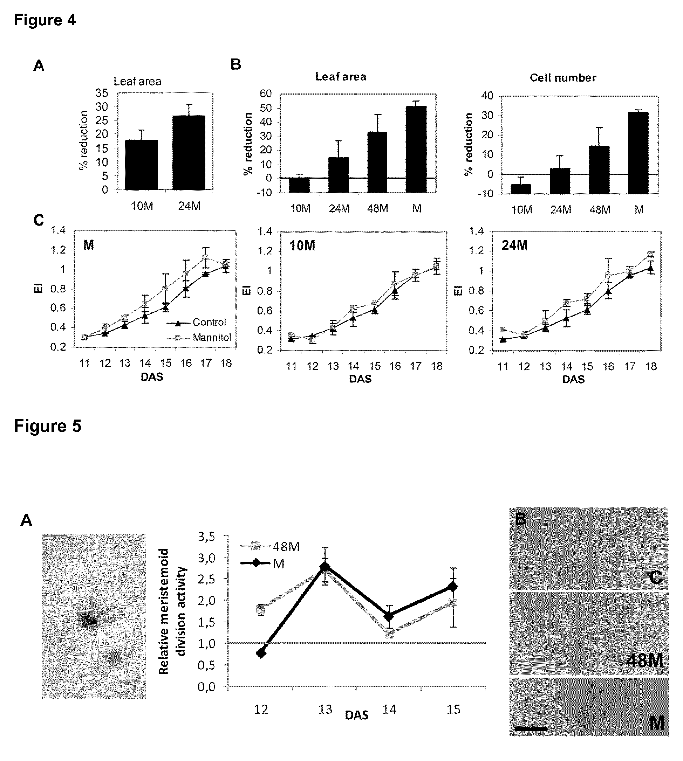

Figure 4: Effects of varying stress duration on proliferating leaves.
   Plants were transferred to mannitol-containing plates at 9 DAS and afterward transferred back to control plates after 10 h (10M), 24 h (24M), or 48 h (48M) of mannitol treatment, or kept on mannitol plates (M). Third leaf was dissected for further analysis.
   (A) Reduction of leaf area at 10 DAS (24 h after first transfer).
   (B) Reduction of leaf area and cell number at 14 DAS.
   (C) Ploidy analysis. EI stands for endoreduplication index and represents the average number of endocycles undergone by a typical nucleus. Data ± SE are means of three independent experiments with multiple leaves measured in each experiment.
Figure 5: Effects of osmotic stress on meristemoid division activity and proliferation zone.
   Plants were transferred to mannitol-containing plates at 9 DAS and afterward transferred back to control plates after 48 h of mannitol treatment (48M) or kept on mannitol plates (M). Third leaf was dissected for further analysis.
   (A) Meristemoid division activity as determined by *CYCB1;1:DBox-GUS* staining, expressed relative to control. Data ± SE are means of three independent experiments with 6-12 leaves measured in each experiment. A representative active meristemoid is depicted on the left.
   (B) Leaf base of 3rd leaf stained for *CYCB1;1:DBox-GUS* expression at 14 DAS. Some mitotic activity can still be seen in the proliferation zone of mannitol-treated leaves. Bar = 0.5 mm.

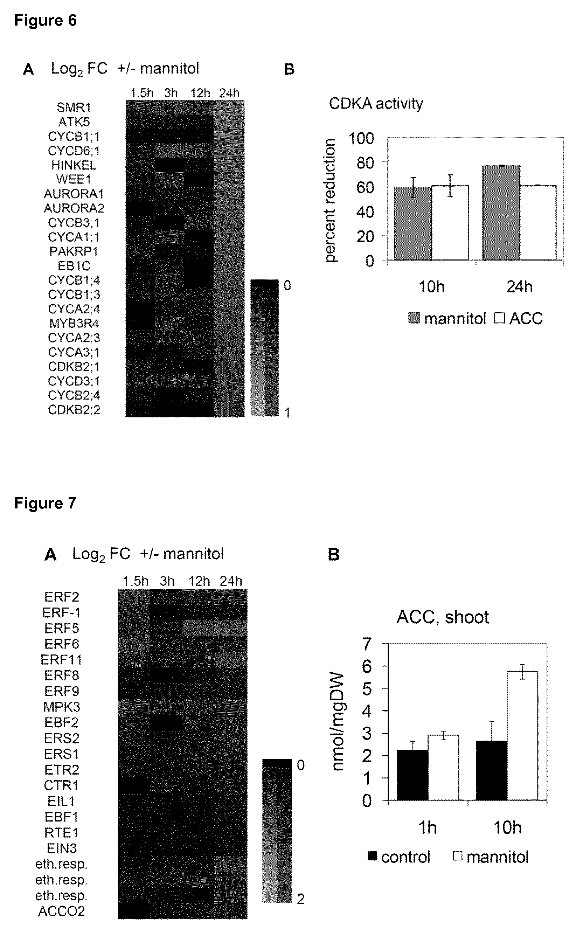

Figure 6: Osmotic stress effects on cell cycle.
   (A) Heat map of selected cell cycle genes differentially regulated by osmotic stress in the 3rd fully proliferating leaf 1.5, 3, 12, and 24 h after stress imposition. Data are from Affymetrix ATH1 arrays and are expressed as the log₂ of fold change (mannitol - control). Red and green indicate up-regulation and down-regulation, respectively.
   (B) Relative CDKA activity measured in the 3rd proliferating leaf, microdissected from plants transferred to control, mannitol- or ACC-containing medium 10 and 24 h after transfer. Data ± SE are means of two (24 h) or three (10 h) independent experiments with ± 50 leaves pooled in each experiment.
Figure 7: Rapid increase in ACC levels after stress imposition.
   (A) Heat map of selected ethylene signaling genes differentially regulated by osmotic stress in the 3rd fully proliferating leaf 1.5, 3, 12, and 24 h after stress imposition. Data are from Affymetrix ATH1 arrays and are expressed as the log₂ of fold change (mannitol - control). Red and green indicate up-regulation and down-regulation, respectively.
   (B) ACC levels were determined in shoots of 9-DAS seedlings 1 h and 10 h after transfer to mannitol. Data are means ± SE of multiple plants from three independent experiments.

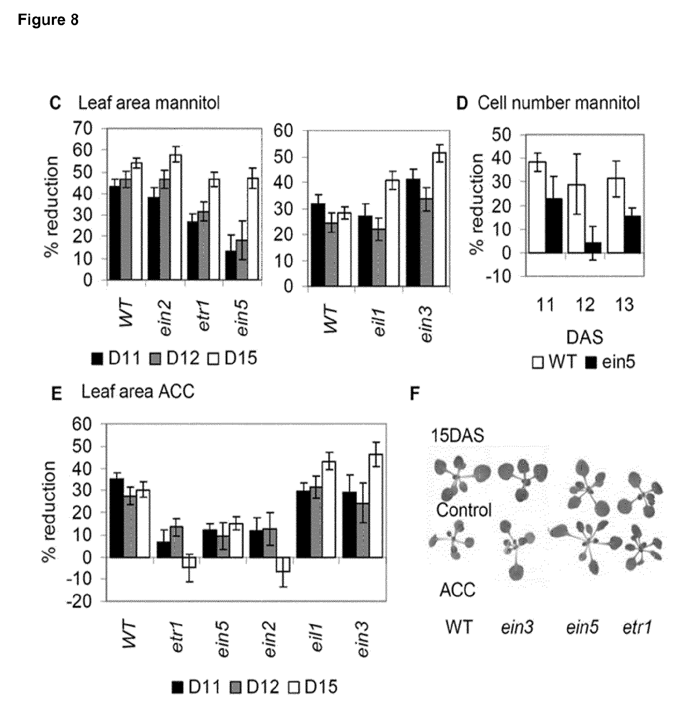

Figure 8: Involvement of ethylene signaling in cell cycle arrest.
   (D-F) Ethylene-insensitive mutants. Percent reduction in leaf area (C) and in cell number of the 3rd leaf of mannitol-treated ethylene-insensitive mutants versus wild-type (WT) plants (D). Percent reduction in leaf area of the 3rd leaf of ACC-treated ethylene-insensitive mutants versus wild-type (WT) plants (E). Example of wild-type (WT), *ein3, etr1,* and *ein5* seedling 6 days after transfer to ACC (F).
   (C-E) Data ± SE are means of two or three independent experiments. Leaf area was measured for minimum 8-10 leaves in each experiment. Cellular data were from four leaves in each experiment. D, DAS.

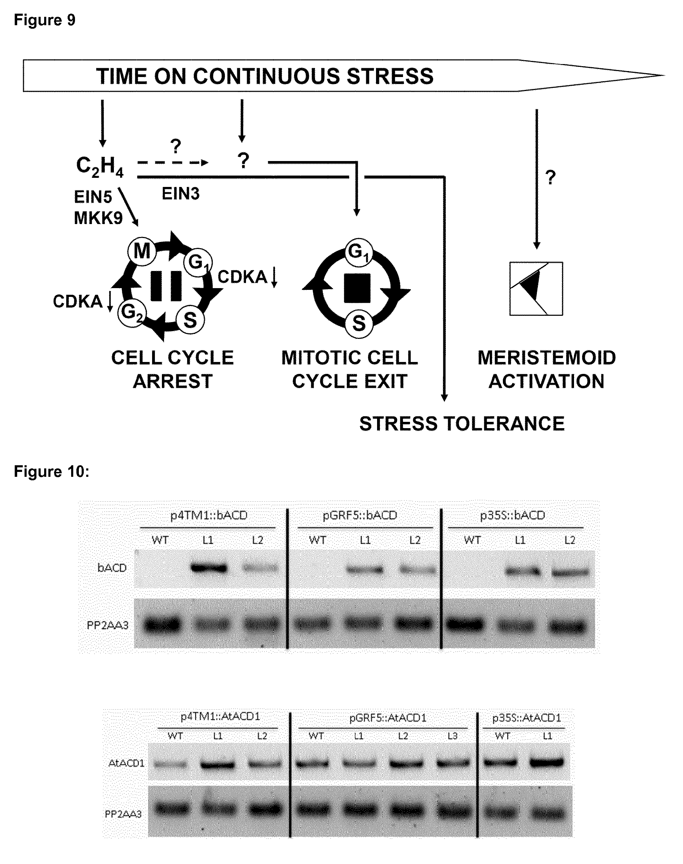

Figure 9: Simplified scheme depicting the regulation of cell cycle inhibition and cell differentiation in response to osmotic stress.
   Very rapidly (within hours) after stress imposition, ethylene production is triggered, inhibiting CDKA activity through a posttranscriptional mechanism that reversibly inhibits the cell cycle by G1/S and G2/M arrest. Cell cycle arrest is independent of EIN3 transcriptional control and possibly mediated by a MAPK signaling pathway or the ribonuclease EIN5. In a later phase, a different signal leads to permanent inhibition and exit from the mitotic cell cycle in favor of the endocycle and cell differentiation. Later in leaf development, meristemoid division activity becomes higher in stressed leaves and the enhanced meristemoid division results in a small recovery of cell numbers.
Figure 10: Expression was measured on RNA isolated from 8 day-old shoots by PCR with primers specific for either the bacterial ACC deaminase or the Arabidopsis thaliana ACC deaminase. As a control the reference gene PP2AA3 (genbank entry AT1G13320) was used.
Figure 11: Leaf area at D11 (48 hours after stress onset); blue bars (left bar) = control, red bars (right bar) = 25 mM mannitol; error bars indicate SEM.
Figure 12: Leaf area at D11 (48 hours after stress onset); blue bars (left bars) = control, red bars (right bars) = 25 mM mannitol; error bars indicate SEM
Figure 13: Leaf area at D11 (48 hours after stress onset); blue bars (left bars) = control, red bars (right bars) = 25 mM mannitol; error bars indicate SEM
Figure 14: Leaf area at D11 (48 hours after stress onset); blue bars (left bars) = control, red bars (right bars) = 25 mM mannitol; error bars indicate SEM
Figure 15: Leaf area at D11 (48 hours after stress onset); blue bars (left bars) = control, red bars (right bars) = 25 mM mannitol; error bars indicate SEM
Figure 16: Leaf area at D11 (48 hours after stress onset); blue bars (left bars) = control, red bars (right bars) = 25 mM mannitol; error bars indicate SEM

### Detailed description of the invention

To facilitate the understanding of this invention a number of terms are defined below. Terms defined herein (unless otherwise specified) have meanings as commonly understood by a person of ordinary skill in the areas relevant to the present invention. As used in this specification and its appended claims, terms such as "a", "an" and "the" are not intended to refer to only a singular entity, but include the general class of which a specific example may be used for illustration, unless the context dictates otherwise. The terminology herein is used to describe specific embodiments of the invention, but their usage does not delimit the invention, except as outlined in the claims.

Despite its importance for agriculture, environmental stress-induced growth inhibition, which is responsible for significant yield reductions, is only poorly understood. In the present invention we unraveled the molecular mechanisms underlying cell cycle inhibition in young proliferating leaves of the model plant *Arabidopsis thaliana* when subjected to mild osmotic stress. A detailed cellular analysis demonstrated that as soon as osmotic stress is sensed, cell cycle progression rapidly arrests, but cells are kept in an ambivalent state allowing a quick recovery ("pause"). Remarkably, we found that cell cycle arrest coincides with an increase in 1-aminocyclopropane-1-carboxylate (ACC) levels and the activation of ethylene signaling. Careful study showed that ethylene acts on cell cycle progression via inhibition of cyclin-dependent kinase A activity independently of *EIN3* transcriptional control. However, when the stress persists, cells exit the mitotic cell cycle and initiate the differentiation process ("stop"), reflected by early endoreduplication onset, in a process independent of ethylene.

Accordingly the present invention provides for a method for producing an abiotic stress tolerant plant relative to a control plant, by decreasing the ethylene signal transduction pathway in said plant during the period of abiotic stress imposed on said plants comprising introducing and expressing in said plant a chimeric gene comprising of a meristem specific promoter operably linked to a gene or gene fragment derived from the ethylene signal transduction pathway. In a particular embodiment said abiotic stress (or environmental stress which is equivalent) is mild stress. The meaning of "mild stress" is apparent from the text of the application and the further appended examples.

The wording 'decreasing the ethylene signal transduction pathway' implies that the production of ethylene is reduced (at a certain point in time, i.e. when the plant suffers from abiotic stress) in the plant with respect to a control plant. Genes involved in the ethylene signal transduction pathway are well known in the art. A list of genes for which at least one gene needs to be downregulated to have the effect for the reduced production of ethylene consists of the following list: ACO1, ACO2, ETR2, ETR1, CTR1, MKK7, MKK9, EIN3, EIN5 or EIL1. Conversely, a list of genes for which at least one gene needs to be upregulated to have the effect for the reduced production of ethylene consists of the following list: EBF1, EBF2, ACC deaminase, a dominant negative ethylene receptor or a dominant negative transcription factor EIN3 or EIL1. It is understood in the present invention that the reduction of ethylene in the transgenic plant preferentially occurs in the proliferative tissues of interest in the plant, more particularly in the meristematic tissues of the transgenic plants.

The concept of a dominant negative ethylene receptor is well known in the art. A receptor comprising a dominant mutation disrupts ethylene-binding activity and transgenic plants comprising a chimeric gene encoding a dominant negative ethylene receptor are insensitive to ethylene. A commonly used ETR1 dominant receptor mutant is the etr1-1 dominant mutation as described in Bleecker et al (1998) Science 241: 1086-9.

The concept of a dominant negative transcription factor is also well described in the art. Non-limiting examples are transcription factors with mutations in the DNA-binding domain or transcription factors comprising mutations in the transactivation domain.

A representative of the ACC deaminase from *Arabidopsis thaliana* is AT1G48420 (i.e. the AGI code for the *Arabidopis* gene). ACC is the ethylene precursor and ACC deaminases are a group of enzymes that cleave ACC to ammonia and α-ketobutyrate. Several orthologous genes for the ACC deaminase genes can be identified in publically available sequence databases (the database accession numbers are between parenthesis: Arabidopsis lyrata (AL1G42050), Arabidopsis thaliana (AT1G48420), Brachypodium distachyon (BD3G57520), Brachypodium distachyon (BD4G03710), Carica papaya (CP00007G01400), Chlamydomonas reinhardtii (CR07G01440), Glycine max (GM05G33540),Glycine max (GM08G06170), Lotus japonicas (LJ0G028460), Malus domestica (MD14G022550), Malus domestica (MD00G198360), Manihot esculenta (ME04612G00040), Micromonas sp. RCC299 (MRCC299_11G02790), Medicago truncatula (MT4G61890), Ostreococcus lucimarinus (OL17G01760), Oryza sativa ssp. Japonica (OS02G53330), Oryza sativa ssp. Indica (OSINDICA_02G51690), Ostreococcus tauri (OT09G01960), Physcomitrella patens (PP00224G00230), Populus trichocarpa (PT12G03410), Ricinus communis (RC29662G00090), Sorghum bicolor (SB04G034640), Selaginella moellendorffii (SM00001G01880), Selaginella moellendorffii (SM00016G03880), Selaginella moellendorffii (SM00016G03890), Selaginella moellendorffii (SM00001G01870), Volvox carteri (VC00048G01110), Vitis vinifera (VV00G26680), Zea mays (ZM05G34830).

A representative for the ACO2 gene (ACC oxidase) from *Arabidopsis thaliana* is AT1 G62380 (i.e. the AGI code for the *Arabidopis* gene). ACC is transported to meristems from roots where it is converted to ethylene by the activity of ACC oxidases. Several orthologous genes for the ACO2 genes can be identified in publically available sequence databases (the database accession numbers are depicted after the species name): Arabidopsis lyrata AL2G03110, Arabidopsis lyrata AL1G12340, Arabidopsis thaliana AT1G62380, Arabidopsis thaliana AT1G12010, Carica papaya CP00132G00310, Carica papaya CP00152G00570, Glycine max GM07G39420, Glycine max GM17G01330, Glycine max GM15G11930, Glycine max GM09G01110, Malus domestica MD00G333820, Malus domestica MD00G333830, Malus domestica MD17G009150, Malus domestica MD05G001020, Malus domestica MD10G027080, Manihot esculenta ME08317G00820, Medicago truncatula MT3G41110, Medicago truncatula MT5G67830, Medicago truncatula MT8G56840, Medicago truncatula MT8G56830, Populus trichocarpa PT04G00270, Populus trichocarpa PT11G00780, Vitis vinifera VV12G11440, Brachypodium distachyon BD3G57620, Lotus japonicas LJ0G427530, Oryza sativa ssp. Japonica OS09G27750, Oryza sativa ssp. Indica OSINDICA_09G22130, Physcomitrella patens PP00287G00300, Ricinus communis RC49629G00010, Sorghum bicolor SB04G034520, Selaginella moellendorffii SM00010G00400, Selaginella moellendorffii SM00026G03400, Selaginella moellendorffii SM00006G03800, Selaginella moellendorffii SM00006G03820, Selaginella moellendorffii SM00006G03790, Selaginella moellendorffii SM00006G03780, Zea mays ZM07G12430.

A representative for the ACO1 gene (ACC oxidase) from *Arabidopsis thaliana* is AT2G19590 (i.e. the AGI code for the *Arabidopsis* gene). ACC is transported to meristems from roots where it is converted to ethylene by the activity of ACC oxidases. Several orthologous genes for the ACO1 genes can be identified in publically available sequence databases (the database accession numbers are depicted after the species name): Arabidopsis lyrata AL3G36600, Arabidopsis thaliana AT2G19590, Brachypodium distachyon BD1G37420, Glycine max GM07G15480, Glycine max GM05G36310, Glycine max GM08G03310, Glycine max GM01G01170, Lotus japonicas LJ2G008930, Malus domestica MD15G014610, Manihot esculenta ME01863G00320, Oryza sativa ssp. Japonica OS06G37590, Oryza sativa ssp. Indica OSINDICA_06G33260, Populus trichocarpa PT06G15230, Ricinus communis RC29912G00850, Sorghum bicolor SB10G022640, Vitis vinifera VV11G03750, Zea mays ZM06G11580, Carica papaya CP00132G00310, Medicago truncatula MT3G41110, Medicago truncatula MT5G67830, Physcomitrella patens PP00046G00760, Physcomitrella patens PP00283G00400, Selaginella moellendorffii SM00026G03400, Selaginella moellendorffii SM00098G00980.

A representative for the EIN3 gene from *Arabidopsis thaliana* is AT3G20770 (i.e. the AGI code for the *Arabidopsis* gene). EIN3 is a central transcription factor in the ethylene signaling cascade. Several orthologous genes for the EIN3 genes can be identified in publically available sequence databases (the database accession numbers are depicted after the species name): Arabidopsis lyrata AL3G22790, Arabidopsis lyrata AL4G10480, Arabidopsis thaliana AT3G20770, Arabidopsis thaliana AT2G27050, Brachypodium distachyon BD1G63780, Brachypodium distachyon BD3G46690, Brachypodium distachyon BD3G46680, Brachypodium distachyon BD4G33750, Brachypodium distachyon BD3G39970, Brachypodium distachyon BD5G12130, Carica papaya CP00103G00550, Carica papaya CP00054G00220, Glycine max GM20G12250, Glycine max GM13G03700, Glycine max GM13G03660, Glycine max GM14G04550, Glycine max GM02G44220, Lotus japonicas LJ2G036110, Malus domestica MD00G054980, Malus domestica MD02G025170, Malus domestica MD00G131400, Malus domestica MD00G407350, Malus domestica MD08G022690, Malus domestica MD07G004090, Malus domestica MD00G335120, Manihot esculenta ME04048G00040, Medicago truncatula MT5G69950, Medicago truncatula MT0G29830, Oryza sativa ssp. Japonica OS03G20790, Oryza sativa ssp. Japonica OS03G20780, Oryza sativa ssp. japonica OS08G39830, Oryza sativa ssp. japonica OS07G17160, Oryza sativa ssp. japonica OS02G36510, Oryza sativa ssp. japonica OS07G48630, Oryza sativa ssp. japonicaOS09G31400, Oryza sativa ssp. japonica OS04G38400, Oryza sativa ssp. Indica OSINDICA_03G19810, Oryza sativa ssp. indica OSINDICA_04G29950, Oryza sativa ssp. indica OSINDICA_02G35300, Oryza sativa ssp. Indica OSINDICA_07G14940, Oryza sativa ssp. indica OSINDICA_09G25260, Oryza sativa ssp. indica OSINDICA_08G38220, Oryza sativa ssp. Indica OSINDICA_07G40580, Oryza sativa ssp. indica OSINDICA_05G02810, Oryza sativa ssp. indica OSINDICA_08G38160, Physcomitrella patens PP00087G00240, Populus trichocarpa PT09G15890, Populus trichocarpa PT04G19690, Populus trichocarpa PT08G01010, Populus trichocarpa PT10G24230, Populus trichocarpa PT01G05180, Populus trichocarpa PT01G05170 1, Ricinus communis RC29708G00010, Ricinus communis RC30169G01650, Sorghum bicolor SB01G036740, Sorghum bicolor SB07G027790, Sorghum bicolor SB04G023730, Sorghum bicolor SB06G018710, Sorghum bicolorSB02G043350, Sorghum bicolor SB02G028390, Selaginella moellendorffii SM00043G02600, Selaginella moellendorffii SM00144G00050, Selaginella moellendorffii SM00042G02400, Selaginella moellendorffii SM00088G00860, Zea mays ZM01G12220, Zea mays ZM09G17170, Zea mays ZM02G11960, Zea mays ZM05G25920, Zea mays ZM07G25000, Zea mays ZM02G33750, Zea mays ZM07G14110, Vitis vinifera VV06G06520, Vitis vinifera VV06G12160.

Representative for the EIL1 genes from *Arabidopsis thaliana* are AT2G27050, At5G21120 and At1G73730 (i.e. the AGI codes for the *Arabidopsis* genes). EIL1 genes encode for central transcription factors in the ethylene signaling cascade. Several orthologous genes for the EIL1 genes can be identified in publically available sequence databases (the database accession numbers are depicted after the species name:

### I) orthologous genes for AT2G27050:

Arabidopsis lyrata AL4G10480, Arabidopsis lyrata AL3G22790, Arabidopsis thaliana AT2G27050, Arabidopsis thaliana AT3G20770, Brachypodium distachyon BD1G63780, Brachypodium distachyon BD3G46690, Brachypodium distachyon BD3G46680, Brachypodium distachyon BD4G33750, Brachypodium distachyon BD3G39970, Brachypodium distachyon BD5G12130, Carica papaya CP00103G00550, Carica papaya CP00054G00220, Glycine max GM14G04550, Glycine max GM02G44220, Glycine max GM20G12250, Glycine max GM13G03700, Glycine max GM13G03660, Glycine max GM06G47160, Lotus japonicus LJ2G036110, Malus domestica MD00G054980, Malus domestica MD02G025170, Malus domestica MD00G131400, Malus domestica MD07G004090, Malus domestica MD00G407350, Malus domestica MD08G022690, Malus domestica MD00G335120, Manihot esculenta ME04048G00040, Medicago truncatula MT5G69950, Medicago truncatula MT0G29830, Oryza sativa ssp. japonica OS03G20780, Oryza sativa ssp. japonica OS03G20790, Oryza sativa ssp. japonica OS02G36510, Oryza sativa ssp. japonica OS07G17160, Oryza sativa ssp. japonica OS04G38400, Oryza sativa ssp. japonica OS08G39830, Oryza sativa ssp. japonica OS07G48630, Oryza sativa ssp. japonica OS09G31400 Oryza sativa ssp. indica OSINDICA_03G19810, Oryza sativa ssp. Indica OSINDICA_04G29950, Oryza sativa ssp. indica OSINDICA_02G35300, Oryza sativa ssp. indica OSINDICA_07G14940, Oryza sativa ssp. Indica OSINDICA_09G25260, Oryza sativa ssp. indica OSINDICA_08G38220, Oryza sativa ssp. indica OSINDICA_07G40580, Oryza sativa ssp. Indica OSINDICA_05G02810, Oryza sativa ssp. indica OSINDICA_08G38160, Physcomitrella patens PP00087G00240, Populus trichocarpa PT09G15890, Populus trichocarpa PT04G19690, Populus trichocarpa PT10G24230, Populus trichocarpa PT08G01010, Ricinus communis RC29708G00010, Sorghum bicolor SB01G036740, Sorghum bicolor SB04G023730, Sorghum bicolor SB06G018710, Sorghum bicolor SB07G027790, Sorghum bicolor SB02G043350, Sorghum bicolor SB02G028390, Selaginella moellendorffii SM00043G02600, Selaginella moellendorffii SM00144G00050, Selaginella moellendorffii SM00042G02400, Selaginella moellendorffii SM00088G00860, Zea mays ZM09G17170, Zea mays ZM01G12220, Zea mays ZM02G33750, Zea mays ZM05G25920, Zea mays ZM02G11960, Zea mays ZM07G25000, Zea mays ZM07G14110, Vitis vinifera VV06G06520, Vitis vinifera VV06G12160.

### II) orthologous genes for AT5G21120:

Arabidopsis lyrata AL6G21370, Arabidopsis lyrata AL4G10480, Arabidopsis thaliana AT5G21120, Carica papaya CP00103G00550, Carica papaya CP00020G02350, Glycine max GM02G44220, Glycine max GM13G03660, Glycine max GM13G03700, Glycine max GM14G04550, Glycine max GM20G12250, Glycine max GM05G31410, Glycine max GM08G14630, Glycine max GM18G02190, Glycine max GM13G41750, Glycine max GM15G03650, Glycine max GM06G47160, Glycine max GM04G14900, Lotus japonicus LJ2G036110, Lotus japonicus LJ3G015320, Medicago truncatula MT0G29830, Medicago truncatula MT3G36600, Medicago truncatula MT4G55800, Medicago truncatula MT6G16890, Medicago truncatula MT2G47310, Medicago truncatula MT5G69950, Populus trichocarpa PT04G19690, Populus trichocarpa PT09G15890 , Populus trichocarpa PT01G05170, Populus trichocarpa PT01G05180, Populus trichocarpa PT03G21140 , Vitis vinifera VV06G06520, Vitis vinifera VV06G12160, Brachypodium distachyon BD1G63780, Brachypodium distachyon BD3G46690, Brachypodium distachyon BD3G46680, Brachypodium distachyon BD4G33750, Brachypodium distachyon BD3G39970, Brachypodium distachyon BD5G12130, Malus domestica MD00G054980, Malus domestica MD00G131400, Malus domestica MD00G407350, Malus domestica MD02G025170, Malus domestica MD00G437780, Malus domestica MD00G437790, Malus domestica MD00G058260, Malus domestica MD00G436270, Malus domestica MD00G436370, Malus domestica MD00G058250, Malus domestica MD11G001760, Manihot esculenta ME04048G00040, Manihot esculenta ME10578G00030, Manihot esculenta ME04747G00240, Manihot esculenta ME02229G00960, Oryza sativa ssp. japonica OS03G20780, Oryza sativa ssp. japonica OS03G20790, Oryza sativa ssp. japonica OS02G36510, Oryza sativa ssp. japonica OS07G17160, Oryza sativa ssp. japonica OS09G31400, Oryza sativa ssp. japonica OS08G39830, Oryza sativa ssp. japonica OS04G38400, Oryza sativa ssp. indica OSINDICA_03G19810, Oryza sativa ssp. indica OSINDICA_02G35300, Oryza sativa ssp. indica OSINDICA_07G14940, Oryza sativa ssp. indica OSINDICA_04G29950, Oryza sativa ssp. indica OSINDICA_09G25260, Oryza sativa ssp. indica OSINDICA_08G38160, Oryza sativa ssp. indica OSINDICA_08G38220, Physcomitrella patens PP00087G00240, Physcomitrella patens PP00011G01730, Ricinus communis RC29708G00010, Ricinus communis RC29713G00060, Ricinus communis RC30169G01650, Ricinus communis RC29713G00070, Sorghum bicolor SB01G036740, Sorghum bicolor SB04G023730, Sorghum bicolor SB06G018710, Sorghum bicolor SB02G028390, Sorghum bicolor SB07G027790, Selaginella moellendorffii SM00144G00050, Selaginella moellendorffii SM00043G02600, Selaginella moellendorffii SM00042G02400, Selaginella moellendorffii SM00088G00860, Zea mays ZM01G12220, Zea mays ZM05G25920, Zea mays ZM07G14110, Zea mays ZM02G11960

### III) orthologous genes for AT1G73730:

Arabidopsis lyrata AL2G22590 4, Arabidopsis thaliana AT1G73730, Brachypodium distachyon BD4G33750, Brachypodium distachyon BD3G39970, Glycine max GM15G03650, Glycine max GM13G41750, Lotus japonicus LJ3G015320, Malus domestica MD11G001760, Malus domestica MD00G058250, Malus domestica MD00G436270, Malus domestica MD00G058260, Malus domestica MD00G436370, Manihot esculenta ME04747G00240, Manihot esculenta ME02229G00960, Medicago truncatula MT2G47310, Medicago truncatula MT6G16890, Oryza sativa ssp. japonica OS09G31400, Oryza sativa ssp. japonica OS08G39830, Oryza sativa ssp. indica OSINDICA_09G25260, Oryza sativa ssp. indica OSINDICA_08G38220, Oryza sativa ssp. indica OSINDICA_08G38160, Populus trichocarpa PT01G05170, Populus trichocarpa PT03G21140, Populus trichocarpa PT01G05180, Ricinus communis RC30169G01650, Sorghum bicolor SB02G028390, Sorghum bicolor SB07G027790, Vitis vinifera VV06G06520, Zea mays ZM07G14110, Carica papaya CP00103G00550, Carica papaya CP00020G02350, Physcomitrella patens PP00087G00240, Physcomitrella patens PP00011G01730, Selaginella moellendorffii SM00144G00050, Selaginella moellendorffii SM00043G02600, Selaginella moellendorffii SM00042G02400, Selaginella moellendorffii SM00088G00860.

A representative gene for the EBF1 gene from *Arabidopsis thaliana* is AT5G25350 (i.e. the AGI code for the *Arabidopsis* gene). The EBF1 gene encodes for a protein that mediates the degradation of the EIN3 and EIL1 transcription factors. Several orthologous genes for the EBF1 gene can be identified in publically available sequence databases (the database accession numbers are depicted after the species name): Arabidopsis lyrata AL6G25490, Arabidopsis lyrata AL4G06680, Arabidopsis thaliana AT5G25350, Arabidopsis thaliana AT2G25490, Brachypodium distachyon BD3G07400, Brachypodium distachyon BD1G36530, Carica papaya CP00750G00010, Carica papaya CP00046G00080, Glycine max GM17G31940, Glycine max GM14G14410, Glycine max GM04G07110, Glycine max GM06G07200, Glycine max GM17G12270, Glycine max GM13G23510, Glycine max GM04G20330, Lotus japonicus LJ5G007560, Lotus japonicus LJ0G118050, Malus domestica MD15G006340, Malus domestica MD08G015310, Malus domestica MD00G485600, Malus domestica MD00G097680, Malus domestica MD15G010590, Malus domestica MD00G026250, Manihot esculenta ME10594G01970, Manihot esculenta ME09799G00070, Manihot esculenta ME02262G00040, Medicago truncatula MT1G06810, Medicago truncatula MT3G54680, Medicago truncatula MT8G41850, Medicago truncatula MT8G41910, Oryza sativa ssp. japonica OS02G10700, Oryza sativa ssp. japonica OS06G40360, Oryza sativa ssp. indica OSINDICA_02G10230, Oryza sativa ssp. indica OSINDICA_06G35900, Physcomitrella patens PP00352G00060, Physcomitrella patens PP00188G00250, Populus trichocarpa PT18G01520, Populus trichocarpa PT06G25310, Populus trichocarpa PT18G12120, Populus trichocarpa PT06G06580, Ricinus communis RC29848G01830, Ricinus communis RC28320G00720, Sorghum bicolor SB04G006870, Sorghum bicolor SB10G023670, Selaginella moellendorffii SM00051G01700, Selaginella moellendorffii SM00071G00630, Selaginella moellendorffii SM00003G03080, Selaginella moellendorffii SM00025G01420, Vitis vinifera VV04G12150, Vitis vinifera VV11G06560, Zea mays ZM05G19050, Zea mays ZM09G10590, Zea mays ZM06G11160, Chlamydomonas reinhardtii CR12G15690, Micromonas sp. RCC299 MRCC299_14G00750, Volvox carteri VC00010G02190.

A representative gene for the EBF2 gene from *Arabidopsis thaliana* is AT2G25490 (i.e. the AGI code for the *Arabidopsis* gene). The EBF2 gene encodes for a protein that mediates the degradation of the EIN3 and EIL1 transcription factors. Several orthologous genes for the EBF2 gene can be identified in publically available sequence databases (the database accession numbers are depicted after the species name): Arabidopsis lyrata AL4G06680, Arabidopsis lyrata AL6G25490, Arabidopsis thaliana AT2G25490, Arabidopsis thaliana AT5G25350, Brachypodium distachyon BD3G07400, Brachypodium distachyon BD1G36530, Carica papaya CP00750G00010, Carica papaya CP00046G00080, Glycine max GM14G14410, Glycine max GM17G31940, Glycine max GM04G07110, Glycine max GM06G07200, Glycine max GM17G12270, Glycine max GM13G23510, Glycine max GM04G20330, Lotus japonicus LJ5G007560, Lotus japonicus LJ0G118050, Malus domestica MD15G006340, Malus domestica MD08G015310, Malus domestica MD00G485600, Malus domestica MD00G097680, Malus domestica MD15G010590, Malus domestica MD00G026250, Manihot esculenta ME10594G01970, Manihot esculenta ME09799G00070, Manihot esculenta ME02262G00040, Medicago truncatula MT1G06810, Medicago truncatula MT3G54680, Medicago truncatula MT8G41850, Medicago truncatula MT8G41910, Oryza sativa ssp. japonica OS02G10700, Oryza sativa ssp. japonica OS06G40360, Oryza sativa ssp. indica OSINDICA_02G10230, Oryza sativa ssp. indica OSINDICA_06G35900, Physcomitrella patens PP00352G00060, Physcomitrella patens PP00188G00250, Populus trichocarpa PT18G01520, Populus trichocarpa PT06G25310, Populus trichocarpa PT18G12120, Populus trichocarpa PT06G06580, Ricinus communis RC29848G01830, Ricinus communis RC28320G00720, Sorghum bicolor SB04G006870, Sorghum bicolor SB10G023670, Selaginella moellendorffii SM00051G01700, Selaginella moellendorffii SM00071G00630, Selaginella moellendorffii SM00003G03080, Selaginella moellendorffii SM00025G01420, Vitis vinifera VV04G12150, Vitis vinifera VV11G06560, Zea mays ZM05G19050, Zea mays ZM09G10590, Zea mays ZM06G11160, Chlamydomonas reinhardtii CR28G00230, Micromonas sp. RCC299 MRCC299_14G00750, Volvox carteri VC00010G02190.

A representative gene for the EIN5 gene from *Arabidopsis thaliana* is AT1G54490 (i.e. the AGI code for the *Arabidopsis* gene). The EIN5 gene encodes for an exoribonuclease upstream of EBF1 and EBF2 transcripts. Several orthologous genes for the EIN5 gene can be identified in publically available sequence databases (the database accession numbers are depicted after the species name):
Arabidopsis thaliana AT1G54490, Brachypodium distachyon BD1G05660, Brachypodium distachyon BD2G56440, Carica papaya CP00117G00640, Carica papaya CP00076G00580, Chlamydomonas reinhardtii CR16G06690, Chlamydomonas reinhardtii CR06G06580, Chlamydomonas reinhardtii CR03G00960, Glycine max GM19G43160, Glycine max GM03G40500, Glycine max GM20G37260, Glycine max GM10G30150, Glycine max GM14G40510, Lotus japonicus LJ0G413420, Lotus japonicus LJ5G017580, Malus domestica MD12G023600, Malus domestica MD04G017840, Malus domestica MD00G306770, Malus domestica MD00G371520, Manihot esculenta ME10645G00090, Manihot esculenta ME07086G00150, Medicago truncatula MT7G55810, Medicago truncatula MT1G31820, Medicago truncatula MT7G60440, Medicago truncatula MT7G60430, Medicago truncatula MT0G01060, Ostreococcus lucimarinus OL06G03410, Ostreococcus lucimarinus OL06G03860, Oryza sativa ssp. japonica OS03G58060, Oryza sativa ssp. japonica OS01G65220, Oryza sativa ssp. indica OSINDICA_03G55400, Oryza sativa ssp. indica OSINDICA_01G61870, Ostreococcus tauri OT06G03610, Ostreococcus tauri OT06G04160, Populus trichocarpa PT00G17430, Populus trichocarpa PT05G04870, Populus trichocarpa PT05G04880, Ricinus communis RC30128G00400, Ricinus communis RC30073G00230, Sorghum bicolor SB01G005340, Sorghum bicolor SB07G005160, Sorghum bicolor SB03G010040, Volvox carteri VC00001G07190, Vitis vinifera VV16G06360, Vitis vinifera VV14G03630, Vitis vinifera VV06G09530, Zea mays ZM05G02360, Zea mays ZM03G07230, Arabidopsis lyrata AL8G08670, Micromonas sp. RCC299 MRCC299_02G03810, Physcomitrella patens PP00509G00110, Selaginella moellendorffii SM00091G00690.

A representative gene for the MKK9 gene from *Arabidopsis thaliana* is AT1G73500 (i.e. the AGI code for the *Arabidopsis* gene). The MKK9 gene encodes for a MAP kinase kinase kinase which is situated downstream of the ethylene receptors. Several orthologous genes for the MKK9 gene can be identified in publically available sequence databases (the database accession numbers are depicted after the species name):
Arabidopsis lyrata AL2G22310, Arabidopsis lyrata AL1G19280, Arabidopsis thaliana AT1G73500, Arabidopsis thaliana AT1G18350, Brachypodium distachyon BD1G10800, Brachypodium distachyon BD1G69400, Carica papaya CP00003G03590, Carica papaya CP01155G00030, Glycine max GM09G30300, Glycine max GM07G11910, Lotus japonicus LJ0G032430, Malus domestica MD06G019470, Malus domestica MD00G332250, Manihot esculenta ME04612G00380, Medicago truncatula MT6G21840, Oryza sativa ssp. japonica OS06G09180, Oryza sativa ssp. japonica OS03G12390, Oryza sativa ssp. indica OSINDICA_02G52870, Oryza sativa ssp. indica OSINDICA_03G10820, Physcomitrella patens PP00114G00510, Physcomitrella patens PP00016G01910, Populus trichocarpa PT15G04450, Populus trichocarpa PT12G03820, Sorghum bicolor SB04G035370, Sorghum bicolor SB01G042350, Selaginella moellendorffii SM00121G00650, Selaginella moellendorffii SM00017G02550, Vitis vinifera VV17G07540, Zea mays ZM05G04760, Zea mays ZM01G07530, Zea mays ZM01G41810, Zea mays ZM03G27150, Zea mays ZM03G27550, Chlamydomonas reinhardtii CR06G00150, Micromonas sp. RCC299 MRCC299_07G06300, Ostreococcus lucimarinus OL04G03760, Ostreococcus tauri OT04G04050, Ricinus communis RC29748G00070, Volvox carteri VC00006G02490.

A representative gene for the MKK7 gene from *Arabidopsis thaliana* is AT1G18350 (i.e. the AGI code for the *Arabidopsis* gene). The MKK7 gene encodes for a MAP kinase kinase kinase which is situated downstream of the ethylene receptors. Several orthologous genes for the MKK7 gene can be identified in publically available sequence databases (the database accession numbers are depicted after the species name):
Arabidopsis thaliana AT1G18350, Arabidopsis thaliana AT1G73500, Brachypodium distachyon BD1G46880, Brachypodium distachyon BD1G10790, Brachypodium distachyon BD1G10770, Oryza sativa ssp. indica OSINDICA_02G52870, Oryza sativa ssp. indica OSINDICA_03G48910, Sorghum bicolor SB01G010180, Zea mays ZM01G07530, Zea mays ZM01G41830, Arabidopsis lyrata AL2G22310, Arabidopsis lyrata AL1G19280, Glycine max GM09G30300, Glycine max GM07G11910, Glycine max GM09G30310, Vitis vinifera VV17G07540, Carica papaya CP00003G03590, Chlamydomonas reinhardtii CR06G00150, Lotus japonicus LJ0G032430, Malus domestica MD06G019470, Malus domestica MD00G332250, Manihot esculenta ME04612G00380, Micromonas sp. RCC299 MRCC299_07G06300, Medicago truncatula MT6G21840, Ostreococcus lucimarinus OL04G03760, Oryza sativa ssp. japonica OS06G09180, Ostreococcus tauri OT04G04050, Physcomitrella patens PP00114G00510, Physcomitrella patens PP00016G01910, Populus trichocarpa PT15G04450, Populus trichocarpa PT12G03820 1, Ricinus communis RC29748G00070, Selaginella moellendorffii SM00110G00220, Selaginella moellendorffii SM00011G01810, Volvox carteri VC00006G02490.

In yet another embodiment the invention provides for a chimeric gene comprising the following operably linked DNA elements: a) a meristem specific transcription factor, b) a DNA region which when transcribed yields a double-stranded RNA molecule capable of reducing the expression of a gene from the following list: ACO1, ACO2, ETR2, ESR1, CTR1, MKK9, MKK7, EIN3, EIN5 or EIL1 and c) a 3' end region comprising transcription termination and polyadenylation signals functioning in cells of said plant.

In yet another embodiment the invention provides for a chimeric gene comprising the following operably linked DNA elements: a) a meristem specific transcription factor, b) a DNA region which when transcribed yields an antisense RNA molecule capable of reducing the expression of a gene from the following list: ACO1, ACO2, ETR2, ESR1, CTR1, MKK9, MKK7, EIN3, EIN5 or EIL1 and c) a 3' end region comprising transcription termination and polyadenylation signals functioning in cells of said plant.

In yet another embodiment the invention provides for a chimeric gene comprising the following operably linked DNA elements: a) a meristem specific transcription factor, b) a DNA region which when transcribed yields an artificial microRNA molecule capable of reducing the expression of a gene from the following list: ACO1, ACO2, ETR2, ESR1, CTR1, MKK9, MKK7, EIN3, EIN5 or EIL1 and c) a 3' end region comprising transcription termination and polyadenylation signals functioning in cells of said plant.

In yet another embodiment the invention provides for a chimeric gene comprising the following operably linked DNA elements: a) a meristem specific promoter, b) a DNA region encoding for a gene of the following list: EBF1, EBF2, ACC deaminase, a dominant negative ethylene receptor or a dominant negative transcription factor EIN3 or EIL1 and c) a 3' end region comprising transcription termination and polyadenylation signals functioning in cells of said plant.

In yet another embodiment the invention provides a method for producing an abiotic stress tolerant plant relative to a control plant, by decreasing the ethylene signal transduction pathway in said plant during the period of abiotic stress imposed on said plants comprising introducing and expressing in said plant a chimeric gene comprising of a promoter with a specificity for proliferating leaf cells selected from the list consisting of SEQ ID NO: 1, SEQ ID NO: 2, SEQ ID NO: 3 or SEQ ID NO: 4, or a functionally equivalent orthologous promoter of SEQ ID NO: 1, SEQ ID NO: 2, SEQ ID NO: 3 or SEQ ID NO: 4 wherein said promoter is operably linked to a gene or gene fragment derived from the ethylene signal transduction pathway.

In yet another embodiment the expression of said chimeric gene in said plant leads to a downregulation of at least one gene of the following list: ACO1, ACO2, ETR2, ETR1, CTR1, MKK9, MKK7, EIN3, EIN5 or EIL1.

In yet another embodiment the expression of said chimeric gene in said plant leads to an upregulation of at least one gene of the following list: EBF1, EBF2, ACC deaminase, a dominant negative ethylene receptor or a dominant negative transcription factor EIN3 or EIL1.

In yet another embodiment the invention provides a chimeric gene comprising the following operably linked DNA elements: a) a promoter with a specificity for proliferating leaf cells selected from the list consisting of SEQ ID NO: 1, SEQ ID NO: 2, SEQ ID NO: 3 or SEQ ID NO: 4, or a functionally equivalent orthologous promoter of SEQ ID NO: 1, SEQ ID NO: 2, SEQ ID NO: 3 or SEQ ID NO: 4, b) a DNA region which when transcribed yields a double-stranded RNA molecule capable of reducing the expression of a gene from the following list: ACO1, ACO2, ETR2, ESR1, CTR1, MKK9, MKK7, EIN3, EIN5 or EIL1 and c) a 3' end region comprising transcription termination and polyadenylation signals functioning in cells of said plant.

In yet another embodiment the invention provides a chimeric gene comprising the following operably linked DNA elements: a) a promoter with a specificity for proliferating leaf cells selected from the list consisting of SEQ ID NO: 1, SEQ ID NO: 2, SEQ ID NO: 3 or SEQ ID NO: 4, or a functionally equivalent orthologous promoter of SEQ ID NO: 1, SEQ ID NO: 2, SEQ ID NO: 3 or SEQ ID NO: 4, b) a DNA region encoding for a gene of the following list: EBF1, EBF2, ACC deaminase, a dominant negative ethylene receptor or a dominant negative transcription factor EIN3 or EIL1 and c) a 3' end region comprising transcription termination and polyadenylation signals functioning in cells of said plant.

In yet another embodiment the invention provides for a transgenic plant or a transgenic seed or a transgenic plant cell comprising a chimeric gene as herein described before.

Particularly preferred transgenic plants, seeds or plant cells of the invention comprise a crop plant or a monocot or a cereal such as rice, wheat, maize, barley, millet, rye, triticale, sorghum emmer, spelt, secale, einkorn and oats.

A "chimeric gene" or "chimeric construct" is a recombinant nucleic acid sequence in which a promoter or regulatory nucleic acid sequence is operatively linked to, or associated with, a nucleic acid sequence that codes for an mRNA, such that the regulatory nucleic acid sequence is able to regulate transcription or expression of the associated nucleic acid coding sequence. The regulatory nucleic acid sequence of the chimeric gene is not normally operatively linked to the associated nucleic acid sequence as found in nature.

In the present invention a "plant promoter" comprises regulatory elements, which mediate the expression of a coding sequence segment in plant cells. For expression in plants, the nucleic acid molecule must be linked operably to or comprise a suitable promoter which expresses the gene at the right point in time and with the required spatial expression pattern. In a preferred aspect the plant promoter of the invention is induced when the plant encounters the abiotic stress. Particularly preferred is a plant promoter active during the cell proliferation phase of the plant. A preferred promoter is a meristem specific promoter. Another preferred promoter is a stress inducible promoter. Most preferred is a stress inducible promoter which is also meristem specific.

The term "operably linked" as used herein refers to a functional linkage between the promoter sequence and the gene of interest, such that the promoter sequence is able to initiate transcription of the gene of interest.

A "constitutive promoter" refers to a promoter that is transcriptionally active during most, but not necessarily all, phases of growth and development and under most environmental conditions, in at least one cell, tissue or organ. In the present invention a constitutive promoter is not preferred because of the pleiotropic effects of ethylene on plant growth.

Non-limiting examples of meristem-specific promoters are the *Arabidopsis thaliana* promoter p4TM1 (derived from the gene AT5G16250) and homologous and orthologous promoters thereof, KNOLLE (derived from the gene AT1G08560) and other plant orthologous promoters thereof; said promoters are strongly induced in mitotic cells. Another promoter is the GRF5 promoter (derived from the gene AT3G13960) or a homologue thereof, the latter promoter is strongly expressed in proliferating leaf primordia. The present invention advantageously shows that the pATM1 promoter, homologous promoters (as further outlined hereafter) and orthologous promoters (as further outlined hereafter) thereof are particularly preferred and advantageous in the present invention.

### 4TM plant promoters

The present invention shows (for example outlined in example 8) that the *Arabidopsis thaliana* promoters derived from the gene family consisting of the *Arabidopsis thaliana* genes At5g16250 (promoter derived from said gene and designated as p4TM1 and depicted in SEQ ID NO: 1), At3G02640 (promoter derived from said gene and designated as p4TM2 and depicted in SEQ ID NO: 2), At5g36710 (promoter derived from said gene and designated as p4TM3a and depicted in SEQ ID NO: 3) and At5g36800 (promoter derived from said gene and designated as p4TM3b and depicted in SEQ ID NO: 4) are particularly advantageous for the construction of chimeric genes which are able to modulate the ethylene signal transduction pathway when transformed and expressed in plants. SEQ ID NO: 1, 2, 3 and 4 are promoters which are strongly active in plant meristematic tissues, particularly active in proliferating leaf cells. The present invention shows that chimeric genes of the invention comprising promoters from the group (SEQ ID NO: 1, 2, 3 and 4) are more advantageous than chimeric genes of the invention comprising other meristematic promoters (such as the GRF5 promoter (depicted in SEQ ID NO: 5) and are also more advantageous than chimeric genes of the invention comprising constitutive promoters (such as the CaMV 35S promoter) for overcoming abiotic stress when these chimeric genes are transformed an expressed in plants.

Several methods are available for the skilled person to isolate orthologous promoter sequences from SEQ ID NO: 1, 2, 3 or 4. One method is an *in silico* method and is based on publically available bioinformatics tools to search for orthologous genes (in the available plant genomic sequence databases) of At5g16250, At3g02640, At5g36710 and/or At5g36800 (annotated genes from which SEQ ID NO: 1, 2, 3 and 4 were derived). Orthologous genes of the latter *Arabidopis thaliana* genes in other plants are: ZM02G11160, ZM03G24300, ZM06G28010, ZM08G30050 and ZM10G19490 derived from Zea *mays*; OSINDICA_01G63710, OSINDICA_04G33930 and OSINDICA_05G38340 from *Oryza sativa ssp. indica;* OS01G67110, OS04G41900 and OS05G43140 from *Oryza sativa ssp. japonica;* PT05G25880, PT08G07570, PT10G17500, PT13G12590 and PT19G11530 derived from *Populus trichocarpa;* SB03G042590, SB06G021400 and SB09G024920 derived from *Sorghum bicolor,* BD2G57580 and BD5G14540 from *Brachypodium distachyon.* The above reference numbers to the orthologous genes were retrieved from the program publically availiable program PLAZA (http://bioinformatics.psb.ugent.be/plaza and the link to retrieve orthologous genes to At5g16250, At3g02640, At5g36710 and/or At5g36800 is: http://bioinformatics.psb.ugent.be/plaza/gene families/view/HOM002837. The above orthologous promoters can be conveniently obtained by isolating 1000 bp, 1500 bp or 2000 bp upstream from the start codon of the sequences of the identified orthologous genes, for which the nucleotide sequence is publically available.

For the identification of functionally equivalent ortologous promoters, the promoter strength and/or expression pattern of a candidate ortologous promoter may be analysed for example by operably linking said promoter (or fragments thereof) to a reporter gene and assaying the expression level and pattern of the reporter gene in various tissues of the plant. Suitable well-known reporter genes include for example beta-glucuronidase or beta-galactosidase. The promoter activity is assayed by measuring the enzymatic activity of the beta-glucuronidase or beta-galactosidase. The promoter strength and/or expression pattern in meristematic tissues may then be compared to that of a reference promoter (such as the one used in the examples of the present invention). Alternatively, promoter strength may be assayed by quantifying mRNA levels or by comparing mRNA levels of the nucleic acid used in the methods of the present invention, with mRNA levels of housekeeping genes such as 18S rRNA, using methods known in the art, such as Northern blotting with densitometric analysis of autoradiograms, quantitative real-time PCR or RT- PCR (Heid et al., 1996 Genome Methods 6: 986-994). Generally by "weak promoter" is intended a promoter that drives expression of a coding sequence at a low level. By "low level" is intended at levels of about 1/10,000 transcripts to about 1/100,000 transcripts, to about 1/500,0000 transcripts per cell. Conversely, a "strong promoter" drives expression of a coding sequence at high level, or at about 1/10 transcripts to about 1/100 transcripts to about 1/1000 transcripts per cell. Generally, by "medium strength promoter" is intended a promoter that drives expression of a coding sequence at a lower level than a strong promoter, in particular at a level that is in all instances below that obtained when under the control of a 35S CaMV promoter.

In addition, equivalent (or orthologous) promoters of SEQ ID NO: 1, 2, 3 and 4 can be isolated from other plants by hybridization. To this end, orthologous promoter fragments may be isolated from other plants using SEQ ID NO: 1, 2, 3 or 4 or a functional fragment having at least 50 consecutive nucleotides thereof as a probe and identifying nucleotide sequences from these other plants which hybridize under the herein described hybridization conditions. By way of example, a promoter of the invention may be used to screen a genomic library of a crop or plant of interest to isolate corresponding promoter sequences according to techniques well known in the art. Thus, a promoter sequence of the invention may be used as a probe for hybridization with a genomic library under medium to high stringency conditions. As an alternative equivalent promoters can be isolated using the coding sequences of At5g16250, At3g02640, At5g36710 and/or At5g36800 to screen a genomic library (e.g. by hybridization) of a crop of interest. When sufficient identity between the coding sequences is obtained (as a rule higher than 85% identity) then promoter regions can be isolated upstream of the orthologous At5g16250, At3g02640, At5g36710 and/or At5g36800 genes. The term "hybridization" refers to the ability of a first strand of nucleic acid to join with a second strand via hydrogen bond base pairing when the two nucleic acid strands have sufficient sequence identity. Hybridization occurs when the two nucleic acid molecules anneal to one another under appropriate conditions. Nucleic acid hybridization is a technique well known to those of skill in the art of DNA manipulation. The hybridization property of a given pair of nucleic acids is an indication of their similarity or identity. Another indication that two nucleic acid sequences are substantially identical is that the two molecules hybridize to each other under stringent conditions. The phrase "hybridizing specifically to" refers to the binding, duplexing, or hybridizing of a molecule only to a particular nucleotide sequence under stringent conditions when that sequence is present in a complex mixture (e.g., total cellular) DNA or RNA. "Bind(s) substantially" refers to complementary hybridization between a probe nucleic acid and a target nucleic acid and embraces minor mismatches that can be accommodated by reducing the stringency of the hybridization media to achieve the desired detection of the target nucleic acid sequence. "Stringent hybridization conditions" and "stringent hybridization wash conditions" in the context of nucleic acid hybridization experiments such as Southern and Northern hybridization are sequence dependent, and are different under different environmental parameters. An example of highly stringent wash conditions is 0.15 M NaCl at 72°C for about 15 minutes. An example of stringent wash conditions is a 0.2 X SSC wash at 65°C for 15 minutes. Often, a high stringency wash is preceded by a low stringency wash to remove background probe signal. An example medium stringency wash for a duplex of, e.g., more than 100 nucleotides, is 1 X SSC at 45°C for 15 minutes. An example low stringency wash for a duplex of, e.g., more than 100 nucleotides, is 4 to 6 X SSC at 40°C for 15 minutes. For short probes (e.g., about 10 to 50 nucleotides), stringent conditions typically involve salt concentrations of less than about 1.5 M, more preferably about 0.01 to 1.0 M, Na ion concentration (or other salts) at pH 7.0 to 8.3, and the temperature is typically at least about 30°C and at least about 60°C for long probes (e.g., >50 nucleotides). Stringent conditions may also be achieved with the addition of destabilizing agents such as formamide. In general, a signal to noise ratio of 2 X (or higher) than that observed for an unrelated probe in the particular hybridization assay indicates detection of a specific hybridization. Nucleic acids that do not hybridize to each other under stringent conditions are still substantially identical if the proteins that they encode are substantially identical. This occurs, e.g., when a copy of a nucleic acid is created using the maximum codon degeneracy permitted by the genetic code. Very stringent conditions are selected to be equal to the Tₘ for a particular probe. An example of stringent conditions for hybridization of complementary nucleic acids which have more than 100 complementary residues on a filter in a Southern or Northern blot is 50% formamide, e.g., hybridization in 50% formamide, 1 M NaCl, 1% SDS at 37°C, and a wash in 0.1 x SSC at 60 to 65°C. Exemplary low stringency conditions include hybridization with a buffer solution of 30 to 35% formamide, 1 M NaCl, 1% SDS (sodium dodecyl sulphate) at 37°C, and a wash in 1 X to 2 X SSC (20 X SSC=3.0 M NaCl/0.3 M trisodium citrate) at 50 to 55°C. Exemplary moderate stringency conditions include hybridization in 40 to 45% formamide, 1.0 M NaCl, 1% SDS at 37°C, and a wash in 0.5 X to 1 X SSC at 55 to 60°C. The following are examples of sets of hybridization/wash conditions that may be used to clone orthologous nucleotide sequences that are substantially identical to reference nucleotide sequences of the present invention: a reference nucleotide sequence preferably hybridizes to the reference nucleotide sequence in 7% sodium dodecyl sulfate (SDS), 0.5 M NaPO₄, 1 mM EDTA at 50°C with washing in 2 X SSC, 0. 1% SDS at 50°C, more desirably in 7% sodium dodecyl sulfate (SDS), 0.5 M NaPO₄, 1 mM EDTA at 50°C with washing in 1 X SSC, 0.1% SDS at 50°C, more desirably still in 7% sodium dodecyl sulfate (SDS), 0.5 M NaPO₄, 1 mM EDTA at 50°C with washing in 0.5 X SSC, 0. 1% SDS at 50°C, even more desirably still in 7% sodium dodecyl sulfate (SDS), 0.5 M NaPO₄, 1 mM EDTA at 50°C with washing in 0.1 X SSC, 0.1% SDS at 50°C.

In another embodiment of the present invention meristematic promoters are provided which comprise a nucleotide sequence having at least 40%, at least 50%, or at least 60%, or at least 70%, or at least 80%, or at least 90%, or at least 95% sequence identity to SEQ ID NOI: 1, 2, 3 or 4. The term "variant" with respect to the transcription regulating nucleotide sequences SEQ ID NO: 1, 2, 3 and 4 of the invention is intended to mean substantially similar sequences. Naturally occurring allelic variants such as these can be identified with the use of well-known molecular biology techniques, as, for example, with polymerase chain reaction (PCR) and hybridization techniques as herein outlined before. Variant nucleotide sequences also include synthetically derived nucleotide sequences, such as those generated, for example, by using site-directed mutagenesis of SEQ ID NO: 1, 2, 3 or 4. Generally, nucleotide sequence variants of the invention will have at least 40%, 50%, 60%, to 70%, e.g., preferably 71%, 72%, 73%, 74%, 75%, 76%, 77%, 78%, to 79%, generally at least 80%, e.g., 81% to 84%, at least 85%, e.g., 86%, 87%, 88%, 89%, 90%, 91%, 92%, 93%, 94%, 95%, 96%, 97%, to 98% and 99% nucleotide sequence identity to the native (wild type or endogenous) nucleotide sequence. Derivatives of the DNA molecules disclosed herein may include, but are not limited to, deletions of sequence, single or multiple point mutations, alterations at a particular restriction enzyme site, addition of functional elements, or other means of molecular modification which may enhance, or otherwise alter promoter expression. Techniques for obtaining such derivatives are well-known in the art (see, for example, J. F. Sambrook, D. W. Russell, and N. Irwin (2000) Molecular Cloning: A Laboratory Manual, 3rd edition Volumes 1 , 2, and 3. Cold Spring Harbor Laboratory Press). For example, one of ordinary skill in the art may delimit the functional elements within the promoters disclosed herein and delete any non-essential elements. Functional elements may be modified or combined to increase the utility or expression of the sequences of the invention for any particular application. Those of skill in the art are familiar with the standard resource materials that describe specific conditions and procedures for the construction, manipulation, and isolation of macromolecules (e.g., DNA molecules, plasmids, etc.), as well as the generation of recombinant organisms and the screening and isolation of DNA molecules. As used herein, the term "percent sequence identity" refers to the percentage of identical nucleotides between two segments of a window of optimally aligned DNA. Optimal alignment of sequences for aligning a comparison window are well-known to those skilled in the art and may be conducted by tools such as the local homology algorithm of Smith and Waterman (Waterman, M. S. Introduction to Computational Biology: Maps, sequences and genomes. Chapman & Hall. London (1995), the homology alignment algorithm of Needleman and Wunsch (J. Mol. Biol., 48:443-453 (1970), the search for similarity method of Pearson and Lipman (Proc. Natl. Acad. ScL, 85:2444 (1988), and preferably by computerized implementations of these algorithms such as GAP, BESTFIT, FASTA, and TFASTA available as part of the GCG (Registered Trade Mark), Wisconsin Package (Registered Trade Mark from Accelrys Inc., San Diego, Calif.). An "identity fraction" for aligned segments of a test sequence and a reference sequence is the number of identical components that are shared by the two aligned sequences divided by the total number of components in the reference sequence segment, i.e., the entire reference sequence or a smaller defined part of the reference sequence. Percent sequence identity is represented as the identity fraction times 100. The comparison of one or more DNA sequences may be to a full-length DNA sequence or a portion thereof, or to a longer DNA sequence.

The promoters of the present invention (SEQ ID NO: 1, 2, 3 and 4) may be operably linked to a nucleic acid sequence that is heterologous with respect to the promoter. The nucleic acid sequence may generally be any nucleic acid sequence for which an increased level or altered level (e.g. in a different organ) of transcription is desired in a meristematic tissue, in particular a leaf meristematic tissue. The nucleic acid sequence can for example encode a polypeptide that is suitable for incorporation into the diet of a human or an animal or can provide some other agricultural or industrial important feature. Suitable heterologous nucleic acid sequences include, without limitation, those encoding fatty acid pathway enzymes, epoxidases, hydroxylases, cytochrome P450 mono-oxygenases, desaturases, tocopherol biosynthetic enzymes, carotenoid biosynthesis enzymes, amino acid biosynthetic enzymes, steroid pathway enzymes, and starch branching enzymes.

Thus in another particular embodiment the invention provides an expression cassette for regulating meristeme specific expression in plants comprising a promoter operably linked to a nucleic acid which is heterologous in relation to said promoter and wherein said promoter is selected from the group consisting of a) SEQ ID NO: 1, SEQ ID NO: 2, SEQ ID NO: 3 or SEQ ID NO: 4, b) a functional fragment of at least 50 consecutive base pairs of SEQ ID NO: 1, SEQ ID NO: 2, SEQ ID NO: 3 or SEQ ID NO: 4 having promoter activity, and (c) a nucleotide sequence hybridizing under conditions equivalent to hybridization in 7% sodium dodecyl sulfate, 0.5 M NaPO₄, 1mM EDTA at 50°C with washing in 2XSSC, 0.1% SDS at 50°C to a nucleotide sequence depicted in SEQ ID NO: 1 or SEQ ID NO: 2 or SEQ ID NO: 3 or SEQ ID NO: 4 or a fragment of at least 50 consecutive bases of SEQ ID NO: 1 , SEQ ID NO: 2, SEQ ID NO: 3 or SEQ ID NO: 4 or the complement thereof.

In a particular embodiment the expression cassette when transformed in a plant expresses a nucleic acid which results in expression of a protein, or expression of an antisense RNA, sense or double stranded RNA.

In yet another embodiment the invention provides a recombinant vector which comprises an expression cassette as herein described before.

The term "terminator" encompasses a control sequence which is a DNA sequence at the end of a transcriptional unit which signals 3' processing and polyadenylation of a primary transcript and termination of transcription. The terminator can be derived from the natural gene, from a variety of other plant genes, or from T-DNA. The terminator to be added may be derived from, for example, the nopaline synthase or octopine synthase genes, or alternatively from another plant gene, or less preferably from any other eukaryotic gene.

"Selectable marker", "selectable marker gene" or "reporter gene" includes any gene that confers a phenotype on a cell in which it is expressed to facilitate the identification and/or selection of cells that are transfected or transformed with a nucleic acid construct of the invention. These marker genes enable the identification of a successful transfer of the nucleic acid molecules via a series of different principles. Suitable markers may be selected from markers that confer antibiotic or herbicide resistance, that introduce a new metabolic trait or that allow visual selection. Examples of selectable marker genes include genes conferring resistance to antibiotics (such as nptll that phosphorylates neomycin and kanamycin, or hpt, phosphorylating hygromycin, or genes conferring resistance to, for example, bleomycin, streptomycin, tetracyclin, chloramphenicol, ampicillin, gentamycin, geneticin (G418), spectinomycin or blasticidin), to herbicides (for example bar which provides resistance to Basta^{®}; aroA or gox providing resistance against glyphosate, or the genes conferring resistance to, for example, imidazolinone, phosphinothricin or sulfonylurea), or genes that provide a metabolic trait (such as manA that allows plants to use mannose as sole carbon source or xylose isomerase for the utilisation of xylose, or antinutritive markers such as the resistance to 2-deoxyglucose). Expression of visual marker genes results in the formation of colour (for example β-glucuronidase, GUS or β-galactosidase with its coloured substrates, for example X-Gal), luminescence (such as the luciferin/luceferase system) or fluorescence (Green Fluorescent Protein, GFP, and derivatives thereof). This list represents only a small number of possible markers. The skilled worker is familiar with such markers. Different markers are preferred, depending on the organism and the selection method.

It is known that upon stable or transient integration of nucleic acids into plant cells, only a minority of the cells takes up the foreign DNA and, if desired, integrates it into its genome, depending on the expression vector used and the transfection technique used. To identify and select these integrants, a gene coding for a selectable marker (such as the ones described above) is usually introduced into the host cells together with the gene of interest. These markers can for example be used in mutants in which these genes are not functional by, for example, deletion by conventional methods. Furthermore, nucleic acid molecules encoding a selectable marker can be introduced into a host cell on the same vector that comprises the sequence encoding the polypeptides of the invention or used in the methods of the invention, or else in a separate vector. Cells which have been stably transfected with the introduced nucleic acid can be identified for example by selection (for example, cells which have integrated the selectable marker survive whereas the other cells die).

Since the marker genes, particularly genes for resistance to antibiotics and herbicides, are no longer required or are undesired in the transgenic host cell once the nucleic acids have been introduced successfully, the process according to the invention for introducing the nucleic acids advantageously employs techniques which enable the removal or excision of these marker genes. One such a method is what is known as co-transformation. The co-transformation method employs two vectors simultaneously for the transformation, one vector bearing the nucleic acid according to the invention and a second bearing the marker gene(s). A large proportion of transformants receives or, in the case of plants, comprises (up to 40% or more of the transformants), both vectors. In case of transformation with Agrobacteria, the transformants usually receive only a part of the vector, i.e. the sequence flanked by the T-DNA, which usually represents the expression cassette. The marker genes can subsequently be removed from the transformed plant by performing crosses. In another method, marker genes integrated into a transposon are used for the transformation together with desired nucleic acid (known as the Ac/Ds technology). The transformants can be crossed with a transposase source or the transformants are transformed with a nucleic acid construct conferring expression of a transposase, transiently or stable. In some cases (approx. 10%), the transposon jumps out of the genome of the host cell once transformation has taken place successfully and is lost. In a further number of cases, the transposon jumps to a different location. In these cases the marker gene must be eliminated by performing crosses. In microbiology, techniques were developed which make possible, or facilitate, the detection of such events. A further advantageous method relies on what is known as recombination systems; whose advantage is that elimination by crossing can be dispensed with. The best-known system of this type is what is known as the Cre/lox system. Cre1 is a recombinase that removes the sequences located between the loxP sequences. If the marker gene is integrated between the loxP sequences, it is removed once transformation has taken place successfully, by expression of the recombinase. Further recombination systems are the HIN/HIX, FLP/FRT and REP/STB system (Tribble et al., J. Biol. Chem., 275, 2000: 22255-22267; Velmurugan et al., J. Cell Biol., 149, 2000: 553-566). A site-specific integration into the plant genome of the nucleic acid sequences according to the invention is possible.

For the purposes of the invention, "transgenic", "transgene" or "recombinant" means with regard to, for example, a nucleic acid sequence, an expression cassette, gene construct or a vector comprising the nucleic acid sequence or an organism transformed with the nucleic acid sequences, expression cassettes or vectors according to the invention.

A transgenic plant for the purposes of the invention is thus understood as meaning, as above, that the nucleic acids used in the method of the invention are not present in, or originating from, the genome of said plant, or are present in the genome of said plant but not at their natural locus in the genome of said plant, it being possible for the nucleic acids to be expressed homologously or heterologously. However, as mentioned, transgenic also means that, while the nucleic acids according to the invention or used in the inventive method are at their natural position in the genome of a plant, the sequence has been modified with regard to the natural sequence, and/or that the regulatory sequences of the natural sequences have been modified. Transgenic is preferably understood as meaning the expression of the nucleic acids according to the invention at an unnatural locus in the genome, i.e. homologous or, heterologous expression of the nucleic acids takes place. Preferred transgenic plants are mentioned herein.

For the purpose of this invention related or orthologous genes of the ethylene signal transduction pathway as described herein before can be isolated from the (publically) available sequence databases. The "sequence identity" of two related nucleotide or amino acid sequences, expressed as a percentage, refers to the number of positions in the two optimally aligned sequences which have identical residues (x100) divided by the number of positions compared. A gap, i.e., a position in an alignment where a residue is present in one sequence but not in the other is regarded as a position with non-identical residues. The alignment of the two sequences is performed by the Needleman and Wunsch algorithm (Needleman and Wunsch (1970) J Mol Biol. 48: 443-453) The computer-assisted sequence alignment above, can be conveniently performed using standard software program such as GAP which is part of the Wisconsin Package Version 10.1 (Genetics Computer Group, Madision, Wisconsin, USA) using the default scoring matrix with a gap creation penalty of 50 and a gap extension penalty of 3. Sequences are indicated as "essentially similar" when such sequence have a sequence identity of at least about 75%, particularly at least about 80 %, more particularly at least about 85%, quite particularly about 90%, especially about 95%, more especially about 100%, quite especially are identical. It is clear than when RNA sequences are the to be essentially similar or have a certain degree of sequence identity with DNA sequences, thymine (T) in the DNA sequence is considered equal to uracil (U) in the RNA sequence.

Alternatively the skilled person can isolate orthologous plant genes involved in the ethylene signal transduction pathway through methods of genetic hybridization. Such methods are well known to the skilled (plant) molecular biologist.

The term "modulation" means in relation to expression or gene expression, a process in which the expression level is changed by said gene expression in comparison to the control plant, the expression level may be increased or decreased. The original, unmodulated expression may be of any kind of expression of a structural RNA (rRNA, tRNA) or mRNA with subsequent translation. For the purposes of this invention, the original unmodulated expression may also be absence of any expression. The term "modulating the activity" shall mean any change of the expression of the inventive nucleic acid sequences or encoded proteins, which leads to increased yield and/or increased growth of the plants. The expression can increase from zero (absence of, or immeasurable expression) to a certain amount, or can decrease from a certain amount to immeasurable small amounts or zero.

The term "expression" or "gene expression" means the transcription of a specific gene or specific genes or specific genetic construct. The term "expression" or "gene expression" in particular means the transcription of a gene or genes or genetic construct into structural RNA (rRNA, tRNA) or mRNA with or without subsequent translation of the latter into a protein. The process includes transcription of DNA and processing of the resulting mRNA product.

The term "increased expression" or "overexpression" as used herein means any form of expression that is additional to the original wild-type expression level. For the purposes of this invention, the original wild-type expression level might also be zero, i.e. absence of expression or immeasurable expression.

Methods for increasing expression of genes or gene products are well documented in the art and include, for example, overexpression driven by appropriate promoters (as described herein before), the use of transcription enhancers or translation enhancers. Isolated nucleic acids which serve as promoter or enhancer elements may be introduced in an appropriate position (typically upstream) of a non-heterologous form of a polynucleotide so as to upregulate expression of a nucleic acid encoding the polypeptide of interest. If polypeptide expression is desired, it is generally desirable to include a polyadenylation region at the 3'-end of a polynucleotide coding region. The polyadenylation region can be derived from the natural gene, from a variety of other plant genes, or from T-DNA. The 3' end sequence to be added may be derived from, for example, the nopaline synthase or octopine synthase genes, or alternatively from another plant gene, or less preferably from any other eukaryotic gene.

An intron sequence may also be added to the 5' untranslated region (UTR) or the coding sequence of the partial coding sequence to increase the amount of the mature message that accumulates in the cytosol. Inclusion of a spliceable intron in the transcription unit in both plant and animal expression constructs has been shown to increase gene expression at both the mRNA and protein levels up to 1000-fold (Buchman and Berg (1988) Mol. Cell biol. 8: 4395-4405; Callis et al. (1987) Genes Dev 1 :1 183-1200). Such intron enhancement of gene expression is typically greatest when placed near the 5' end of the transcription unit. Use of the maize introns Adh1 -S intron 1, 2, and 6, the Bronze-1 intron are known in the art. For general information see: The Maize Handbook, Chapter 1 16, Freeling and Walbot, Eds., Springer, N.Y. (1994).

### Strategies for downregulation (or decreased expression) of genes from the list consisting of ACO1, ACO2, ETR2, ETR1, CTR1, MKK7, MKK9, EIN3, EIN5 and EIL1.

Reference herein to "decreased expression" or "reduction or substantial elimination" of expression is taken to mean a decrease in endogenous gene expression and/or polypeptide levels and/or polypeptide activity relative to control plants. The reduction or substantial elimination is in increasing order of preference at least 10%, 20%, 30%, 40% or 50%, 60%, 70%, 80%, 85%, 90%, or 95%, 96%, 97%, 98%, 99% or more reduced compared to that of control plants.

For the reduction or substantial elimination of expression an endogenous gene in a plant, preferentially in the meristematic tissue, more preferentially in a meristematic tissue during the period of (mild) abiotic stress, a sufficient length of substantially contiguous nucleotides of a nucleic acid sequence is required. In order to perform gene silencing, this may be as little as 20, 19, 18, 17, 16, 15, 14, 13, 12, 1 1 , 10 or fewer nucleotides, alternatively this may be as much as the entire gene (including the 5' and/or 3' UTR, either in part or in whole). The stretch of substantially contiguous nucleotides may be derived from the nucleic acid encoding the protein of interest (target gene), or from any nucleic acid capable of encoding an orthologue, paralogue or homologue of the protein of interest. Preferably, the stretch of substantially contiguous nucleotides is capable of forming hydrogen bonds with the target gene (either sense or antisense strand), more preferably, the stretch of substantially contiguous nucleotides has, in increasing order of preference, 50%, 60%, 70%, 80%, 85%, 90%, 95%, 96%, 97%, 98%, 99%, 100% sequence identity to the target gene (either sense or antisense strand). A nucleic acid sequence encoding a (functional) polypeptide is not a requirement for the various methods discussed herein for the reduction or substantial elimination of expression of an endogenous gene.

This reduction or substantial elimination of expression may be achieved using routine tools and techniques. A preferred method for the reduction or substantial elimination of endogenous gene expression is by introducing and expressing in a plant a genetic construct into which the nucleic acid (in this case a stretch of substantially contiguous nucleotides derived from the gene of interest, or from any nucleic acid capable of encoding an orthologue, paralogue or homologue of any one of the protein of interest) is cloned as an inverted repeat (in part or completely), separated by a spacer (non-coding DNA).

In such a preferred method, expression of the endogenous gene is reduced or substantially eliminated through RNA-mediated silencing using an inverted repeat of a nucleic acid or a part thereof (in this case a stretch of substantially contiguous nucleotides derived from the gene of interest, or from any nucleic acid capable of encoding an orthologue, paralogue or homologue of the protein of interest), preferably capable of forming a hairpin structure. The inverted repeat is cloned in an expression vector comprising control sequences. A non- coding DNA nucleic acid sequence (a spacer, for example a matrix attachment region fragment (MAR), an intron, a polylinker, etc.) is located between the two inverted nucleic acids forming the inverted repeat. After transcription of the inverted repeat, a chimeric RNA with a self-complementary structure is formed (partial or complete). This double-stranded RNA structure is referred to as the hairpin RNA (hpRNA). The hpRNA is processed by the plant into siRNAs that are incorporated into an RNA-induced silencing complex (RISC). The RISC further cleaves the mRNA transcripts, thereby substantially reducing the number of mRNA transcripts to be translated into polypeptides. For further general details see for example, Grierson et al. (1998) WO 98/53083; Waterhouse et al. (1999) WO 99/53050).

Performance of the methods of the invention does not rely on introducing and expressing in a plant a genetic construct into which the nucleic acid is cloned as an inverted repeat, but any one or more of several well-known "gene silencing" methods may be used to achieve the same effects.

One such method for the reduction of endogenous gene expression is RNA-mediated silencing of gene expression (downregulation). Silencing in this case is triggered in a plant by a double stranded RNA sequence (dsRNA) that is substantially similar to the target endogenous gene. This dsRNA is further processed by the plant into about 20 to about 26 nucleotides called short interfering RNAs (siRNAs). The siRNAs are incorporated into an RNA-induced silencing complex (RISC) that cleaves the mRNA transcript of the endogenous target gene, thereby substantially reducing the number of mRNA transcripts to be translated into a polypeptide. Preferably, the double stranded RNA sequence corresponds to a target gene.

Another example of an RNA silencing method involves the introduction of nucleic acid sequences or parts thereof (in this case a stretch of substantially contiguous nucleotides derived from the gene of interest, or from any nucleic acid capable of encoding an orthologue, paralogue or homologue of the protein of interest) in a sense orientation into a plant. "Sense orientation" refers to a DNA sequence that is homologous to an mRNA transcript thereof. Introduced into a plant would therefore be at least one copy of the nucleic acid sequence. The additional nucleic acid sequence will reduce expression of the endogenous gene, giving rise to a phenomenon known as co-suppression. The reduction of gene expression will be more pronounced if several additional copies of a nucleic acid sequence are introduced into the plant, as there is a positive correlation between high transcript levels and the triggering of co-suppression.

Another example of an RNA silencing method involves the use of antisense nucleic acid sequences. An "antisense" nucleic acid sequence comprises a nucleotide sequence that is complementary to a "sense" nucleic acid sequence encoding a protein, i.e. complementary to the coding strand of a double-stranded cDNA molecule or complementary to an mRNA transcript sequence. The antisense nucleic acid sequence is preferably complementary to the endogenous gene to be silenced. The complementarity may be located in the "coding region" and/or in the "non-coding region" of a gene. The term "coding region" refers to a region of the nucleotide sequence comprising codons that are translated into amino acid residues. The term "non-coding region" refers to 5' and 3' sequences that flank the coding region that are transcribed but not translated into amino acids (also referred to as 5' and 3' untranslated regions).

Antisense nucleic acid sequences can be designed according to the rules of Watson and Crick base pairing. The antisense nucleic acid sequence may be complementary to the entire nucleic acid sequence (in this case a stretch of substantially contiguous nucleotides derived from the gene of interest, or from any nucleic acid capable of encoding an orthologue, paralogue or homologue of the protein of interest), but may also be an oligonucleotide that is antisense to only a part of the nucleic acid sequence (including the mRNA 5' and 3' UTR). For example, the antisense oligonucleotide sequence may be complementary to the region surrounding the translation start site of an mRNA transcript encoding a polypeptide. The length of a suitable antisense oligonucleotide sequence is known in the art and may start from about 50, 45, 40, 35, 30, 25, 20, 15 or 10 nucleotides in length or less. The antisense nucleic acid sequence can be produced in the plant cell using an expression vector (comprising a chimeric gene of the invention) into which a nucleic acid sequence has been subcloned in an antisense orientation (i.e., RNA transcribed from the inserted nucleic acid will be of an antisense orientation to a target nucleic acid of interest). Preferably, production of antisense nucleic acid sequences in plants occurs by means of a stably integrated nucleic acid construct comprising a promoter, an operably linked antisense oligonucleotide, and a terminator.

The nucleic acid molecules used for silencing in the methods of the invention (whether introduced into a plant or generated in situ) hybridize with or bind to mRNA transcripts and/or genomic DNA encoding a polypeptide to thereby inhibit expression of the protein, e.g., by inhibiting transcription and/or translation. The hybridization can be by conventional nucleotide complementarity to form a stable duplex, or, for example, in the case of an antisense nucleic acid sequence which binds to DNA duplexes, through specific interactions in the major groove of the double helix. Antisense nucleic acid sequences may be introduced into a plant by transformation or direct injection at a specific tissue site.

The reduction or substantial elimination of endogenous gene expression may also be performed using ribozymes. Ribozymes are catalytic RNA molecules with ribonuclease activity that are capable of cleaving a single-stranded nucleic acid sequence, such as an mRNA, to which they have a complementary region. Thus, ribozymes (e.g., hammerhead ribozymes (described in Haselhoff and Gerlach (1988) Nature 334, 585-591) can be used to catalytically cleave mRNA transcripts encoding a polypeptide, thereby substantially reducing the number of mRNA transcripts to be translated into a polypeptide. A ribozyme having specificity for a nucleic acid sequence can be designed (see for example: Cech et al. U.S. Patent No. 4,987,071 ; and Cech et al. U.S. Patent No. 5,116,742). Alternatively, mRNA transcripts corresponding to a nucleic acid sequence can be used to select a catalytic RNA having a specific ribonuclease activity from a pool of RNA molecules (Bartel and Szostak (1993) Science 261, 141 1 -1418). The use of ribozymes for gene silencing in plants is known in the art (e.g., Atkins et al. (1994) WO 94/00012; Lenne et al. (1995) WO 95/03404; Lutziger et al. (2000) WO 00/00619; Prinsen et al. (1997) WO 97/13865 and Scott et al. (1997) WO 97/38116).

A further approach to gene silencing is by targeting nucleic acid sequences complementary to the regulatory region of the gene (e.g., the promoter and/or enhancers) to form triple helical structures that prevent transcription of the gene in target cells. See Helene, C, Anticancer Drug Res. 6, 569-84, 1991 ; Helene et al., Ann. N.Y. Acad. Sci. 660, 27-36 1992; and Maher, L.J. Bioassays 14, 807-15, 1992.

Other methods, such as the use of antibodies directed to an endogenous polypeptide for inhibiting its function in planta, or interference in the signalling pathway in which a polypeptide is involved, will be well known to the skilled man. In particular, it can be envisaged that manmade molecules may be useful for inhibiting the biological function of a target polypeptide, or for interfering with the signalling pathway in which the target polypeptide is involved.

Artificial and/or natural microRNAs (miRNAs) may be used to knock out gene expression and/or mRNA translation. Endogenous miRNAs are single stranded small RNAs of typically 19-24 nucleotides long. They function primarily to regulate gene expression and/ or mRNA translation. Most plant microRNAs (miRNAs) have perfect or near-perfect complementarity with their target sequences. However, there are natural targets with up to five mismatches. They are processed from longer non-coding RNAs with characteristic fold-back structures by double-strand specific RNases of the Dicer family. Upon processing, they are incorporated in the RNA-induced silencing complex (RISC) by binding to its main component, an Argonaute protein. MiRNAs serve as the specificity components of RISC, since they base-pair to target nucleic acids, mostly mRNAs, in the cytoplasm. Subsequent regulatory events include target mRNA cleavage and destruction and/or translational inhibition. Effects of miRNA overexpression are thus often reflected in decreased mRNA levels of target genes.

Artificial microRNAs (amiRNAs), which are typically 21 nucleotides in length, can be genetically engineered specifically to negatively regulate gene expression of single or multiple genes of interest. Determinants of plant microRNA target selection are well known in the art. Empirical parameters for target recognition have been defined and can be used to aid in the design of specific amiRNAs, (Schwab et al., Dev. Cell 8, 517-527, 2005). Convenient tools for design and generation of amiRNAs and their precursors are also available to the public (Schwab et al., Plant Cell 18, 1121 -1 133,2006).

For optimal performance, the gene silencing techniques used for reducing expression in a plant of an endogenous gene requires the use of nucleic acid sequences from monocotyledonous plants for transformation of monocotyledonous plants, and from dicotyledonous plants for transformation of dicotyledonous plants. Preferably, a nucleic acid sequence from any given plant species is introduced into that same species. For example, a nucleic acid sequence from rice is transformed into a rice plant. However, it is not an absolute requirement that the nucleic acid sequence to be introduced originates from the same plant species as the plant in which it will be introduced. It is sufficient that there is substantial homology between the endogenous target gene and the nucleic acid to be introduced.

In yet another embodiment protein interference as described in the patent application WO2007071789 (means and methods for mediating protein interference) can be used to downregulate a gene product. The latter technology is a knock-down technology which in contrast to RNAi acts at the post-translational level (i.e. it works directly on the protein level by inducing a specific protein aggregation of a chosen target). Protein aggregation is essentially a misfolding event which occurs through the formation of intermolecular beta-sheets resulting in a functional knockout of a selected target. Through the use of a dedicated algorithm it is possible to accurately predict which amino acidic stretches in a chosen target protein sequence have the highest self-associating tendency (Fernandez-Escamilla A. M. et al (2004) Nat Biotechnol 22(10): 1302-6. By expressing these specific peptides in the cells the protein of interest can be specifically targeted by inducing its irreversible aggregation and thus its functional knock-out.

Described above are examples of various methods for the reduction or substantial elimination of expression in a plant of an endogenous gene or an endogenous gene product. A person skilled in the art would readily be able to adapt the aforementioned methods for silencing so as to achieve reduction of expression of an endogenous gene in a whole plant or in parts thereof through the use of an appropriate promoter, for example.

The term "introduction" or "transformation" as referred to herein encompass the transfer of an exogenous polynucleotide into a host cell, irrespective of the method used for transfer. Plant tissue capable of subsequent clonal propagation, whether by organogenesis or embryogenesis, may be transformed with a genetic construct of the present invention and a whole plant regenerated there from. The particular tissue chosen will vary depending on the clonal propagation systems available for, and best suited to, the particular species being transformed. Exemplary tissue targets include leaf disks, pollen, embryos, cotyledons, hypocotyls, megagametophytes, callus tissue, existing meristematic tissue (e.g., apical meristem, axillary buds, and root meristems), and induced meristem tissue (e.g., cotyledon meristem and hypocotyl meristem). The polynucleotide may be transiently or stably introduced into a host cell and may be maintained non-integrated, for example, as a plasmid. Alternatively, it may be integrated into the host genome. The resulting transformed plant cell may then be used to regenerate a transformed plant in a manner known to persons skilled in the art.

The transfer of foreign genes into the genome of a plant is called transformation. Transformation of plant species is now a fairly routine technique. Advantageously, any of several transformation methods may be used to introduce the gene of interest into a suitable ancestor cell. The methods described for the transformation and regeneration of plants from plant tissues or plant cells may be utilized for transient or for stable transformation. Transformation methods include the use of liposomes, electroporation, chemicals that increase free DNA uptake, injection of the DNA directly into the plant, particle gun bombardment, transformation using viruses or pollen and microprojection. Methods may be selected from the calcium/polyethylene glycol method for protoplasts (Krens, F.A. et al., (1982) Nature 296, 72-74; Negrutiu I et al. (1987) Plant Mol Biol 8: 363- 373); electroporation of protoplasts (Shillito R.D. et al. (1985) Bio/Technol 3, 1099-1 102); microinjection into plant material (Crossway A et al., (1986) Mol. Gen Genet 202: 179-185); DNA or RNA-coated particle bombardment (Klein TM et al., (1987) Nature 327: 70) infection with (non-integrative) viruses and the like. Transgenic plants, including transgenic crop plants, are preferably produced via Agrobacterium-mediated transformation. An advantageous transformation method is the transformation in planta. To this end, it is possible, for example, to allow the agrobacteria to act on plant seeds or to inoculate the plant meristem with agrobacteria. It has proved particularly expedient in accordance with the invention to allow a suspension of transformed agrobacteria to act on the intact plant or at least on the flower primordia. The plant is subsequently grown on until the seeds of the treated plant are obtained (Clough and Bent, Plant J. (1998) 16, 735-743). Methods for Agrobacterium-mediated transformation of rice include well known methods for rice transformation, such as those described in any of the following: European patent application EP1198985, Aldemita and Hodges (Planta 199: 612-617, 1996); Chan et al. (Plant Mol Biol 22 (3): 491 -506, 1993), Hiei et al. (Plant J 6 (2): 271 -282, 1994), In the case of corn transformation, the preferred method is as described in either Ishida et al. (Nat. Biotechnol 14(6): 745-50, 1996) or Frame et al. (Plant Physiol 129(1): 13-22, 2002). Said methods are further described by way of example in B. Jenes et al., Techniques for Gene Transfer, in: Transgenic Plants, Vol. 1 , Engineering and Utilization, eds. S.D. Kung and R. Wu, Academic Press (1993) 128-143 and in Potrykus Annu. Rev. Plant Physiol. Plant Molec. Biol. 42 (1991) 205-225). The nucleic acids or the construct to be expressed is preferably cloned into a vector, which is suitable for transforming Agrobacterium tumefaciens, for example pBin19 (Bevan et al (1984) Nucl. Acids Res. 12-8711). Agrobacteria transformed by such a vector can then be used in known manner for the transformation of plants, such as plants used as a model, like Arabidopsis (Arabidopsis thaliana is within the scope of the present invention not considered as a crop plant), or crop plants such as, by way of example, tobacco plants, for example by immersing bruised leaves or chopped leaves in an agrobacterial solution and then culturing them in suitable media. The transformation of plants by means of *Agrobacterium tumefaciens* is described, for example, by Hofgen and Willmitzer in Nucl. Acid Res. (1988) 16, 9877 or is known inter alia from F.F. White, Vectors for Gene Transfer in Higher Plants; in Transgenic Plants, Vol. 1 , Engineering and Utilization, eds. S.D. Kung and R. Wu, Academic Press, 1993, pp. 15-38.

In addition to the transformation of somatic cells, which then have to be regenerated into intact plants, it is also possible to transform the cells of plant meristems and in particular those cells which develop into gametes. In this case, the transformed gametes follow the natural plant development, giving rise to transgenic plants. Thus, for example, seeds of Arabidopsis are treated with agrobacteria and seeds are obtained from the developing plants of which a certain proportion is transformed and thus transgenic [Feldman, KA and Marks MD (1987). Mol Gen Genet 208:1 -9; Feldmann K (1992). In: C Koncz, N-H Chua and J Shell, eds, Methods in Arabidopsis Research. Word Scientific, Singapore, pp. 274-289]. Alternative methods are based on the repeated removal of the inflorescences and incubation of the excision site in the center of the rosette with transformed agrobacteria, whereby transformed seeds can likewise be obtained at a later point in time (Chang (1994). Plant J. 5: 551 -558; Katavic (1994). Mol Gen Genet, 245: 363-370). However, an especially effective method is the vacuum infiltration method with its modifications such as the "floral dip" method. In the case of vacuum infiltration of Arabidopsis, intact plants under reduced pressure are treated with an agrobacterial suspension [Bechthold, N (1993). CR Acad Sci Paris Life Sci, 316: 1 194-1 199], while in the case of the "floral dip" method the developing floral tissue is incubated briefly with a surfactant-treated agrobacterial suspension [Clough, SJ and Bent AF (1998) The Plant J. 16, 735-743]. A certain proportion of transgenic seeds are harvested in both cases, and these seeds can be distinguished from non-transgenic seeds by growing under the above-described selective conditions. In addition the stable transformation of plastids is of advantages because plastids are inherited maternally is most crops reducing or eliminating the risk of transgene flow through pollen. The transformation of the chloroplast genome is generally achieved by a process which has been schematically displayed in Klaus et al., 2004 [Nature Biotechnology 22 (2), 225-229]. Briefly the sequences to be transformed are cloned together with a selectable marker gene between flanking sequences homologous to the chloroplast genome. These homologous flanking sequences direct site specific integration into the plastome. Plastidal transformation has been described for many different plant species and an overview is given in Bock (2001) Transgenic plastids in basic research and plant biotechnology. J Mol Biol. 2001 Sep 21; 312 (3):425-38 or Maliga, P (2003) Progress towards commercialization of plastid transformation technology. Trends Biotechnol. 21 , 20-28. Further biotechnological progress has recently been reported in form of marker free plastid transformants, which can be produced by a transient co-integrated maker gene (Klaus et al., 2004, Nature Biotechnology 22(2), 225-229).

The genetically modified plant cells can be regenerated via all methods with which the skilled worker is familiar. Suitable methods can be found in the abovementioned publications by S.D. Kung and R. Wu, Potrykus or Hofgen and Willmitzer.

Generally after transformation, plant cells or cell groupings are selected for the presence of one or more markers which are encoded by plant-expressible genes co-transferred with the gene of interest, following which the transformed material is regenerated into a whole plant. To select transformed plants, the plant material obtained in the transformation is, as a rule, subjected to selective conditions so that transformed plants can be distinguished from untransformed plants. For example, the seeds obtained in the above-described manner can be planted and, after an initial growing period, subjected to a suitable selection by spraying. A further possibility consists in growing the seeds, if appropriate after sterilization, on agar plates using a suitable selection agent so that only the transformed seeds can grow into plants. Alternatively, the transformed plants are screened for the presence of a selectable marker such as the ones described above.

Following DNA transfer and regeneration, putatively transformed plants may also be evaluated, for instance using Southern analysis, for the presence of the gene of interest, copy number and/or genomic organisation. Alternatively or additionally, expression levels of the newly introduced DNA may be monitored using Northern and/or Western analysis, both techniques being well known to persons having ordinary skill in the art.

The generated transformed plants may be propagated by a variety of means, such as by clonal propagation or classical breeding techniques. For example, a first generation (or T1) transformed plant may be selfed and homozygous second-generation (or T2) transformants selected, and the T2 plants may then further be propagated through classical breeding techniques. The generated transformed organisms may take a variety of forms. For example, they may be chimeras of transformed cells and non-transformed cells; clonal transformants (e.g., all cells transformed to contain the expression cassette); grafts of transformed and untransformed tissues (e.g., in plants, a transformed rootstock grafted to an untransformed scion).

Usually an increase in yield and/or growth rate occurs whether the plant is under non-stress conditions. Plants typically respond to exposure to stress by growing more slowly. In conditions of severe stress, the plant may even stop growing altogether. Mild stress on the other hand is defined herein as being any stress to which a plant is exposed which does result in the plant ceasing to grow slower (or temporarily) but still has the capacity to resume growth when the (mild) stress disappears. Mild stress in the sense of the invention leads to a reduction in the growth of the stressed plants of less than 40%, 35%, 30% or 25%, more preferably less than 20% or 15% in comparison to the control plant under non-stress conditions. Due to advances in agricultural practices (irrigation, fertilization, pesticide treatments) severe stresses are not often encountered in cultivated crop plants. As a consequence, the compromised growth induced by mild stress is often an undesirable feature for agriculture. "Mild stresses" are the everyday biotic and/or abiotic (environmental) stresses to which a plant is exposed. Abiotic stresses may be due to drought or excess water, anaerobic stress, salt stress, chemical toxicity, oxidative stress and hot, cold or freezing temperatures.

"Biotic stresses" are typically those stresses caused by pathogens, such as bacteria, viruses, fungi, nematodes and insects.

The "abiotic stress" may be an osmotic stress caused by a water stress, e.g. due to drought, salt stress, or freezing stress. Abiotic stress may also be an oxidative stress or a cold stress. "Freezing stress" is intended to refer to stress due to freezing temperatures, i.e. temperatures at which available water molecules freeze and turn into ice. "Cold stress", also called "chilling stress", is intended to refer to cold temperatures, e.g. temperatures below 10°, or preferably below 5°C, but at which water molecules do not freeze. As reported in Wang et al. (Planta (2003) 218: 1 -14), abiotic stress leads to a series of morphological, physiological, biochemical and molecular changes that adversely affect plant growth and productivity. Drought, salinity, extreme temperatures and oxidative stress are known to be interconnected and may induce growth and cellular damage through similar mechanisms. Rabbani et al. (Plant Physiol (2003) 133: 1755-1767) describes a particularly high degree of "cross talk" between drought stress and high-salinity stress. For example, drought and/or salinisation are manifested primarily as osmotic stress, resulting in the disruption of homeostasis and ion distribution in the cell. Oxidative stress, which frequently accompanies high or low temperature, salinity or drought stress, may cause denaturing of functional and structural proteins. As a consequence, these diverse environmental stresses often activate similar cell signalling pathways and cellular responses, such as the production of stress proteins, up-regulation of anti-oxidants, accumulation of compatible solutes and growth arrest. The term "non-stress" conditions as used herein are those environmental conditions that allow optimal growth of plants. Persons skilled in the art are aware of normal soil conditions and climatic conditions for a given location. Plants with optimal growth conditions, (grown under non-stress conditions) typically yield in increasing order of preference at least 97%, 95%, 92%, 90%, 87%, 85%, 83%, 80%, 77% or 75% of the average production of such plant in a given environment. Average production may be calculated on harvest and/or season basis. Persons skilled in the art are aware of average yield productions of a crop.

In particular, the methods of the present invention may be performed under non-stress conditions. In an example, the methods of the present invention may be performed under non-stress conditions such as mild drought to give plants having increased yield relative to control plants.

In another embodiment, the methods of the present invention may be performed under stress conditions.

In an example, the methods of the present invention may be performed under stress conditions such as drought to give plants having increased yield relative to control plants. In another example, the methods of the present invention may be performed under stress conditions such as nutrient deficiency to give plants having increased yield relative to control plants.

Nutrient deficiency may result from a lack of nutrients such as nitrogen, phosphates and other phosphorous-containing compounds, potassium, calcium, magnesium, manganese, iron and boron, amongst others.

In yet another example, the methods of the present invention may be performed under stress conditions such as salt stress to give plants having increased yield relative to control plants. The term salt stress is not restricted to common salt (NaCl), but may be any one or more of: NaCl, KCl, LiCl, MgC , CaC , amongst others.

In yet another example, the methods of the present invention may be performed under stress conditions such as cold stress or freezing stress to give plants having increased yield relative to control plants.

The terms "increase", "improve" or "enhance" are interchangeable and shall mean in the sense of the application at least a 3%, 4%, 5%, 6%, 7%, 8%, 9% or 10%, preferably at least 15% or 20%, more preferably 25%, 30%, 35% or 40% more yield and/or growth in comparison to control plants as defined herein.

The term "plant" as used herein encompasses whole plants, ancestors and progeny of the plants and plant parts, including seeds, shoots, stems, leaves, roots (including tubers), flowers, and tissues and organs, wherein each of the aforementioned comprise the gene/nucleic acid of interest. The term "plant" also encompasses plant cells, suspension cultures, callus tissue, embryos, meristematic regions, gametophytes, sporophytes, pollen and microspores, again wherein each of the aforementioned comprises the gene/nucleic acid of interest.

Plants that are particularly useful in the methods of the invention include in particular monocotyledonous and dicotyledonous plants including fodder or forage legumes, ornamental plants, food crops, trees or shrubs selected from the list comprising Acer spp., Actinidia spp., Abelmoschus spp., Agave sisalana, Agropyron spp., Agrostis stolonifera, Allium spp., Amaranthus spp., Ammophila arenaria, Ananas comosus, Annona spp., Apium graveolens, Arachis spp, Artocarpus spp., Asparagus officinalis, Avena spp. (e.g. Avena sativa, Avena fatua, Avena byzantina, Avena fatua var. sativa, Avena hybrida), Averrhoa carambola, Bambusa sp., Benincasa hispida, Bertholletia excelsea, Beta vulgaris, Brassica spp. (e.g. Brassica napus, Brassica rapa ssp. [canola, oilseed rape, turnip rape]), Cadaba farinosa, Camellia sinensis, Canna indica, Cannabis sativa, Capsicum spp., Carex elata, Carica papaya, Carissa macrocarpa, Carya spp., Carthamus tinctorius, Castanea spp., Ceiba pentandra, Cichorium endivia, Cinnamomum spp., Citrullus lanatus, Citrus spp., Cocos spp., Coffea spp., Colocasia esculenta, Cola spp., Corchorus sp., Coriandrum sativum, Corylus spp., Crataegus spp., Crocus sativus, Cucurbita spp., Cucumis spp., Cynara spp., Daucus carota, Desmodium spp., Dimocarpus longan, Dioscorea spp., Diospyros spp., Echinochloa spp., Elaeis (e.g. Elaeis guineensis, Elaeis oleifera), Eleusine coracana, Eragrostis tef, Erianthus sp., Eriobotrya japonica, Eucalyptus sp., Eugenia uniflora, Fagopyrum spp., Fagus spp., Festuca arundinacea, Ficus carica, Fortunella spp., Fragaria spp., Ginkgo biloba, Glycine spp. (e.g. Glycine max, Soja hispida or Soja max), Gossypium hirsutum, Helianthus spp. (e.g. Helianthus annuus), Hemerocallis fulva, Hibiscus spp., Hordeum spp. (e.g. Hordeum vulgare), Ipomoea batatas, Juglans spp., Lactuca sativa, Lathyrus spp., Lens culinaris, Linum usitatissimum, Litchi chinensis, Lotus spp., Luffa acutangula, Lupinus spp., Luzula sylvatica, Lycopersicon spp. (e.g. Lycopersicon esculentum, Lycopersicon lycopersicum, Lycopersicon pyriforme), Macrotyloma spp., Malus spp., Malpighia emarginata, Mammea americana, Mangifera indica, Manihot spp., Manilkara zapota, Medicago sativa, Melilotus spp., Mentha spp., Miscanthus sinensis, Momordica spp., Morus nigra, Musa spp., Nicotiana spp., Olea spp., Opuntia spp., Ornithopus spp., Oryza spp. (e.g. Oryza sativa, Oryza latifolia), Panicum miliaceum, Panicum virgatum, Passiflora edulis, Pastinaca sativa, Pennisetum sp., Persea spp., Petroselinum crispum, Phalaris arundinacea, Phaseolus spp., Phleum pratense, Phoenix spp., Phragmites australis, Physalis spp., Pinus spp., Pistacia vera, Pisum spp., Poa spp., Populus spp., Prosopis spp., Prunus spp., Psidium spp., Punica granatum, Pyrus communis, Quercus spp., Raphanus sativus, Rheum rhabarbarum, Ribes spp., Ricinus communis, Rubus spp., Saccharum spp., Salix sp., Sambucus spp., Secale cereale, Sesamum spp., Sinapis sp., Solanum spp. (e.g. Solanum tuberosum, Solanum integrifolium or Solanum lycopersicum), Sorghum bicolor, Spinacia spp., Syzygium spp., Tagetes spp., Tamarindus indica, Theobroma cacao, Trifolium spp., Tripsacum dactyloides, Triticosecale rimpaui, Triticum spp. (e.g. Triticum aestivum, Triticum durum, Triticum turgidum, Triticum hybernum, Triticum macha, Triticum sativum, Triticum monococcum or Triticum vulgare), Tropaeolum minus, Tropaeolum majus, Vaccinium spp., Vicia spp., Vigna spp., Viola odorata, Vitis spp., Zea mays, Zizania palustris, Ziziphus spp., amongst others.

The choice of suitable control plants is a routine part of an experimental setup and may include corresponding wild type plants or corresponding plants without the gene of interest. The control plant is typically of the same plant species or even of the same variety as the plant to be assessed. The control plant may also be a nullizygote of the plant to be assessed. Nullizygotes are individuals missing the transgene by segregation. A "control plant" as used herein refers not only to whole plants, but also to plant parts, including seeds and seed parts.

The invention provides a genetically modified plant, which can be a transgenic plant, that is more tolerant to a stress condition than a corresponding reference plant. As used herein, the term "tolerant" when used in reference to a stress condition of a plant, means that the particular plant, when exposed to a stress condition, shows less of an effect, or no effect, in response to the condition as compared to a corresponding reference plant (naturally occurring wild-type plant or a plant not containing a construct of the present invention). As a consequence, a plant encompassed within the present invention shows improved agronomic performance as a result of enhanced abiotic stress tolerance and grows better under more widely varying conditions, such as increased biomass and/or higher yields and/or produces more seeds. Preferably, the transgenic plant is capable of substantially normal growth under environmental conditions where the corresponding reference plant shows reduced growth, yield, metabolism or viability, or increased male or female sterility.

As used herein, the term "drought-tolerance" refers to the more desirable productivity of a plant under conditions of water deficit stress. Water deficit stress develops as the evapotranspiration demand for water exceeds the supply of water. Water deficit stress can be of large or small magnitude (e.g., days or weeks of little or no accessible water), but drought tolerant plants will show better growth and/or recovery from the stress, as compared to drought sensitive plants. As used herein, the term "water use efficiency" refers to the more desirable productivity of a plant per unit of water applied. The applied water may be the result of precipitation or irrigation. As used herein, the term "salt-tolerance" refers to the more desirable productivity of a plant under conditions of salinity stress. While for each species, the threshold at which soil and/or water salinity (often expressed as conductivity) differs, a salt-tolerant plant would have a higher salinity threshold before yields decline. Salt-tolerance also refers to the sensitivity of yield to water and/or soil salinity beyond the threshold. So a salt-tolerant plant would show less impact on yield per unit of salinity than a salt-sensitive plant. Salt-tolerance refers to an increased threshold and/or a decreased sensitivity beyond the threshold of yield to salinity.

The term "expression cassette" refers to any recombinant expression system for the purpose of expressing a nucleic acid sequence of the invention in vitro or in vivo, constitutively or inducibly, in any cell, including, in addition to plant cells, prokaryotic, yeast, fungal, insect or mammalian cells. The term includes linear and circular expression systems. The term includes all vectors. The cassettes can remain episomal or integrate into the host cell genome. The expression cassettes can have the ability to self-replicate or not (i.e., drive only transient expression in a cell). The term includes recombinant expression cassettes that contain only the minimum elements needed for transcription of the recombinant nucleic acid.

The term "inducible" or "inducibly" means the polypeptides of the present invention are not expressed, or are expressed at very low levels, in the absence of an inducing agent. The expression of the polypeptides of the present invention is greatly induced in response to an inducing agent.

The following non-limiting Examples describe methods and means according to the invention. Unless stated otherwise in the Examples, all techniques are carried out according to protocols standard in the art. The following examples are included to illustrate embodiments of the invention. Those of skill in the art should, in light of the present disclosure, appreciate that many changes can be made in the specific embodiments which are disclosed and still obtain a like or similar result. More specifically, it will be apparent that certain agents which are both chemically and physiologically related may be substituted for the agents described herein while the same or similar results would be achieved.

### Examples

### 1.Osmotic stress affects cell proliferation and endoreduplication onset

To decipher the mechanisms by which water deficit inhibits cell proliferation, an experimental set-up was developed that reproducibly reduced the leaf area by approximately 50%. The best results were obtained by addition of mannitol to the growth medium at a low concentration (25 mM), thereby decreasing the water potential of the medium and, hence, water uptake of the exposed roots. *Arabidopsis* seedlings were germinated and grown on nylon meshes overlaying control medium until 9 days after stratification (DAS), when the third true leaf is fully proliferating (Skirycz et al., 2010), and subsequently transferred to control or mannitol-containing medium (Figures 1A to 1C). Kinematic analysis was performed, whereby leaves were harvested daily throughout development of leaf 3 (9-20 DAS) and based on drawings of the abaxial epidermis cell number, cell area, number of guard cells and cell division and cell expansion rates were calculated (De Veylder et al., 2001). Decrease in leaf area was already apparent 24 h after the transfer (Figure 2A; *t*-test, *p*-value = 0.003) and resulted from reduced proliferation rates, as demonstrated by cellular measurements (Figure 2B). However, this reduction was short-term as cell division rates of stressed plants were indistinguishable from controls within 72 h of transfer, and afterward even slightly increased as a compensation for the initial decrease (Figure 2B). Importantly, transfer itself did not inhibit leaf growth and the reduced cell numbers could be fully attributed to osmotic stress (not shown). Leaf and plant morphology were not altered by mannitol (Figure 2C). Cell expansion was also affected by stress and the reduced final leaf area was a combination of fewer and smaller cells (Figure 2D). The stomatal index, which represents the number of stomata as a fraction of the total number of cells, was reduced as well (Figure 2E). To learn more about the mechanisms underlying the rapid reduction of cell division upon stress onset, we harvested leaf samples daily after transfer. As a measure of cellular differentiation, the ploidy distribution was examined by flow cytometry. This revealed significant differences starting from 48 h after stress imposition (Figure 3A). In stressed leaves, 4C nuclei started to accumulate approximately one day earlier than in controls at the expense of 2C nuclei (Figure 3A). Analogously, the number of 8C nuclei increased sharply at 14 DAS in stressed leaves, but only at 15 DAS in control samples (Figure 3A). Again, transfer itself had no effect on ploidy levels (not shown). Faster onset of endoreduplication implied that stress induced mitotic exit. This observation was further investigated with a *CYCB1;1:Dbox-GUS* reporter line. Staining for *CYCB1;1:Dbox-GUS* activity visualizes cells at the G2-to-M transition, reflecting mitotic activity (Colón-Carmona et al., 1999). The most apparent differences were observed 48 h after transfer. Whereas the developmental differentiation manifested by strong staining at the leaf base but no staining at the leaf tip could be clearly seen in both control and stressed leaves, the overall GUS activity was much weaker in the stressed leaves corresponding to a reduced number of mitotic cells (Figure 3B). While the relative size of the cell proliferation zone was similar in mannitol-treated leaves, the remaining proliferating cells were found in a dispersed pattern throughout this zone. In conclusion, exposure of proliferating leaves to mild osmotic stress leads to a rapid decrease in cell division rates and a faster onset of endoreduplication, indicative of an early mitotic exit as also observed by the reduced expression of *CYCB1;1:Dbox-GUS* upon stress treatment. Within a few days after transfer, division rates adapted to the new conditions and compensation effects were observed.

### 2. Cell cycle arrest and exit depend on stress duration

To investigate the dynamics of stress signaling, we transferred 9-DAS-old seedlings to mannitol for 10, 24 or 48 h and subsequently transferred them back to control medium. Whereas in all cases osmotic stress resulted in a cell cycle arrest, illustrated by a reduced leaf area measured at 10 DAS (24 h after the initial transfer) (Figure 4A), 10 h of stress imposition was too short to trigger mitotic exit, while after 24 h some early differentiation was visible (Figure 4C). Moreover, the initial reduction in cell number measured after exposure for 10 h and 24 h was completely compensated and no changes in cell number could be detected at 14 DAS, while this recovery was only partial for plants exposed to stress for 48 h (Figure 4B). Along with cell numbers, the stomatal index recovered as well in these plants (not shown). In conclusion, a short stress exposure reversibly arrests cell cycle and only when stress persists mitotic exit and differentiation are observed.

### 3. Increased meristemoid division activity aids in cell number recovery

After the initial arrest of cell division in the cell proliferation zone and subsequent mitotic cell cycle exit, cell division rates recovered and became slightly higher in mannitol-treated leaves (Figure 2B) starting at 13 DAS, a time point at which the defined cell division zone is reduced to a very narrow zone near the base of the leaf. Furthermore, when plants were stressed for 48 h, at which time differentiation had occurred, and were then transferred back to control medium, their cell numbers partially recovered and, simultaneously, stomatal index fully recovered as well (Figure 4C). The divisions associated with the formation of stomata might account for this recovery. Using the *CYCB1;1:DBox-GUS* line, we could indeed show that at the time of cell number recovery (13-14 DAS) meristemoid division activity was higher in mannitol-treated samples and in samples recovering from mannitol treatment than in control samples (Figure 5A). As the meristemoid lineage is restricted to the epidermis, we also checked for division activity in the internal tissues of the leaf and observed that at 14 DAS there was still some mitotic activity at the base of the leaf of mannitol-grown plants, while in control leaves the cell proliferation zone had completely disappeared (Figure 5B).

### 4. Molecular insight into growth inhibition revealed by transcript profiling

To obtain a better understanding of the molecular mechanisms inhibiting cell division during stress, we subjected proliferating leaf primordia to whole-genome transcript profiling. Statistical analysis identified 27, 189, 351, and 886 up-regulated and 31, 145, 84, and 622 down-regulated transcripts at 1.5, 3, 12 and 24 h after transfer to 25 mM mannitol, respectively. Selected microarray data could be validated with a nCounter platform containing probes for 100 genes involved in growth, stress, and hormonal regulation. Differential transcripts were used to construct Venn diagrams and were subjected to *k*-means clustering. The number of differentially expressed genes was proportional to the stress exposure time and the majority of the genes that were up- or down-regulated at earlier time points remained high or low at 24 h, respectively. Subsequently, the differential transcripts were examined with PageMan to calculate the functional overrepresentation of MapMan categories (Thimm et al., 2004; Usadel et al., 2006) and were compared to selected publicly available microarray experiments (see "Materials and Methods"). To explain the reduced cell proliferation rates, cell cycle genes were among the prime suspects. Indeed, a number of transcripts encoding A-, B- and D-type cyclins, CDKB, SIAMESE-related proteins, and a MYB3R4 transcription factor were significantly down-regulated (Figure 6A). Comparison with expression data obtained from synchronized cell cultures (Menges et al., 2003) revealed a significant overrepresentation of mitotic genes among the down-regulated transcripts, such as the *AURORA* kinases and the kinesin *HINKEL* that are involved in cytokinesis (see Figure 6A). Strikingly, the magnitude of change for all of the above-mentioned cell cycle genes was similar (approximately 30%) and occurred only at 24 h (Figure 6A). Besides the cell cycle-related transcripts, we were particularly interested in genes related to hormonal signaling. Both the comparison to publicly available hormone addition experiments (Goda et al., 2008) and the PageMan analysis revealed changes in ethylene signaling. ACC-responsive genes were enriched among the transcripts up-regulated in proliferating leaves as early as 1.5 and 3 h after stress onset. The expression of genes directly involved in ethylene signaling was also induced, namely the ethylene receptors *(ETR2* and *ESR1*), *CTR1* and *MPK3* MAPKs, *EIN3* and *EIL1* transcription factors, *EBF1* and *EBF2* involved in EIN3 protein degradation, and a number of ethylene-responsive transcription factors *(ERF1, ERF2, ERF5, ERF6,* and *ERF11)* (Figure 7A). While transcripts encoding ACC biosynthetic enzymes were not affected, ACC oxidase (ACO2), which converts ACC to ethylene, was up-regulated (Figure 7A). Significantly, no activation of neither ABA nor jasmonate signaling, two other classical stress hormones, was apparent from the transcriptome analysis. Importantly, transfer itself did not affect the expression of selected cell cycle and stress genes as measured by quantitative reverse-transcription (qRT)-PCR, further showing that there is no basal stress response in controls due to the transfer that could mask stress responses, such as an ABA response, in plants exposed to mannitol. In summary, short-term stress exposure resulted in rapid induction of genes involved in ethylene signaling. Cell cycle-related genes were concomitantly down-regulated, but only 24 h after stress onset.

### 5. ACC accumulates in shoots of stressed plants

To find out whether differential transcripts reflect changes in hormone levels, we determined concentrations of the ethylene precursor ACC in complete shoots of 9-day-old plants transferred to mannitol. Already 1 h after stress onset, ACC levels were 30% higher in stressed than in control samples, although this was not significant, and by 10 h, the increase was more than 2-fold and significant (Figure 7B). At the time of analysis, the shoot samples were mainly composed of expanding cells, reflecting the overall importance of ethylene signaling for the response of growing tissues to stress.

### 6. CDKA activity is reduced within hours of stress onset

As transcripts of the cell cycle genes were down-regulated by stress relatively late, it is unlikely that transcriptional mechanisms contribute to the rapid cell cycle arrest. To study the involvement of posttranscriptional mechanisms, we investigated the activity and protein abundance of CDKA. CDKA is a non-redundant protein situated at the heart of the cell cycle regulation and promotes both G1-to-S and G2-to-M transitions (Inzé and De Veylder, 2006). Although transcript and protein levels of CDKA were stable throughout the stress treatment, CDKA activity decreased as early as 10 h after stress onset and remained low at 24 h (Figure 6B). The rapid decrease in CDKA activity upon stress coincides with the cell cycle arrest.

### 7. Ethylene arrests the mitotic cell cycle

The transcriptome analysis revealed an early stress-dependent activation of ethylene-responsive genes in leaf primordia. To assess the role of ethylene in cell cycle regulation, we analyzed the effect of ACC. To this end, seedlings were transferred to medium containing 5 µM ACC (Goda et al., 2008) with the same set-up as that used for the mannitol treatments. Similarly to osmotic stress, transfer of seedlings to ACC resulted in a rapid reduction of cell proliferation rates (Figure 2B) and CDKA activity decreased as early as 10 h after transfer (Figure 6B). However, in contrast to mannitol treatment, the ploidy analysis revealed no changes in endoreduplication onset (Figure 3A) and no difference in the expression of the mitotic cell cycle genes *CDKB2;1* and *CYCB1;1*. Consistently, staining for *CYCB1;1:DBox-*GUS activity revealed no changes in the number of mitotic cells (Figure 3B). Stomatal index was not affected either (Figure 2E). The possible involvement of ethylene in the stress-induced inhibition of cell division predicts that ethylene-insensitive mutants would be less affected by transfer to mannitol. To test this prediction, we selected mutants with no or little effect on growth under normal conditions. This hypothesis proved true for the receptor mutant *etr1.3* and for *ein5.1,* defective in the activity of the *XRN4* exoribonuclease upstream of the EBF1 and EBF2 F-Box proteins (Figures 8C and 8D). A particularly pronounced difference was measured 72 h after transfer (12 DAS); whereas the reduction in leaf area of the wild type was ∼45%, it was only ∼20% and ∼30% for *ein5.1* and *etr1.3,* respectively (Figure 8C). A difference, albeit not significant, was also measured for the *mkk9* mutant. Importantly, early endoreduplication onset measured in stress-treated wild-type leaves could also be detected in both *ein5.1* and *etr1.3* mutants (data not shown) exposed to mannitol. As reported before, when left on mannitol-containing medium until 22 DAS, all of the mutants, and particularly *ein2.5,* developed severe phenotypes and were overall much more affected by stress than the wild-type plants (Skirycz et al., 2010). In addition to mannitol, leaf growth of ethylene-insensitive mutants was also tested after transfer to ACC. While ACC-related decrease in leaf area in *ein3.1* and *eil1* mutants was comparable to that of the wild-type, it was significantly less in *etr1.3, ein5.1, mkk9,* and *ein2.5* mutants (Figures 8E and 8F). Consistently, CDKA activity was reduced by ACC treatment in wild-type and *ein3.1* but not *etr1.3* and *ein5.1* mutants. In conclusion, exogenous ACC addition or activation of ethylene production reduces cell proliferation without significantly affecting the onset of endoreduplication and subsequent cellular differentiation in an EIN3-independent manner.

### 8. Expression of a bacterial and a plant ACC deaminase under control of three different promoters

In this experiment we aimed for a downregulation of the ethylene signaling pathway only in proliferating leaf cells (chimeric genes with 2 different meristematic promoters: p4TM1 and pGR5) as compared to a downregulation of the ethylene signaling in the entire plant (chimeric gene with the 335S promoter). The transgene which was used for downregulation of the ethylene signaling was the ACC deaminase (a bacterial form and a plant form), an enzyme that degrades the ethylene precursor ACC. The 6 different chimeric genes were constructed by cloning either a bacterial ACC deaminase (bACD) or the endogenous *Arabidopsis thaliana* ACC deaminase (AtACD1) under control of the promoters of two genes whose expression is mainly limited to proliferating leaf cells, pGRF5 and p4TM1, and under control of the 35S promoter, which is expressed in the entire plant through development, as a control. For each of these 6 chimeric constructs a wild-type was isolated, along with one to three transgenic lines showing a good expression of the transgene ACC deaminase.

To clone the promoters of GRF5 (derived from the gene AT3G13960) and 4TM1 (derived from the gene AT5G16250), the 2 kb-fragment (designated as pGRF5 and depicted in SEQ ID NO: 5) or 400 bp-fragment (designated as p4TM1 and depicted in SEQ ID NO: 1) preceding the translation start site was amplified from genomic DNA with primers carrying attB-sites, allowing Gateway recombination into pDONR-P4-P1R vectors. The bacterial ACC deaminase (nucleotide sequence is depicted in SEQ ID NO: 7), isolated from *Pseudomonas putida* UW4, was obtained from Prof. B.R. Glick (Shah et al (1998) Can. J. of Microbiol. 44:833-843). The coding sequence for AtACD1 (annotated gene AT1G48420) was amplified from cDNA, and both coding sequences were recombined into the pDONR221 vector, the AtACD1 nucleotide sequence is depicted in SEQ ID NO: 8. These entry vectors were then recombined using Multisite Gateway into pK7m34GW-FAST, and the resulting expression vectors were transformed into *Agrobacterium tumefaciens* C58C1 pMP90, which was used to transform *Arabidopsis thaliana* accession Col-0. For each construct several single-locus T2 lines were selected carrying either a homozygous T-DNA insertion or no insertion (wild-type controls).

All lines were grown in vitro on ½ MS plates with 1% sucrose, and all transgenic lines were grown on the same plate with the corresponding wild-type control. For each line, 5 shoots were harvested and pooled, and RNA was extracted using Trizol followed by RNase-free DNase treatment and clean-up with the Qiagen RNeasy Plant Mini kit. Starting from 1 ug of RNA, cDNA was synthesized using the Bio-Rad iScript cDNA Synthesis kit. By PCR a specific fragment of both the bacterial ACC deaminase and the endogenous ACC deaminase was amplified, along with the reference gene PP2AA3 (AT1G13320), expression of which was reported to be very stable (Czechowski et al (2005) Plant Physiology 139: 5-17). PCR products were separated on a 2% agarose gel. The output of the expression analysis is depicted in Figure 10.

Plant were transferred to plates containing 25 mM mannitol, hereby exposing them to mild mannitol stress, at a stage where the third leaf is fully proliferating, allowing to study the response of proliferating leaf cells to stress. After 48 hours on mild mannitol stress, leaf 3 was microdissected and its size was measured to calculate the extent of growth inhibition. WT and the different transgenic lines were each time grown on the same plate to allow comparison.

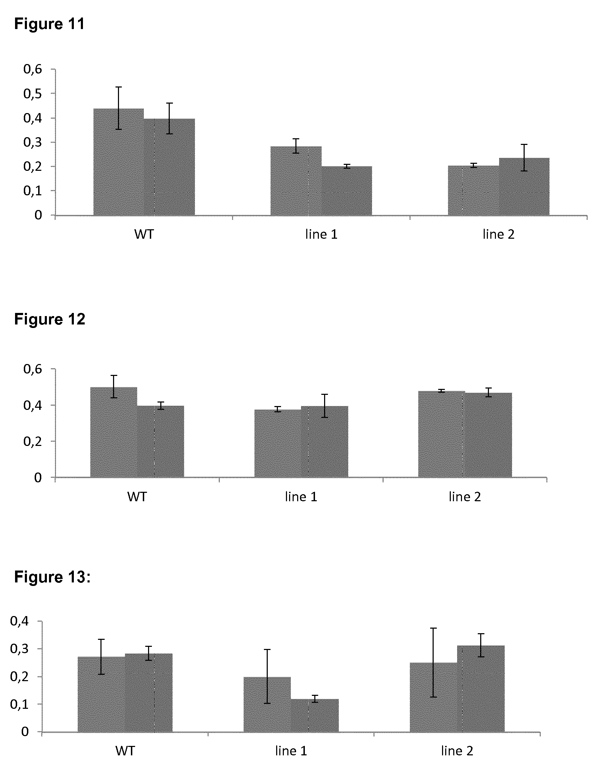

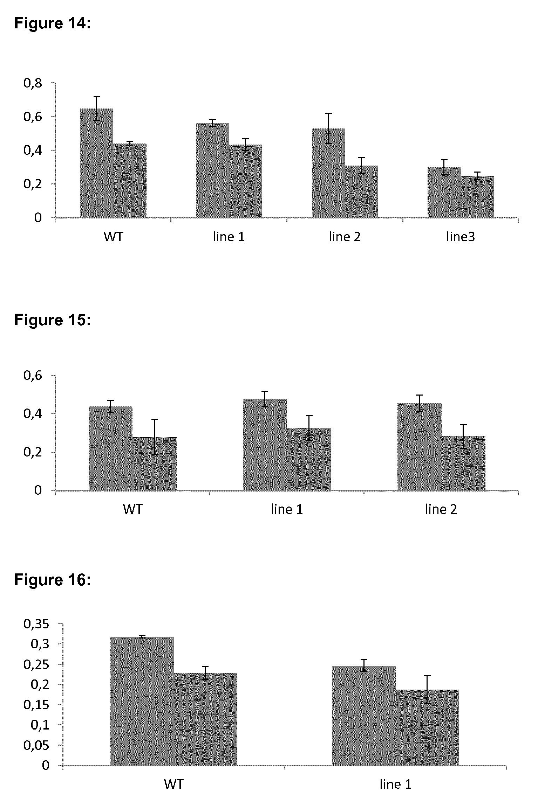

The effect on mild abiotic stress of the expression of the ACC deaminase genes in plants was studied with the transformation of the six different chimeric genes in *Arabidopsis* plants and is hereunder discussed:
1) Chimeric gene wherein the bacterial ACC deaminase is under control of the pGRF5 promoter (pGRF5::bACD)
   The data show (see Figure 11) that the wild type was only inhibited by about 10% under abiotic stress. The transgenic line 2 clearly performs better under abiotic stress. Both transgenic lines are however clearly smaller than the WT, indicating a negative effect of the bacterial ACC deaminase transgene expression in plants expressed under control of the GRF5 promoter.
2) Chimeric gene wherein the bacterial ACC deaminase is under control of the p4TM1 promoter (p4TM1 ::bACD)
   The data show (see Figure 12) that the expression of the bacterial ACC deaminase in transgenic lines, driven by the 4TM promoter, almost completely abolished the growth inhibition by stress. For transgenic line 1 a small negative effect on leaf size under on abiotic stress conditions can be seen. However, this negative effect is certainly less than when the bacterial ACC deaminase transgene is under control of the GRF5 promoter.
3) Chimeric gene wherein the bacterial ACC deaminase is under control of the p35S promoter (p35S::bACD)
   The data show (see Figure 13) a reduction of the growth of the transgenic plants (even under non-abiotic stress conditions) upon expression of the chimeric gene.
4) Chimeric gene wherein the *Arabidopsis thaliana* ACC deaminase is under control of the pGRF5 promoter (pGRF5::AtACD1)
   The data show (see Figure 14) that similar to the bacterial ACC deaminase (compare the data from Figure 11), that the mere expression of the endogenous deaminase under control of the GRF5 promoter inhibits growth.
5) Chimeric gene wherein the *Arabidopsis thaliana* ACC deaminase is under control of the p4TM1 promoter (p4TM1::AtACD1)
   The data show (see Figure 15) that the expression from the 4TM1 promoter in the transgenic lines again eliminates the negative effect of abiotic stress on leaf area.
6) Chimeric gene wherein the *Arabidopsis thaliana* ACC deaminase is under control of the p35S promoter (p35S::AtACD1)
   The data show (see Figure 16) that a similar pattern is observed as with the expression of the transgene driven by the GRF5 promoter (compare with Figure 14). There is a clear negative effect on the normal growth (i.e. non abiotic stress) upon expression of the transgene while the growth inhibition by abiotic stress is not affected.

We conclude that the expression of both ACC deaminases has a negative effect on the plant growth for chimeric genes wherein the expression is under control of the p35S and pGRF5 promoters. However, this effect is surprisingly not present when the expression of ACC deaminase is under control from the 4TM1 promoter. Further, the expression of the chimeric gene comprising the 4TM1 promoter and the bacterial ACC deaminase can overcome growth defects due to abiotic stress. While not limiting the invention to a particular mechanism of action the expression of the endogenous *A. th.* ACC deaminase under control of the 4TM1 promoter, has less effect under mild stress than the bacterial ACC deaminase, possibly due to posttranslational regulation mechanisms.

### 9. Meristem-specific down-regulation of the ACC oxidase enzyme in Arabidopsis thaliana

A chimeric gene is constructed containing the following DNA elements
- 4TM1 promoter (SEQ ID NO: 1) from Arabidopsis thaliana
- An antisense RNA encoding ACC oxidase from Arabidopsis thaliana
- A CaMV 35S terminator

This chimeric gene is introduced into a T-DNA vector (pK7m24GW-FAST) in combination with a selectable GFP marker. The T-DNA vector is introduced into *Agrobacterium tumefaciens* and used to produce transgenic *Arabidopsis.*

Leaf growth of the transgenic is plants is analysed under optimal and stress conditions.

Wild-type and transgenic seeds are grown in vitro with Murashige and Skoog (MS) medium containing 0.5% sucrose under a 16-h/8-h photoperiod. For osmotic stress, wild-type and transgenic seeds are allowed to germinate for 5-7 days and transferred to mannitol containing agar plates (Skirycz et al. 2010). Shoot fresh and dry weight, leaf area, root length and mass are measured. Under soil conditions, a high-throughput, fully automated water monitoring system, named WIWAM, implemented at the host institute is used (Skirycz et al. 2011). This system enables to keep stable water levels and is capable of taking digital images of individual plants that can be used to determine rosette growth, leaf area and leaf shape. Plants are grown under control watering regime until stage 1.04 (approximately 12-13 days old), after which control or limited watering are applied for additional 10-12 days. At the end of the experiment, plants are harvested and the shoot production is recorded as a measurement of yield.

As alternatives for the 4TM1 promoter also the 4TM2 (SEQ ID NO: 2); 4TM3a (SEQ ID NO: 3) or 4TM3b (SEQ ID NO: 4) of Arabidopsis thaliana (or plant orthologous promoters thereof) can be used.

### 10. Modulation of the ethylene production in meristems of corn

Two different chimeric genes are constructed containing the following DNA elements:
- an orthologous *Brachypodium distachyon* promoter of the *Arabidopsis thaliana* 4TM1 promoter operably linked to
- a sense ACC deaminase from Pseudomonas putida UW4 (SEQ ID NO: 7), and
- a CaMV 35S terminator
   and
- an orthologous *Brachypodium distachyon* promoter of the *Arabidopsis thaliana* 4TM1 promoter operably linked to
- an antisense ACC oxidase from *Zea mays,* and
- a CaMV 35S terminator

These two different 2 chimeric genes are introduced into the destination vectors (pBbm42GW7), containing the BASTA herbicide under control of 35S CaMV promoter and followed by a nos terminator as a selectable marker. These constructs are then separately introduced into *Agrobacterium tumefaciens* (EHA101) and used to transform immature maize embryos of B104 which are regenerated by tissue culture to produce 2 different types of transgenic plants (i.e. transgenic maize plants overexpressing an ACC deaminase and transgenic maize plants downregulating the ACC oxidase). The transgenic plants are backcrossed to B104, resulting in a working population segregating in 50% sensitive and thus control plants and 50% transgenic plants. Leaf growth of the segregating population is analyzed under optimal and drought stress conditions. The plants are grown in soil and watered daily: the drought treated plants receive 70% of the water that is added to the control plants. The leaf growth is monitored by daily measuring the leaf length of the fourth leaf upon its appearance, providing data on the leaf elongation rate and the final leaf length. In addition final plant height, fresh weight and dry weight plants will be determined as a measure for plant biomass.

### Materials and methods

### 1.Plant growth

Seedlings of *Arabidopsis thaliana* (L.) Heyhn. ecotype Columbia-0 (Col-0) were grown in vitro in half-strength Murashige and Skoog medium (Murashige and Skoog, 1962), supplemented with 1% sucrose under a 16-h day (110 µmol m⁻² s⁻¹) and 8-h night regime. Plates were overlaid with nylon mesh (Prosep, Zaventem, Belgium) of 20 µm pore size to avoid that roots grew into the medium. Depending on the experiment, 32 or 64 seeds were equally distributed on a 150-mm diameter plate. Mutant plants were grown together with their wild-type controls on the same plate.

### 2.Stress and chemical treatments

At 9 DAS, when the third leaf is fully proliferating, seedlings were transferred to plates containing control medium or medium supplemented with 25 mM mannitol (Sigma-Aldrich) or 5 µM ACC (Sigma-Aldrich) by gently lifting the nylon mesh with forceps. For the CDKA activity assay the transfer was done at 11 DAS; at this stage, the third leaf is still dividing and can be quickly harvested without the need for RNAlater solution (see below) that would inhibit CDKA activity. All transfers were performed 2-3 h into the day.

### 3.Growth analysis

Growth analysis was performed on the third true leaf harvested at different time points after transfer. After clearing with 70% ethanol, leaves were mounted in lactic acid on microscopic slides. For each experiment, 8-12 leaves were photographed with a binocular, and epidermal cells (40-300) were drawn for four representative leaves with a DMLB microscope (Leica) fitted with a drawing tubus and a differential interference contrast objective. Photographs of leaves and drawings were used to measure leaf and cell area, respectively, with ImageJ v1.41o (NIH; http://rsb.info.nih.gov/ij/), from which the cell numbers were calculated. The stomatal index was defined as the percentage of stomata per all cells. For the kinematic analysis In-transformed means of leaf area, cell size, and cell number were locally fitted to a quadratic function of which the first derivative was taken as the relative growth, expansion, and division rate, respectively (De Veylder et al., 2001).

### 4. Sampling for expression analysis

Leaf 3 was harvested from plants at 1.5, 3, 12, and 24 h. Briefly, whole seedlings were harvested in an excess of RNAlater solution (Ambion) and, after overnight storage at 4°C, dissected under a binocular microscope on a cooling plate with precision microscissors. Dissected leaves were transferred to a new tube, frozen in liquid nitrogen, and ground with a Retsch machine and 3-mm metal balls. Samples were obtained from three independent biological experiments and from multiple plates within the experiment.

### 5. RNA extraction

RNA was extracted with TriZol (Invitrogen) according to the manufacturer's instructions with 4 µg of glycogen as carrier during the precipitation step. RNA samples were subjected to DNA digestion with RNase-free DNase I (Roche) and subsequently cleaned up with the RNeasy Mini Kit (Qiagen).

### 6. ATH1 expression profiling and data analysis

RNA samples were hybridized to single Affymetrix ATH1 Genome arrays at the VIB Microarray Facility (Leuven, Belgium). Expression data were processed with Robust Multichip Average (RMA) (background correction, normalization, and summarization) as implemented in BioConductor (Irizarry et al., 2003a, 2003b; Gentleman et al., 2004). An alternative cdf ("tinesath1cdf") was used, in which each probe is uniquely assigned to one transcript (Casneuf et al., 2007) (http://www.bioconductor.org/packages/release/data/experiment/html/tinesath1cdf.html). The BioConductor package *Limma* was used to identify differentially expressed genes (Smyth, 2004). For comparisons of interest, moderated t statistics were calculated using the eBayes function and P values were corrected for multiple testing for each contrast separately using topTable. False-discovery rate (FDR)-corrected p-value < 0.05 was used as a cut-off (Benjamini and Hochberg, 1995).

### 7. Flow cytometry

For flow cytometry analysis, nuclei were extracted by chopping 4-32 leaves with a razor blade in 1 ml of 45 mM MgCl₂, 30 mM sodium citrate, 20 mM 3-(N-morpholino)propanesulfonic acid, pH 7, and 1% Triton X-100 (Galbraith et al., 1983). From a stock of 1 mg/mL 4,6-diamidino-2-phenylindole (DAPI), 1 µL was added to the filtered supernatant. The nuclei were analyzed with a CyFlow flow cytometer with the FloMax Software (Partec).

### 8. CDKA activity assay

Total soluble protein was extracted from 50 leaves by adding extraction buffer (Van Leene et al., 2007) to ground samples, followed by two freeze-thaw steps and two centrifugation steps (20,817g, 10 min, 4°C), whereby the supernatant was collected each time. Equal amounts of total protein were incubated with p9^{CKS1Hs}--sepharose beads (De Veylder et al., 1997) and kinase assays were performed as described (De Veylder et al., 1997) with histone H1 as CDK substrate. To correct for the amount of CDKA protein purified by p9^{CKS1Hs}-spharose beads, an aliquot of each sample was used for protein gel blot analysis with primary rabbit anti-PSTAIRE antibodies (Santa Cruz) (diluted 1:5000) and a secondary horseradish peroxidase-conjugated donkey anti-rabbit antibodies (GE-Healthcare) (diluted 1:10,000). Proteins were detected by chemiluminescence (Western Lightning Plus ECL, PerkinElmer Life Sciences). CDK activity and CDKA amount were quantified with ImageJ v1.41o. Control samples were arbitrarily set at 100%.

### 9. Comparison to publicly available microarray data

Selected publicly available microarray data were grouped according to experiment type (such as abiotic stress and hormone treatment). Groups of experiments were RMA processed and subjected to *Limma* analysis, as described above. Sets of responsive genes were delineated always with a 2-fold expression change and FDR-corrected P value < 0.05 cut-offs. Although these cut-offs were chosen somewhat arbitrarily, we assessed the robustness of the results by testing more and less stringent cut-offs. All tests gave very similar results. The lists of responsive genes were compared to those identified in our microarray experiment to identify global trends in the functional repertoire of the affected genes that were used as hints to explore the results in more detail. Overrepresentation was tested by means of Fisher exact tests followed by Bonferroni P value correction.

### 10. qRT-PCR

For cDNA synthesis, 100 ng to 2 µg of RNA was used with the SuperScript Reverse III reagent (Invitrogen) according to the manufacturer's instructions. Primers were designed with the QuantPrime website (Arvidsson et al., 2008; Skirycz et al., 2010). qRT-PCR was done on a LightCycler 480 (Roche Diagnostics) in 384-well plates with LightCycler 480 SYBR Green I Master (Roche) according to the manufacturer's instructions. Melting curves were analyzed to check primer specificity. Normalization was done against the average of housekeeping genes *UBQ10, GAPDH,* and *CBP20;* ΔCt = Ct (gene) - Ct (mean (housekeeping genes)) and ΔΔCT = ΔCt(control)- ΔCt(mannitol). ΔCt values for the three biological replicates were used for statistical analysis. Ct refers to the number of cycles at which SYBR Green fluorescence reaches an arbitrary value during the exponential phase of the cDNA amplification.

### 11. Nanostring

mRNA expression levels were measured using an nCounter Analysis System (NanoString Technologies) by the VIB MicroArray Facility (www.microarrays.be) as described (Geiss et al., 2008). Total RNA extract (100 ng) was hybridized. Gene expression was measured simultaneously for all genes in multiplexed reactions. The nCounter code set contained probe pairs for 100 *Arabidopsis* genes. The data were normalized by a two-step procedure with internal spike-in controls and the three most stable reference genes included in the probe set (*CDKA_1, UBC,* and *CBP20*)*.*

### 12. Quantification of ACC

Freeze-dried samples were dissolved in 500 µl of methanol, including two internal standard compounds (4 µM methionine sulfone used for compensation of the peak area after capillary electrophoresis-mass spectrometry (CE-MS) analysis and 0.2 µM D4-ACC for quantification of ACC. After addition of 500 µl of chloroform and 200 µl of water, the mixture was vortexed for 3 min and centrifuged at 20,400g for 3 min at 4°C. The upper layer was evaporated for 30 min at 45°C by a centrifugal concentrator and then separated into two layers. The upper layer (100-200 µl) was centrifugally filtered through a Millipore 5-kD cutoff filter at 9,100*g* for 90 min. The filtrate was dried for 120 min by a centrifugal concentrator. The residue was dissolved in 10 µl of water containing a reference compound (3-aminopyrrolidine). The final solution (10 µL) was used to quantify the contents of ACC by cation analysis using CE-MS. The CE-MS system and conditions were as described (Watanabe et al., 2008).

### 13. GUS staining

Whole plantlets were incubated in heptane for 10 min, washed in 100 mM Tris-HCI/50 mM NaCl (pH 7.0), and subsequently incubated in 5-bromo-4-chloro-3-indolyl-β-D-glucuronide (X-Gluc) buffer [100 mM Tris-HCI/50 mM NaCl buffer (pH 7.0), 2 mM K₃[Fe(CN)₆] and 4 mM X-Gluc]) at 37°C for 24 h. Seedlings were washed in 100 mM Tris-HCI/50 mM NaCl (pH 7.0) and cleared overnight in 90% lactic acid. Samples were photographed with a differential interference contrast microscope (Leica).

### 14. Meristemoid division activity measurements

Leaf 3 was cut from CYCB1;1:DBox-GUS plants at several time points in three independent biological replicates and stained as described above. Using a differential interference contrast microscope (Leica), stained meristemoids were counted in a fixed area near the leaf tip, where all normal cell proliferation had ceased, for 6-12 leaves per experiment. Relative values (compared to control samples) were calculated for each experiment separately and averaged over the replicates.

### 15. Transgenic lines and mutants

Seeds of *ACS5*-inducible overexpressing lines were kindly provided by J. Ecker (SALK Institute, CA, USA). *EBF1*-overexpressing plants were kind gifts of T. Potuschak (CNRS, Strasbourg, France). Ethylene-insensitive mutants were obtained from the Arabidopsis Seed Stock Center (*ein2.5* [N8844], *ein3.1* [N8052], *etr1.3* [N3070], and *ein5.1* [N8053; previously annotated as *ein4*])*.* All transgenic lines and mutants are in Col-0 background.

### 16. Accession numbers

Microarray data are deposited in the GEO database (GSE22107).

### 17. Leaf area measurements

The plant growth and stress set-up was the same as previously described (Skirycz et al (2011) Plant Cell 23: 1876-1888). In short, plants were grown on nylon meshes overlaying control plates, and at 9 DAS meshes were transferred to either control plates (as a transfer control) or to plates containing 25 mM mannitol. 48 hours after stress onset leaf 3 was microdissected, cleared in ethanol and mounted in lactic acid on a glass slide. Slides were photographed, and leaf area was measured using ImageJ 1.41o.

### References

Aguirrezabal, L., Bouchier-Combaud, S., Radziejwoski, A., Dauzat, M., Cookson, S.J., and Granier, C. (2006). Plasticity to soil water deficit in Arabidopsis thaliana: dissection of leaf development into underlying growth dynamic and cellular variables reveals invisible phenotypes. Plant Cell Environ. 29: 2216-2227.
Arvidsson, S., Kwasniewski, M., Riaño-Pachón, D.M., and Mueller-Roeber, B. (2008). QuantPrime -- a flexible tool for reliable high-throughput primer design for quantitative PCR. BMC Bioinformatics 9: 465.1-465.15.
Beemster, G.T.S., De Veylder, L., Vercruysse, S., West, G., Rombaut, D., Van Hummelen, P., Galichet, A., Gruissem, W., Inzé, D., and Vuylsteke, M. (2005). Genome-wide analysis of gene expression profiles associated with cell cycle transitions in growing organs of Arabidopsis. Plant Physiol. 138: 734-743.
Benjamini, Y., and Hochberg, Y. (1995). Controlling the false discovery rate: a practical and powerful approach to multiple testing J. R. Stat. Soc. B-Methodol., 57: 289-300.
Bergmann, D.C., and Sack, F.D. (2007). Stomatal development. Annu. Rev. Plant Biol. 58: 163-181.
Binder, B.M., Mortimore, L.A., Stepanova, A.N., Ecker, J.R., and Bleecker, A.B. (2004). Short-term growth responses to ethylene in Arabidopsis seedlings are EIN3/EIL1 independent. Plant Physiol. 136: 2921-2927.
Burssens, S., Himanen, K., van de Cotte, B., Beeckman, T., Van Montagu, M., Inzé, D., and Verbruggen, N. (2000). Expression of cell cycle regulatory genes and morphological alterations in response to salt stress in Arabidopsis thaliana. Planta 211: 632-640.
Casneuf, T., Van de Peer, Y., and Huber, W. (2007). In situ analysis of cross-hybridisation on microarrays and the inference of expression correlation. BMC Bioinformatics 8: 461.1-461.13.
Churchman, M.L., Brown, M.L., Kato, N., Kirik, V., Hülskamp, M., Inzé, D., De Veylder, L., Walker, J.D., Zheng, Z., Oppenheimer, D.G., Gwin, T., Churchman, J., and Larkin, J.C. (2006). SIAMESE, a plant-specific cell cycle regulator, controls endoreplication onset in Arabidopsis thaliana. Plant Cell 18: 3145-3157.
Colón-Carmona, A., You, R., Haimovitch-Gal, T., and Doerner, P. (1999). Spatio-temporal analysis of mitotic activity with a labile cyclin-GUS fusion protein. Plant J. 20: 503-508.
De Veylder, L., Segers, G., Glab, N., Casteels, P., Van Montagu, M., and Inzé, D. (1997). The Arabidopsis Cks1At protein binds the cyclin-dependent kinases Cdc2aAt and Cdc2bAt. FEBS Lett. 412: 446-452.
De Veylder, L., Beeckman, T., Beemster, G.T.S., Krols, L., Terras, F., Landrieu, I., Van Der Schueren, E., Maes, S., Naudts, M., and Inzé, D. (2001). Functional analysis of cyclin-dependent kinase inhibitors of Arabidopsis. Plant Cell 13: 1653-1667.
Donnelly, P.M., Bonetta, D., Tsukaya, H., Dengler, R.E., and Dengler, N.G. (1999). Cell cycling and cell enlargement in developing leaves of Arabidopsis. Dev. Biol. 215: 407-419.
Fricke, W., Akhiyarova, G., Wei, W., Alexandersson, E., Miller, A., Kjellbom, P.O., Richardson, A., Wojciechowski, T., Schreiber, L., Veselov, D., Kudoyarova, G., and Volkov, V. (2006). The short-term growth response to salt of the developing barley leaf. J. Exp. Bot. 57: 1079-1095.
Fujita, M., Mizukado, S., Fujita, Y., Ichikawa, T., Nakazawa, M., Seki, M., Matsui, M., Yamaguchi-Shinozaki, K., and Shinozaki, K. (2007). Identification of stress-tolerance-related transcription-factor genes via mini-scale Full-length cDNA Over-eXpressor (FOX) gene hunting system. Biochem. Biophys. Res. Commun. 364: 250-257.
Galbraith, D.W., Harkins, K.R., Maddox, J.M., Ayres, N.M., Sharma, D.P., and Firoozabady, E. (1983). Rapid flow cytometric analysis of the cell cycle in intact plant tissues. Science 220: 1049-1051.
Geiss, G.K., Bumgarner, R.E., Birditt, B., Dahl, T., Dowidar, N., Dunaway, D.L., Fell, H.P., Ferree, S., George, R.D., Grogan, T., James, J.J., Maysuria, M., Mitton, J.D., Oliveri, P., Osborn, J.L., Peng, T., Ratcliffe, A.L., Webster, P.J., Davidson, E.H., Hood, L., and Dimitrov, K. (2008). Direct multiplexed measurement of gene expression with color-coded probe pairs. Nat. Biotechnol. 26: 317-325 [Err. Nat. Biotechnol. 26, 709].
Gentleman, R.C., Carey, V.J., Bates, D.M., Bolstad, B., Dettling, M., Dudoit, S., Ellis, B., Gautier, L., Ge, Y., Gentry, J., Hornik, K., Hothorn, T., Huber, W., lacus, S., Irizarry, R., Leisch, F., Li, C., Maechler, M., Rossini, A.J., Sawitzki, G., Smith, C., Smyth, G., Tierney, L., Yang, J.Y.H., and Zhang, J. (2004). Bioconductor: open software development for computational biology and bioinformatics. Genome Biol. 5: R80.1-R80.16.
Goda, H., Sasaki, E., Akiyama, K., Maruyama-Nakashita, A., Nakabayashi, K., Li, W., Ogawa, M., Yamauchi, Y., Preston, J., Aoki, K., Kiba, T., Takatsuto, S., Fujioka, S., Asami, T., Nakano, T., Kato, H., Mizuno, T., Sakakibara, H., Yamaguchi, S., Nambara, E., Kamiya, Y., Takahashi, H., Hirai, M.Y., Sakurai, T., Shinosaki, K., Saito, K., Yoshida, S., and Shimada, Y. (2008). The AtGenExpress hormone and chemical treatment data set: experimental design, data evaluation, model data analysis and data access. Plant J. 55: 526-542.
Granier, C., and Tardieu, F. (1999). Water deficit and spatial pattern of leaf development. Variability in responses can be simulated using a simple model of leaf development. Plant Physiol. 119: 609-619.
Granier, C., Inzé, D., and Tardieu, F. (2000). Spatial distribution of cell division rate can be deduced from that of p34cdc2 kinase activity in maize leaves grown at contrasting temperatures and soil water conditions. Plant Physiol. 124: 1393-1402.
Hacham, Y., Holland, N., Butterfield, C., Ubeda-Tomas, S., Bennett, M.J., Chory J., and Savaldi-Goldstein, S. (2011). Brassinosteroid perception in the epidermis controls root meristem size. Development: in press (doi:10.1242/dev/061804).
Hahn, A., and Harter, K. (2009). Mitogen-activated protein kinase cascades and ethylene: signaling, biosynthesis, or both? Plant Physiol. 149: 1207-1210.
Hsiao, T.C., and Xu, L.-K. (2000). Sensitivity of growth of roots versus leaves to water stress: biophysical analysis and relation to water transport. J. Exp. Bot. 51: 1595-1616.
Inzé, D., and De Veylder, L. (2006). Cell cycle regulation in plant development. Annu. Rev. Genet. 40: 77-105.
Irizarry, R.A., Bolstad, B.M., Collin, F., Cope, L.M., Hobbs, B., and Speed, T.P. (2003a). Summaries of Affymetrix GeneChip probe level data. Nucleic Acids Res. 31: e15.
Irizarry, R.A., Hobbs, B., Collin, F., Beazer-Barclay, Y.D., Antonellis, K.J., Scherf, U., and Speed, T.P. (2003b). Exploration, normalization, and summaries of high density oligonucleotide array probe level data. Biostatistics 4: 249-264.
Kalantari, K.M., Smith, A.R., and Hall, M.A. (2000). The effect of water stress on 1-(malonylamino)cyclopropane-l-carboxylic acid concentration in plant tissues. Plant Growth Regul. 31: 183-193.
Kant, P., Kant, S., Gordon, M., Shaked, R., and Barak, S. (2007). STRESS RESPONSE SUPPRESSOR1 and STRESS RESPONSE SUPPRESSOR2, two DEAD-box RNA helicases that attenuate Arabidopsis responses to multiple abiotic stresses. Plant Physiol. 145: 814-830.
Marcotrigiano, M. (2010). A role for leaf epidermis in the control of leaf size and the rate and extent of mesophyll cell division. Am. J. Bot. 97: 224-233.
Menges, M., Hennig, L., Gruissem, W., and Murray, J.A.H. (2003). Genome-wide gene expression in an Arabidopsis cell suspension. Plant Mol. Biol. 53: 423-442.
Murashige, T., and Skoog, F. (1962). A revised medium for rapid growth and bio assays with tobacco tissue cultures. Physiol. Plant. 15: 473-497.
Olmedo, G., Guo, H., Gregory, B.D., Nourizadeh, S.D., Aguilar-Henonin, L., Li, H., An, F., Guzman, P., and Ecker, J.R. (2006). ETHYLENE-INSENSITIVE5 encodes a 5'→3' exoribonuclease required for regulation of the EIN3-targeting F-box proteins EBF1/2. Proc. Natl. Acad. Sci. USA 103: 13286-13293.
Papdi, C., Ábráham, E., Prathiba Joseph, M., Popescu, C., Koncz, C., and Szabados, L. (2008). Functional identification of Arabidopsis stress regulatory genes using the controlled cDNA overexpression system. Plant Physiol. 147: 528-542.
Peres, A., Churchman, M.L., Hariharan, S., Himanen, K., Verkest, A., Vandepoele, K., Magyar, Z., Hatzfeld, Y., Van Der Schueren, E., Beemster, G.T.S., Frankard, V., Larkin, J.C., Inzé, D., and De Veylder, L. (2007). Novel plant-specific cyclin-dependent kinase inhibitors induced by biotic and abiotic stresses. J. Biol. Chem. 282: 25588-25596.
Pettkó-Szandtner, A., Mészáros, T., Horváth, G.V., Bakó, L., Csordás-Tóth, E., Blastyák, A., Zhiponova, M., Miskolczi, P., and Dudits, D. (2006). Activation of an alfalfa cyclin-dependent kinase inhibitor by calmodulin-like domain protein kinase. Plant J. 46: 111-123.
Pierik, R., Tholen, D., Poorter, H., Visser, E.J.W., and Voesenek, L.A.C.J. (2006). The Janus face of ethylene: growth inhibition and stimulation. Trends Plant Sci. 11: 176-183.
Pillitteri, L.J., Sloan, D.B., Bogenschutz, N.L., and Torii, K.U. (2007). Termination of asymmetric cell division and differentiation of stomata. Nature 445: 501-505.
Poiré, R., Wiese-Klinkenberg, A., Parent, B., Mielewczik, M., Schurr, U., Tardieu, F., and Walter, A. (2010). Diel time-courses of leaf growth in monocot and dicot species: endogenous rhythms and temperature effects. J. Exp. Bot. 61: 1751-1759.
Potuschak, T., Vansiri, A., Binder, B.M., Lechner, E., Vierstra, R.D., and Genschik, P. (2006). The exoribonuclease XRN4 is a component of the ethylene response pathway in Arabidopsis. Plant Cell 18: 3047-3057.
Rymen, B., Fiorani, F., Kartal, F., Vandepoele, K., Inzé, D., and Beemster, G.T.S. (2007). Cold nights impair leaf growth and cell cycle progression in maize through transcriptional changes of cell cycle genes. Plant Physiol. 143: 1429-1438.
Savaldi-Goldstein, S., Peto, C., and Chory, J. (2007). The epidermis both drives and restricts plant shoot growth. Nature 446: 199-202.
Schuppler, U., He, P.-H., John, P.C.L., and Munns, R. (1998). Effect of water stress on Cdc2-like cell-cycle kinase activity in wheat leaves. Plant Physiol. 117: 667-678 [Err. Plant Physiol. 117, 1528].
Sharp, R.E., and LeNoble, M.E. (2002). ABA, ethylene and the control of shoot and root growth under water stress. J. Exp. Bot. 53: 33-37.
Skirycz, A., and Inzé, D. (2010). More from less: plant growth under limited water. Curr. Opin. Biotechnol. 21: 197-203.
Skirycz, A., De Bodt, S., Obata, T., De Clercq, I., Claeys, H., De Rycke, R., Andriankaja, M., Van Aken, O., Van Breusegem, F., Fernie, A.R., and Inzé, D. (2010). Developmental stage specificity and the role of mitochondrial metabolism in the response of Arabidopsis leaves to prolonged mild osmotic stress. Plant Physiol. 152: 226-244.
Smyth, G.K. (2004). Linear models and empirical Bayes methods for assessing differential expression in microarray experiments. Stat. Appl. Genet. Mol. Biol. 3: Article 3 (http//www.bepress.com/sagmb/vol3/iss1/art3).
Sobeih, W.Y., Dodd, I.C., Bacon, M.A., Grierson, D., and Davies, W.J. (2004). Long-distance signals regulating stomatal conductance and leaf growth in tomato (Lycopersicon esculentum) plants subjected to partial root-zone drying. J. Exp. Bot. 55: 2353-2363.
Tardieu, F. (2003). Virtual plants: modelling as a tool for the genomics of tolerance to water deficit. Trends Plant Sci. 8: 9-14.
Tardieu, F., Reymond, M., Hamard, H., Granier, C., and Muller, B. (2000). Spatial distributions of expansion rate, cell division rate and cell size in maize leaves: a synthesis of the effects of soil water status, evaporative demand and temperature. J. Exp. Bot. 51: 1505-1514.
Thimm, O., Bläsing, O., Gibon, Y., Nagel, A., Meyer, S., Krüger, P., Selbig, J., Müller, L.A., Rhee, S.Y., and Stitt, M. (2004). MAPMAN: a user-driven tool to display genomics data sets onto diagrams of metabolic pathways and other biological processes. Plant J. 37: 914-939.
Usadel, B., Nagel, A., Steinhauser, D., Gibon, Y., Bläsing, O.E., Redestig, H., Sreenivasulu, N., Krall, L., Hannah, M.A., Poree, F., Fernie, A.R., and Stitt, M. (2006). PageMan: an interactive ontology tool to generate, display, and annotate overview graphs for profiling experiments. BMC Bioinformatics 7: 535.1-535.8.
Van Leene, J., Stals, H., Eeckhout, D., Persiau, G., Van De Slijke, E., Van Isterdael, G., De Clercq, A., Bonnet, E., Laukens, K., Remmerie, N., Henderickx, K., De Vijlder, T., Abdelkrim, A., Pharazyn, A., Van Onckelen, H., Inzé, D., Witters, E., and De Jaeger, G. (2007). A tandem affinity purification-based technology platform to study the cell cycle interactome in Arabidopsis thaliana. Mol. Cell. Proteomics 6: 1226-1238.
Veselov, D.S., Mustafina, A.R., Sabirjanova, I.B., Akhiyarova, G.R., Dedov, A.V., Veselov, S.U., and Kudoyarova, G.R. (2002). Effect of PEG-treatment on the leaf growth response and auxin content in shoots of wheat seedlings. Plant Growth Regul. 38: 191-194.
Wang, H., Qi, Q., Schorr, P., Cutler, A.J., Crosby, W.L., and Fowke, L.C. (1998). ICK1, a cyclin-dependent protein kinase inhibitor from Arabidopsis thaliana interacts with both Cdc2a and CycD3, and its expression is induced by abscisic acid. Plant J. 15: 501-510.
Watanabe, M., Kusano, M., Oikawa, A., Fukushima, A., Noji, N., and Saito, K. (2008). Physiological roles of the β-substituted alanine synthase gene family in Arabidopsis. Plant Physiol. 146: 310-320.
West, G., Inzé, D., and Beemster, G.T.S. (2004). Cell cycle modulation in the response of the primary root of Arabidopsis to salt stress. Plant Physiol. 135: 1050-1058.
Xu, J., Li, Y., Wang, Y., Liu, H., Lei, L., Yang, H., Liu, G., and Ren, D. (2008). Activation of MAPK kinase 9 induces ethylene and camalexin biosynthesis and enhances sensitivity to salt stress in Arabidopsis. J. Biol. Chem. 283: 26996-27006.
Yoo, S.-D., Cho, Y.-H., Tena, G., Xiong, Y., and Sheen, J. (2008). Dual control of nuclear EIN3 by bifurcate MAPK cascades in C2H4 signalling. Nature 451: 789-795.

## Claims

1. A method for producing an abiotic stress tolerant plant relative to a control plant, by decreasing the ethylene signal transduction pathway in said plant during the period of abiotic stress imposed on said plants comprising introducing and expressing in said plant a chimeric gene comprising of a plant 4TM promoter selected from the list consisting of SEQ ID NO: 1, SEQ ID NO: 2, SEQ ID NO: 3 or SEQ ID NO: 4, or a functionally equivalent orthologous plant 4TM promoter of SEQ ID NO: 1, SEQ ID NO: 2, SEQ ID NO: 3 or SEQ ID NO: 4 wherein said promoter is operably linked to a gene or gene fragment derived from the ethylene signal transduction pathway.

2. A method according to claim 1 wherein the expression of said chimeric gene in said plant leads to a downregulation of at least one gene of the following list: ACO1, ACO2, ETR2, ETR1, CTR1, MKK9, MKK7, EIN3, EIN5 or EIL1.

3. A method according to claim 1 wherein the expression of said chimeric gene in said plant leads to an upregulation of at least one gene of the following list: EBF1, EBF2, ACC deaminase, a dominant negative ethylene receptor or a dominant negative transcription factor EIN3 or EIL1.

4. A chimeric gene comprising the following operably linked DNA elements: a) a plant 4TM promoter selected from the list consisting of SEQ ID NO: 1, SEQ ID NO: 2, SEQ ID NO: 3 or SEQ ID NO: 4, or a functionally equivalent orthologous plant 4TM promoter of SEQ ID NO: 1, SEQ ID NO: 2, SEQ ID NO: 3 or SEQ ID NO: 4, b) a DNA region which when transcribed yields a double-stranded RNA molecule capable of reducing the expression of a gene from the following list: ACO1, ACO2, ETR2, ESR1, CTR1, MKK9, MKK7, EIN3, EIN5 or EIL1 and c) a 3' end region comprising transcription termination and polyadenylation signals functioning in cells of said plant.

5. A chimeric gene comprising the following operably linked DNA elements: a) a plant 4TM promoter selected from the list consisting of SEQ ID NO: 1, SEQ ID NO: 2, SEQ ID NO: 3 or SEQ ID NO: 4, or a functionally equivalent orthologous plant 4TM promoter of SEQ ID NO: 1, SEQ ID NO: 2, SEQ ID NO: 3 or SEQ ID NO: 4, b) a DNA region encoding for a gene of the following list: EBF1, EBF2, ACC deaminase, a dominant negative ethylene receptor or a dominant negative transcription factor EIN3 or EIL1 and c) a 3' end region comprising transcription termination and polyadenylation signals functioning in cells of said plant.

6. A transgenic plant or a transgenic seed or a transgenic plant cell comprising a chimeric gene according to claim 4 or according to claim 5.

7. A transgenic plant or transgenic seed or transgenic plant cell according to claim 6 wherein said plant, seed or cell is a crop plant or a monocot or a cereal such as rice, wheat, maize, barley, millet, rye, triticale, sorghum emmer, spelt, secale, einkorn, teff, milo and oats.

## Patentansprüche

1. Verfahren zur Produktion einer Pflanze, die bezüglich einer Kontrollpflanze gegen abiotischen Stress tolerant ist, durch Verringern des Ethylen-Signaltransduktionswegs in der Pflanze während des Zeitraums des abiotischen Stresses, der den Pflanzen auferlegt wird, wobei das Verfahren das Einführen und Exprimieren eines chimären Gens in der Pflanze umfasst, das einen Pflanzen-4TM-Promotor, der aus der Liste bestehend aus SEQ ID Nr. 1, SEQ ID Nr. 2, SEQ ID Nr. 3 oder SEQ ID Nr. 4 ausgewählt ist, oder einen funktionell äquivalenten orthologen Pflanzen-4TM-Promotor von SEQ ID Nr. 1, SEQ ID Nr. 2, SEQ ID Nr. 3 oder SEQ ID Nr. 4 umfasst, wobei der Promotor mit einem Gen oder Genfragment, das von dem Ethylen-Signaltransduktionsweg abgeleitet ist, operativ verknüpft ist.

2. Verfahren nach Anspruch 1, wobei die Expression des chimären Gens in der Pflanze zu einer Herunterregulation mindestens eines Gens der folgenden Liste führt: ACO1, ACO2, ETR2, ETR1, CTR1, MKK9, MKK7, EIN3, EIN5 oder EIL1.

3. Verfahren nach Anspruch 1, wobei die Expression des chimären Gens in der Pflanze zu einer Hochregulation mindestens eines Gens der folgenden Liste führt: EBF1, EBF2, ACC-Deaminase, ein dominanter negativer Ethylenrezeptor oder ein dominanter negativer Transkriptionsfaktor EIN3 oder EIL1.

4. Chimäres Gen, das die folgenden operativ verknüpften DNA-Elemente umfasst: a) einen Pflanzen-4TM-Promotor, der aus der Liste bestehend aus SEQ ID Nr. 1, SEQ ID Nr. 2, SEQ ID Nr. 3 oder SEQ ID Nr. 4 ausgewählt ist, oder einen funktionell äquivalenten orthologen Pflanzen-4TM-Promotor von SEQ ID Nr. 1, SEQ ID Nr. 2, SEQ ID Nr. 3 oder SEQ ID Nr. 4, b) eine DNA-Region, die bei Transkription ein doppelsträngiges DNA-Molekül ergibt, das die Expression eines Gens aus der folgenden Liste verringern kann: ACO1, ACO2, ETR2, ESR1, CTR1, MKK9, MKK7, EIN3, EIN5 oder EIL1, und c) eine 3'-Ende-Region, die Transkriptionsterminations- und Polyadenylierungssignale umfasst, die in Zellen der Pflanze funktionieren.

5. Chimäres Gen, das die folgenden operativ verknüpften DNA-Elemente umfasst: a) einen Pflanzen-4TM-Promotor, der aus der Liste bestehend aus SEQ ID Nr. 1, SEQ ID Nr. 2, SEQ ID Nr. 3 oder SEQ ID Nr. 4 ausgewählt ist, oder einen funktionell äquivalenten orthologen Pflanzen-4TM-Promotor von SEQ ID Nr. 1, SEQ ID Nr. 2, SEQ ID Nr. 3 oder SEQ ID Nr. 4, b) eine DNA-Region, die für ein Gen der folgenden Liste kodiert: EBF1, EBF2, ACC-Deaminase, ein dominanter negativer Ethylenrezeptor oder ein dominanter negativer Transkriptionsfaktor EIN3 oder EIL1, und c) eine 3'-Ende-Region, die Transkriptionsterminations- und Polyadenylierungssignale umfasst, die in Zellen der Pflanze funktionieren.

6. Transgene Pflanze oder transgener Samen oder transgene Pflanzenzelle, die bzw. der ein chimäres Gen nach Anspruch 4 oder nach Anspruch 5 umfasst.

7. Transgene Pflanze oder transgener Samen oder transgene Pflanzenzelle nach Anspruch 6, wobei die Pflanze, der Samen oder die Zelle eine Nutzpflanze oder eine Monokotyledone oder eine Getreidepflanze ist, wie Reis, Weizen, Mais, Gerste, Hirse, Roggen, Triticale, Sorghum, Emmer, Dinkel, Secale, Einkorn, Teff, Milo und Hafer.

## Revendications

1. Procédé de production d'une plante tolérant le stress abiotique par rapport à une plante témoin, en diminuant la voie de transduction du signal d'éthylène dans ladite plante au cours de la période de stress abiotique imposée auxdites plantes, comprenant introduire et exprimer dans ladite plante un gène chimère comprenant un promoteur 4TM de plante sélectionné parmi la liste constituée de SEQ ID N° : 1, SEQ ID N° : 2, SEQ ID N° : 3 ou SEQ ID N° : 4, ou un promoteur 4TM de plante orthologue équivalent du point de vue fonctionnel de SEQ ID N° : 1, SEQ ID N° : 2, SEQ ID N° : 3 ou SEQ ID N° : 4, dans lequel ledit promoteur est lié de manière opérationnelle à un gène ou fragment de gène dérivé de la voie de transduction du signal d'éthylène.

2. Procédé selon la revendication 1, dans lequel l'expression dudit gène chimère dans ladite plante conduit à une régulation à la baisse d'au moins un gène de la liste suivante : ACO1, ACO2, ETR2, ETR1, CTR1, MKK9, MKK7, EIN3, EIN5 ou EIL1.

3. Procédé selon la revendication 1, dans lequel l'expression dudit gène chimère dans ladite plante conduit à une régulation à la hausse d'au moins un gène de la liste suivante : EBF1, EBF2, ACC désaminase, un récepteur d'éthylène négatif dominant ou un facteur de transcription négatif dominant EIN3 ou EIL1.

4. Gène chimère comprenant les éléments d'ADN liés de manière opérationnelle suivants : a) un promoteur 4TM de plante sélectionné parmi la liste constituée de SEQ ID N° : 1, SEQ ID N° : 2, SEQ ID N° : 3 ou SEQ ID N° : 4, ou un promoteur 4TM de plante orthologue équivalent du point de vue fonctionnel de SEQ ID N° : 1, SEQ ID N° : 2, SEQ ID N° : 3 ou SEQ ID N° : 4, b) une région d'ADN qui donne lors de sa transcription une molécule d'ARN à double brin capable de réduire l'expression d'un gène parmi la liste suivante: ACO1, ACO2, ETR2, ESR1, CTR1, MKK9, MKK7, EIN3, EIN5 ou EIL1 et c) une région terminale en 3' comprenant des signaux de terminaison de la transcription et de polyadénylation fonctionnant dans des cellules de ladite plante.

5. Gène chimère comprenant les éléments d'ADN liés de manière opérationnelle suivants : a) un promoteur 4TM de plante sélectionné parmi la liste constituée de SEQ ID N° : 1, SEQ ID N° : 2, SEQ ID N° : 3 ou SEQ ID N° : 4, ou un promoteur 4TM de plante orthologue équivalent du point de vue fonctionnel de SEQ ID N° : 1, SEQ ID N° : 2, SEQ ID N° : 3 ou SEQ ID N° : 4, b) une région d'ADN codant pour un gène de la liste suivante : EBF1, EBF2, ACC désaminase, un récepteur d'éthylène négatif dominant ou un facteur de transcription négatif dominant EIN3 ou EIL1 et c) une région terminale en 3' comprenant des signaux de terminaison de la transcription et de polyadénylation fonctionnant dans des cellules de ladite plante.

6. Plante transgénique ou graine transgénique ou cellule végétale transgénique comprenant un gène chimère selon la revendication 4 ou selon la revendication 5.

7. Plante transgénique ou graine transgénique ou cellule végétale transgénique selon la revendication 6, dans laquelle ladite plante, graine ou cellule est une plante cultivée ou des monocotylées ou une céréale telle que le riz, le blé, le maïs, l'orge, le millet, le seigle, le triticale, l'amidonnier de sorgho, l'épeautre, le fourrage de seigle, l'engrain, le tef, le milo et l'avoine.


### SEQUENCE LISTING

<110> VIB VZW UNIVERSITEIT GENT
<120> Methods and means to produce abiotic stress tolerant plants
<130> DI/ETHY/372
<150> US 61/517,622
   <151> 2011-04-22
<150> GB 1106845.9
   <151> 2011-04-26
<160> 8
<170> PatentIn version 3.5
<210> 1
   <211> 391
   <212> DNA
   <213> Arabidopsis thaliana
<400> 1
   cgtatccttg tctcggataa acatgaatca aatacacggc ccattaggcc ccactatagt        60
   tattactact acagtatatt atacccggat aaacatgacc aatacacggc ccactaggcc       120
   caccttataa ttattatatt ataatcaatc attgggccca ccttattaaa atcaaactag       180
   ccgttacaaa aacagccgtt ccagattcaa caacggtcat atttagaaac tttaccttct       240
   acaatctctc tcttcactaa cttctacata aacaaactcg ttgcgctctt ctcaattcca       300
   cgaatcacac actcttcttc ttcttctgtt catcaaagaa aacgaaaccc atttctcaaa       360
   atccaaaatt ttcaaaaact caaaactcat c                                      391
<210> 2
   <211> 649
   <212> DNA
   <213> Arabidopsis thaliana
<400> 2
   ggttcttaaa ttttagaagc agctcaagat cttggatcga atcattcttc ttctttcaat        60
   ctgagagtaa agaagagaag aagaaacagt ttttgtccta tttgcagtta tggtttcatc       120
   aagactttac tctgtttcaa tggtattgaa acggccttca agtttcatct tgaataaact       180
   gtgtgaaccg aattaaagat tcacaattcg ttttttttgt tcatcaaata attagttcgg        240
   ttggaatgag tcggttcaat ttaagcagac gaaaacacac aagacataaa cttatatttg       300
   cttcgtaagg ataacatatc cgtgttcagg ataaaaatat ccttgtctcg gacaaacaag       360
   aacccaactt ggcccattgg gcttgccagt cttgttctgg aacaaaggaa tagcaaaaaa       420
   cactagccgt tacacacaca acaacaactc actctgtcgg tgatctgtcc ttcacttctg       480
   taaagcctca acggtcatat ttggaaaatc agaccgttta aatcgctctc tacataaacc       540
   caaattcata actcacccaa tcacaattcg caagctatct tcctttcttc ttcgcaatct       600
   caaaatccaa aattttctgc aacccgaatc agaaacctcc aacatcgtc                   649
<210> 3
   <211> 3618
   <212> DNA
   <213> Arabidopsis thaliana
<400> 3
   aacttaataa atatgcaaaa taatatatat gtgtttctta tatgtgaaaa aataatatat 60
   aatgtaagta tgtatcttta atcaatatac taatttcctg aaattatttt atttatacta 120
   atcaaattga catggaaaaa tggtgagaag tataatcacc taattaaaaa aaaaatcata 180
   aaatcgaata ttgttctcat catctctcta catcaatgtt tagatcattt ttttaatctc 240
   ttgctaaaac tagtccaatt tgttcactac cgaatcatta atttataggt ttgctcttta 300
   caattccgta taaatgatta gaaatgattg tgaaagagta gagcaatatc gagttatcta 360
   aaaatgtttt tgtcacttgc tcataacaat aacatttttg tgctacgaat gtcaaaattt 420
   caggatttgt tagttagagt attagagagg atgaattgtt tttaaaaaaa attgtttttc 480
   ttgtttttgt tttttgtttt ttgtttttgt tttcttttgt tttttaatag aatttaatag 540
   ttgatgtatt gcccagtgag ccaaaaatta tgtatatatt taagaaagat attatttagt 600
   tagtttatga atatatttgt aaacttaggt caatgatgca aaaaatgaaa cttgatttgt 660
   aagtatggtc attgatgtaa atttatgtgg atcacacgct tttttacata caaagtgttt 720
   tccactgaat tcattcttaa aacttcatca tatactagat gattttgagg aaagatttga 780
   gggaaaaatt agcaaatggg ttgtgatgat tataaccgaa aagcaaaaga tggtttattg 840
   tgtatacata caactgtttg acccaacgaa agaaacaagc tcatatgcac cattggcttt 900
   tttagttgct gctaatcttg ctttattaat gaaatgagcg tttaatatga agaaaacatc 960
   ggactttttt ccatgaaata aaagacttag aaactaccat tttaaaggat aacaaagaca 1020
   tttacattta actgcattta tgtatttaat attaaagaaa atatgctact caaaatggcg 1080
   tcagttaagc attacagtaa agccactaat tctcgtttat tgttttgaac tcctgattta 1140
   tgcaaggcac aatttgtttt tgacgtttga gactgttact cttttttctt tttttttttt 1200
   tgatatgaga cttacaactg caatttgact taacttggat ttatattcat tttgatgact 1260
   gtaaattttg ggaaacaaat acatgtaata tttaaccaca aaaaatctga gtctttaaca 1320
   aaatagtgag cgcgcttttt tctattatat aaattttgag ttatgataac aaaacataat 1380
   aaaatagata gtttatcttc ttaggaatta agtaatcaag gatcgttgtt tcatatattc 1440
   ttgtaaaaat aatatgatta ttaggtattt aatatttata taataagata atatttctag 1500
   tttaaaatta aatattagtc tttaacaaaa cgatgtgcac attttttctt ttaaaattgt 1560
   tatcataaat tttaagttat acttttaatt tcaatttgca tagttgatgc ttaagaaaat 1620
   ttccaattga tctatttgtt tagaaatttt aagaaaatat cattattgtg gtgtgtgatg 1680
   gatcatggta gtgtttgatg aatatttttg tttagttatt gtaaggaaat atgtgattgt 1740
   gtgaatgatt tattattcac tatgaatttg ataacagaaa agtgtgggta cgatatatcg 1800
   gatatatatc ttatatctat tatatattga agagaaatat aagtgtcagt caaattaaat 1860
   ttattaaaat ctgagttttt ttctattaat ttaattttta atatttttca atccctaaat 1920
   gggcttcgaa tcctctaaaa aaatagatgt ttgaatcctc ataacaaaat ctgaattgga 1980
   atcctcataa caaaataaat gttttcccaa actatatata aagcttattg atgtgaaggg 2040
   acttccacta cgctttctag tcagtttgaa gatctctctt tcccttattg ctcttgtttc 2100
   tagccatgtt tagtttaggg gagtcttcta tatcaagtta atttcataac attgcctaaa 2160
   gtttcaccgc ttaaggattt agtgatgatt tgatcctcaa atgtgtatgc ttccatattt 2220
   tttagccttt ttaggattta ctttgtaatc tctgaatcat tcatttcttg aaatgatatt 2280
   tacattctta tcaaaacaaa tctacatata aagcttacat acctttatct tctttgtagc 2340
   cttgtaggta atggcgactt aatacgttca ctttgtagct agctatggtt ttttatatgt 2400
   ttgcttgtaa cttaatccct tctggggata tggatatata tagtgtccaa ccacgaattt 2460
   taatgtgtta aaaagagttt taatttatac taaataattt taaaacatct caaaattgat 2520
   ttaaattaat atttggtagt tagttcgtgt attacatttg tatatttatt aaaataatga 2580
   aataagtatg actagtatat atttttataa taatattaag gatttattgg tagtttatct 2640
   tgaaaaatta aattttgtga atttagtcaa aattcatgca tcaaatggtt ttatttgact 2700
   aacatgtttt taattacttt atcactacaa gatataccat tttttaaaag tcatagcata 2760
   gttttttttt ttttaattta tatacaaatt actgttatta aaaactgttt gttacgtttg 2820
   attgtatatt agatctttaa ttataagaca taaagatatt tttaaaaaat cattaggagg 2880
   atgttacaaa atattcaatt tatatccata atcatttaca tttttaaaga tgtccccgtt 2940
   accaaatgcg atgtgtgtcc gttcccatat gaaacaagac atttttgtcg aaatttgtat 3000
   tggcaataat atctcacatt tgtaaaactt tgtattgttg aatttcaatt tcaaatggcg 3060
   ttttctacaa ctcttttaga atattaaccc gctattctta acatttaaac taatgtgacc 3120
   attctaaaat tgatattccc tttacgtgtt ggactttatt aataaactga ttaacaaata 3180
   aagtgtatta tttatcatca taatatattt agcaaactca atttcttatc aattgtgtct 3240
   acccaataaa ctaattttta taaatctcat tttatgaaac aaattagtgt cttcagtggg 3300
   acatccgaca ttcacatagt tgaccttaaa acttacaaaa ccatactatt aaaaaccaat 3360
   taaaaaacaa gattaaaaag ttatcgagcc gttacacctt aattgaatta ttctgtcttc 3420
   gtccttaagt cataacggtc atattttcaa aaacaaaaac gaccgttaat cccaacaaaa     3480
   tctctacttt ctcttacctc aactttgttt cttatgtatg catataaacc ccaaatcaaa     3540
   gtgaacttat caagaaacac aaaaacaaac atcaaaaccc caaaaaaaaa ccaaagttct     3600
   cttcttgaat tcgagaaa                                                   3618
<210> 4
   <211> 1997
   <212> DNA
   <213> Arabidopsis thaliana
<400> 4
   tccaattgat ctatttgttt agaaatttta agaaaatatc attattgtgg tgtgtgatgg 60
   atcatggtag tgtttgatga atatttttgt ttagttattg taaggaaata tgtgattgtg 120
   tgaatgattt attattcact atgaatttga taacagaaaa gtgtgggtac gatatatcgg 180
   atatatatct tatatctatt atatattgaa gagaaatata agtgtcagtc aaattaaatt 240
   tattaaaatc tgagtttttt tctattaatt taatttttaa tatttttcaa tccctaaatg 300
   ggcttcgaat cctctaaaaa aatagatgtt tgaatcctca taacaaaatc tgaattggaa 360
   tcctcataac aaaataaatg ttttcccaaa ctatatataa agcttattga tgtgaaggga 420
   cttccactac gctttctagt cagtttgaag atctctcttt cccttattgc tcttgtttct 480
   agccatgttt agtttagggg agtcttctat atcaagttaa tttcataaca ttgcctaaag 540
   tttcaccgct taaggattta gtgatgattt gatcctcaaa tgtgtatgct tccatatttt 600
   ttagcctttt taggatttac tttgtaatct ctgaatcatt catttcttga aatgatattt 660
   acattcttat caaaacaaat ctacatataa agcttacata cctttatctt ctttgtagcc 720
   ttgtaggtaa tggcgactta atacgttcac tttgtagcta gctatggttt tttatatgtt 780
   tgcttgtaac ttaatccctt ctggggatat ggatatatat agtgtccaac cacgaatttt 840
   aatgtgttaa aaagagtttt aatttatact aaataatttt aaaacatctc aaaattgatt 900
   taaattaata tttggtagtt agttcgtgta ttacatttgt atatttatta aaataatgaa 960
   ataagtatga ctagtatata tttttataat aatattaagg atttattggt agtttatctt 1020
   gaaaaattaa attttgtgaa tttagtcaaa attcatgcat caaatggttt tatttgacta 1080
   acatgttttt aattacttta tcactacaag atataccatt ttttaaaagt catagcatag 1140
   tttttttttt tttaatttat atacaaatta ctgttattaa aaactgtttg ttacgtttga 1200
   ttgtatatta gatctttaat tataagacat aaagatattt ttaaaaaatc attaggagga 1260
   tgttacaaaa tattcaattt atatccataa tcatttacat ttttaaagat gtccccgtta 1320
   ccaaatgcga tgtgtgtccg ttcccatatg aaacaagaca tttttgtcga aatttgtatt 1380
   ggcaataata tctcacattt gtaaaacttt gtattgttga atttcaattt caaatggcgt 1440
   tttctacaac tcttttagaa tattaacccg ctattcttaa catttaaact aatgtgacca 1500
   ttctaaaatt gatattccct ttacgtgttg gactttatta ataaactgat taacaaataa 1560
   agtgtattat ttatcatcat aatatattta gcaaactcaa tttcttatca attgtgtcta 1620
   cccaataaac taatttttat aaatctcatt ttatgaaaca aattagtgtc ttcagtggga 1680
   catccgacat tcacatagtt gaccttaaaa cttacaaaac catactatta aaaaccaatt 1740
   aaaaaacaag attaaaaagt tatcgagccg ttacacctta attgaattat tctgtcttcg 1800
   tccttaagtc ataacggtca tattttcaaa aacaaaaacg accgttaatc ccaacaaaat 1860
   ctctactttc tcttacctca actttgtttc ttatgtatgc atataaaccc caaatcaaag 1920
   tgaacttatc aagaaacaca aaaacaaaca tcaaaacccc aaaaaaaaac caaagttctc 1980
   ttcttgaatt cgagaaa 1997
<210> 5
   <211> 2019
   <212> DNA
   <213> Arabidopsis thaliana
<400> 5
   cgtggtcatc gcaaggtaac catctttatt ttcttgggtt gatcttcact agctatatat      60
   taaccaatta catatgtatt attttgtact gaagaaagga tataatttgt aatcgccaaa     120
   tgattgaatt agtatttgct aattacaaaa atttgtagaa atcgcgataa ttatcaactt     180
   ttttactata tcccatcatt ctactattac gcaatgttat caattgtact agtttgtttc     240
   atccactatg taaaatcatt caaaccctaa agtcgatgct catcagctca tgtatatgta     300
   tgtatcatgt atatgtacat aagatataaa acctttatag atgtgcatgt aaaagaatgg     360
   tagatagtag tagtaggtgt gtaaggagaa agatctaggg gcttcaaaaa aaggagagaa     420
   aaggacagag catgtgaaaa gagacagaga aacaaacaga ggagtgaaca gtcttactct     480
   tttttttttc tatgcactta atgaacccaa ccctctctgt atatttctga ttcttcacat     540
   gtcttcctct tcttttttat tccgaaaact tttcttcagt aaaaaccaat gccgaaaaaa      600
   atacataata aaaacgacaa aatatctaac attttttata taaaaaggac aaaagtactt     660
   tcacttcact tgcaaccaaa caaacctagt ccccttcttt atccaatggt ctctcccctg     720
   ctctctattt ctcatctaaa cacccttttc tttctttctt atttaccatt ttgccaactt     780
   atgtaaaaga tgttaagaag aaaatgtgaa aatttagtaa aaatctctct tttgtattgt     840
   cgtcagaaac tgtaaaataa tcttactctt tttcaagtca gcatataagt ttggtttctt     900
   ctcccgagtc agagattcgt gcagtgatga tacaagaagc ttattccgaa ttcttttcat     960
   aaatactcgt atttccttct tctttttttt tttctttttt ttaacatcta tctgttcttc    1020
   ttgcatctat ctgattttcc tctgtctttt agacagtaat aacagagctc actaaatgta    1080
   ataaataaaa aaatgagctc aataaaaagg taaatggcac tcatttttag gccatcaagt 1140
   gttgggatac agctgaaatt aaaaaggcgt cagctcaaag gaaaaaataa ataaaaaaga 1200
   ttcccaaaaa tcaatgcact tttatagaaa aaaatacaca taatttcaaa aaatacttaa 1260
   attttgaaaa ataagatgaa gaaaggattg agaagagagg aaagagtagt cagagagaca 1320
   ggagcatcaa gtgctgcaga agaagcaaca ggaacactgg gcctgagtct cttcagattt 1380
   gccagtcttt ataggaccac actcttcttc ttcatccccc ttacatacat tttatctatt 1440
   tttacatttt acaatattgg atattttaat gttgttttga ttttacacta catggcaaaa 1500
   ctccttaaaa gctcttgtcc tgatgtgatt cttttttctt ttcaacactg tctaaatatc 1560
   tctctcttct ttgtgatcac tctttgattt attaactttc tgattaccat tccccccaaa 1620
   ataaacccta ctagagtgtg actaattgta tagtaatctc tattgtgtaa taatctttta 1680
   tctttcctca atacaaaaaa gtagggtata tatataaaag agggagtggc ctcgtgtaga 1740
   gaaagaacct agctagtgtt catgcaattt gcatctgagg aatatatgct tagaaagaga 1800
   gagagagaga gagagagaga gagagagaga gagagagata tcaaaccaga ggaaaagagt 1860
   gacagagaaa gtggtcatct ttgtctgtct ctgtccgaaa ctcaggagcc ctaatctctg 1920
   aatccatcaa gaaagctctt tctcttcctt cttttatgct tctgaatttg ctacatatat 1980
   aaactatctg aagaaagcta gagaataaga tgatgagtc 2019
<210> 6
   <211> 1119
   <212> DNA
   <213> cauliflower mosaic virus
<400> 6
   atttaggtga cactatagaa tactcaagct atgcatccaa cgcgttggga gctctcccat 60
   atggtcgaga tctcctttgc cccggagatc accatggacg actttctcta tctctacgat 120
   ctaggaagaa agttcgacgg agaaggtgac gataccatgt tcaccaccga taatgagaag 180
   attagcctct tcaatttcag aaagaatgct gacccacaga tggttagaga ggcctacgcg 240
   gcaggtctca tcaagacgat ctacccgagt aataatctcc aggagatcaa ataccttccc 300
   aagaaggtta aagatgcagt caaaagattc aggactaact gcatcaagaa cacagagaaa 360
   gatatatttc tcaagatcag aagtactatt ccagtatgga cgattcaagg cttgcttcat 420
   aaaccaaggc aagtaataga gattggagtc tctaagaaag tagttcctac tgaatcaaag 480
   gccatggagt caaaaattca gatcgaggat ctaacagaac tcgccgtgaa gactggcgaa 540
   cagttcatac agagtctttt acgactcaat gacaagaaga aaatcttcgt caacatggtg 600
   gagcacgaca ctctcgtcta ctccaagaat atcaaagata cagtctcaga agaccaaagg 660
   gctattgaga cttttcaaca aagggtaata tcgggaaacc tcctcggatt ccattgccca 720
   gctatctgtc acttcatcaa aaggacagta gaaaaggaag gtggcaccta caaatgccat 780
   cattgcgata aaggaaaggc tatcgttcaa gatgcctctg ccgacagtgg tcccaaagat 840
   ggacccccac ccacgaggag catcgtggaa aaagaagacg ttccaaccac gtcttcaaag 900
   caagtggatt gatgtgatat ctccactgac gtaagggatg acgcacaatc ccactatcct 960
   tcgcaagacc cttcctctat ataaggaagt tcatttcatt tggagaggac tgcaggacga 1020
   tccgtatttt tacaacaatt accacaacaa aacaaacaac aaacaacatt acaatttact 1080
   attctagtcg acctgcaggc ggccgcacta gtgatatca 1119
<210> 7
   <211> 1083
   <212> DNA
   <213> Pseudomonas putida
<400> 7
   atgaacctga atcgttttga acgttatccg ttgaccttcg gtccatcccc catcactccc 60
   ttgaaacgcc tcagcgagca cctgggcggc aaggtggaac tgtatgccaa gcgtgaagac 120
   tgcaatagcg gcctggcctt cggcgggaac aaaacgcgca agctcgaata tctgattccc 180
   gaggccatcg agcaaggctg cgacaccttg gtgtccatcg gcggtatcca gtcgaaccag 240
   acccgccagg tcgctgcggt cgccgcccac ttgggtatga agtgtgtgct tgtgcaggaa 300
   aactgggtga actactccga cgctgtatat gaccgcgtcg gcaacatcga gatgtcgcgg 360
   atcatgggag cggatgtgcg gcttgatgct gcaggtttcg acattggaat tcggccgagc 420
   tgggaaaagg ccatgagcga tgtcgtggag cgcggcggca aaccgtttcc aattccggcg 480
   ggctgttccg agcatcccta tggagggctc gggtttgtcg gcttcgctga ggaagtgcgg 540
   cagcaggaaa aggagttggg cttcaagttt gactacatcg tggtctgctc ggtgaccggc 600
   agtacccagg ccggcatggt cgtcggtttc gcggctgacg gtcgctcgaa aaacgtgatc 660
   ggggtcgatg cttcggcgaa accggagcaa accaaggcgc agatcctgcg tatcgctcga 720
   cataccgctg aactggtgga gctggggcgc gaaatcactg aagaggatgt ggtgctcgat 780
   acgcgtttcg cctatccgga atatggcttg cccaacgaag ggacgctgga agcgattcgc 840
   ctgtgcggca gtcttgaggg ggtgttgacc gatccggtct acgagggcaa atccatgcac 900
   ggcatgattg aaatggtacg ccgcggggaa ttccctgacg gctccaaagt tctttatgcc 960
   cacctgggcg gcgcacctgc gttgaacgcc tacagcttct tgtttcgcaa cggctgaaat 1020
   tggtcccgga tggatgtttc agaatccgtt ccaagaaaag atggtgttgg gaaaatgttc 1080
   tag 1083
<210> 8
   <211> 1206
   <212> DNA
   <213> Arabidopsis thaliana
<400> 8
   atgagaggac gaagcttgac actctcaaga gtaaagctcg agcttgcgag aagaagcatg      60
   tctgcaacat ccgtaccttc aatggcggat tttctcacca aaaaacctta ctctcctcct     120
   tcttgggctt ctcatcttcg tccgcttcct tctcacactt tctccctcgc tcaccttcct     180
   actccgatcc atcgatggaa tcttcctggt cttcctaatg gcacagaact ctggatcaag     240
   cgagatgatt tcaccggaat ggaattgagt ggaaacaaag tacgaaaact cgaattctta     300
   atggcggaag ctgttgatca acacgctgat actgtaatca ctatcggcgg tattcagagc     360
   aatcattgtc gtgctacagc cactgcatct aactatctta atctcaattc tcatcttatt     420
   ctccgtactt ccaagcttct tgctgatgaa gatcctggat tggttgggaa tctccttgtc     480
   gagcgtctcg ttggagctaa tgttcatcta atctctaaag aagagtattc ttccattggg     540
   agtgaggctc ttactaatgc tctgaaagag aaactggaaa aagaaggaaa gaaaccctat     600
   gttattccag tcggtggatc gaactctttg ggaacttggg gttatataga agcagcaagg     660
   gaaattgagg agcagctgaa ttatagaccc gatgacctga aatttgatga tattgtggta     720
   gcatgtggca gtggtggtac aattgctggt atttcattgg ggtcttggtt gggagctcta     780
   aaagccaagg ttcatgcttt ctcggtttgc gatgatcctg attacttcta tgactttgtc     840
   caagggcttc tggatggact tcacgctggt gttaactctc gtgatatcgt caacatccac     900
   aatgccaaag gaaaaggata tgccatgaac acgtcagagg agcttgagtt tgtaaagaaa     960
   gtagcaagtt caactggtgt tattcttgat ccggtttaca gtgggaaagc tgcgtatggt    1020
   ttgataaatg agatcaccaa agatcccaaa tgttgggagg gaaggaagat attgttcata    1080
   cacactggtg ggcttcttgg gttgtatgat aaggttgatc aaatggcatc tctgatgggt    1140
   aattggtccc ggatggatgt ttcagaatcc gttccaagaa aagatggtgt tgggaaaatg    1200
   ttctag                                                               1206